# EUROPEAN PATENT SPECIFICATION

(11) **EP 4 329 877 B1**
(45) Date of publication and mention of the grant of the patent: **04.02.2026**
(21) Application number: 22724964.6
(22) Date of filing: 25.04.2022
(51) Int. Cl.: A61P 25/00, C07D 498/04, C07D 498/14, A61K 31/4353

(54) **SUBSTITUTED AMIDE MACROCYCLIC COMPOUNDS WITH OREXIN-2 RECEPTOR AGONIST ACTIVITY**
MAKROCYCLISCHE SUBSTITUIERTE AMIDVERBINDUNGEN MIT OREXIN-2-REZEPTORAGONISTENAKTIVITÄT
COMPOSÉS MACROCYCLIQUES D'AMIDE SUBSTITUÉS AYANT UNE ACTIVITÉ AGONISTE DU RÉCEPTEUR DE L'OREXINE 2

(30) Priority: 26.04.2021 US 202163179616 P
(43) Date of publication of application: 06.03.2024
(73) Proprietor: Alkermes, Inc., Waltham, MA 02451 (US)
(72) Inventor: CHOI, Younggi, Stow, MA 01775 (US); HU, Yuan, Waltham, MA 02451 (US); HUYNH, Hoan, Waltham, MA 02453 (US); AQUILA, Brian, M., Marlborough, MA 01752 (US); RAYMER, Brian, Kenneth, Holliston, MA 01746 (US); MUGGE, Ingo, Andreas, Waltham, MA 02451 (US); WOODS, James, R., Waltham, MA 02453 (US); PENNINGTON, Lewis, D., Arlington, MA 02474 (US); BENTZIEN, Jörg, Martin, White Plains, NY 10603 (US); LEHMANN, Jonathan, Ward, Burlington, MA 01803 (US); HALE, Michael, R., Bedford, MA 01730 (US); VALIULIN, Roman, A., Cambridge, MA 02140 (US)
(74) Representative: Pohlman, Sandra M.
(86) International application number: PCT/US2022/026144
(87) International publication number: WO 2022/232025

(56) References cited:
- WO-A1-2020/004536
- WO-A1-2021/108628
- WO-A1-2022/140316

## Description

### RELATED APPLICATION

This application claims the benefit of U.S. Provisional Application No. 63/179,616, filed on April 26, 2021.

### TECHNICAL FIELD

The present invention relates to substituted macrocyclic compounds, particularly, substituted macrocyclic compounds having agonist activity.

### BACKGROUND OF THE INVENTION

Orexin is a neuropeptide synthesized and released by a subpopulation of neurons within the lateral hypothalamus and its surrounding regions. It consists of two subtypes: orexin A and orexin B. Orexin A and orexin B bind to orexin receptors. Orexin receptors are G protein-coupled receptors expressed preferentially in the brain. There are two subtypes (type 1 and type 2) of orexin receptors (Cell, Vol. 92, 573-585, 1998). Activation of orexin receptors is known to be irnportant for a variety of central nervous system functions, such as maintenance of wakefulness, energy homeostasis, reward processing and motivation (Saper et al., TRENDS in Neuroscience 2001; Yamanaka et al., Neuron 2003; Sakurai, Nature Reviews Neuroscience 2014).

Narcolepsy is a neurological disease that results in excessive daytime sleepiness, sudden bouts of muscular paralysis (cataplexy), and disrupted sleep patterns (Mahoney et al., Nature Reviews Neuroscience, 2019). It is known that narcolepsy is caused by the degeneration of orexin neurons. Narcoleptic symptoms can be modeled in transgenic mice engineered to degenerate orexin neurons, and their symptoms can be reversed by intraventricular administration of orexin peptides (Proc. Natl. Acad. Sci. USA, Vol. 101, 4649-4654, 2004). Studies of orexin-2 receptor knockout mice have suggested that the orexin-2 receptor plays a preferential role in maintaining wakefulness (Cell, Vol. 98, 437-451, 1999, Neuron, Vol. 38, 715-730, 2003). As such, orexin-2 receptor agonists can be therapeutic agents for narcolepsy or other disorders exhibiting excessive daytime sleepiness, such as Parkinson's disease (CNS Drugs, Vol. 27, 83-90, 2013; Brain, Vol. 130, 2007, 1586-1595).

A compound having agonist activity at the orexin-2 receptor is hypothesized to be useful as a novel therapeutic agent for narcolepsy, idiopathic hypersomnia, hypersomnia, sleep apnea syndrome, disturbance of consciousness such as coma and the like, narcolepsy syndrome, hypersomnolence syndrome characterized by hypersomnia (e.g., in Parkinson's disease, Guillain-Barre syndrome or Kleine Levin syndrome), Alzheimer's disease, obesity, insulin resistance syndrome, cardiac failure, diseases related to bone loss, or sepsis and the like. (Cell Metabolism, Vol. 9, 64-76, 2009; Neuroscience, Vol. 121, 855-863, 2003; Respiration, Vol. 71, 575-579, 2004; Peptides, Vol. 23, 1683-1688, 2002; WO 2015/073707; Journal of the American College of Cardiology, Vol. 66, 2015, pages 2522-2533; WO 2015/048091; WO 2015/147240).

Some compounds having orexin-2 receptor agonist activity have been reported (U.S. Pat. No. 8,258,163; WO 2015/088000; WO 2014/198880; Journal of Medicinal Chemistry, Vol. 58, pages 7931-7937; US 20190040010; US 20190031611; US 20170226137). WO2020/004536 discloses compounds which are orexin type 2 receptor agonists.

However, it is considered that these compounds are not satisfactory, for example, in terms of activity, pharmacokinetics, permeability into the brain/central nervous system or safety, and the development of an improved compound having orexin-2 receptor agonist activity is desired.

### SUMMARY OF THE INVENTION

The present invention aims to provide substituted macrocyclic compounds having orexin-2 receptor agonist activity of formulae (I) and (II) as defined in the appended claims.

Accordingly, in an initial aspect, the present disclosure provides a compound represented by Formula I-A or a pharmaceutically acceptable salt thereof: 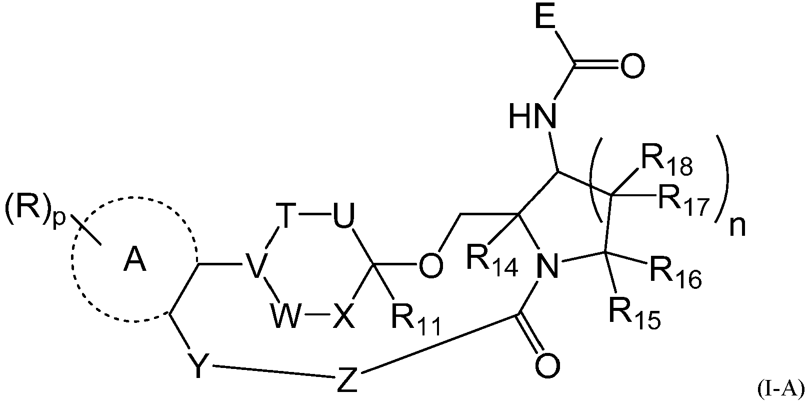 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 1, 2, 3, or 4;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment, provided herein are compounds of Formula I-A having the structure of Formula I or a pharmaceutically acceptable salt thereof: 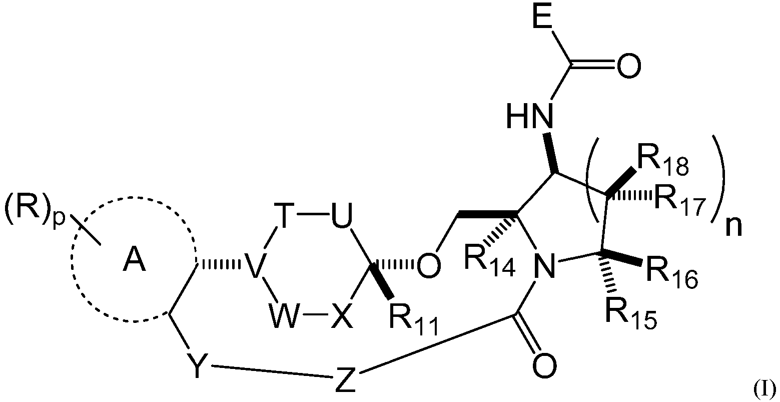 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 1, 2, 3, or 4;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

Also disclosed herein is a compound having the structure of Formula II-A or a pharmaceutically acceptable salt thereof: 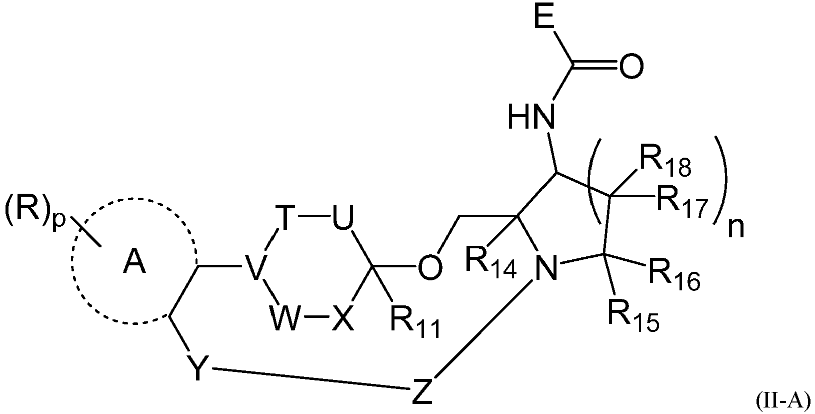 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 2, 3, 4, or 5 when Y is absent; or
m is 1, 2, 3, or 4 when Y is NR₁₀ or O;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment, provided herein are compounds of Formula II-A having the structure of Formula II or a pharmaceutically acceptable salt thereof: 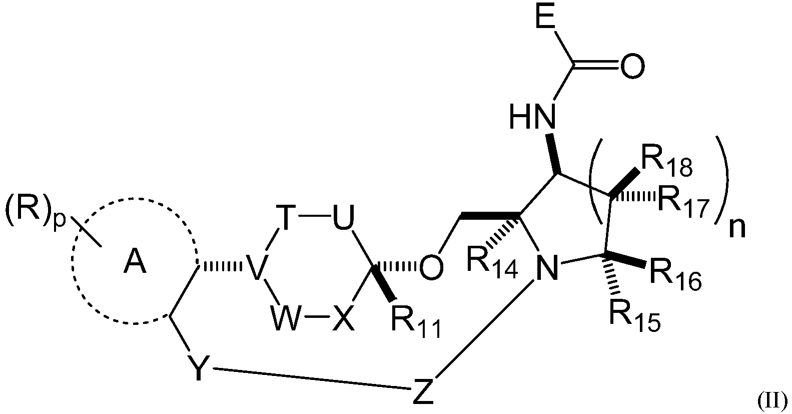 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 2, 3, 4, or 5 when Y is absent; or
m is 1, 2, 3, or 4 when Y is NR₁₀ or O;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

Also disclosed herein is a pharmaceutical composition comprising a compound of any of Formula I-A, I, II-A, or II, or a pharmaceutically acceptable salt thereof, and a pharmaceutically acceptable carrier.

In another aspect, disclosed herein is a method of treating narcolepsy in a subject in need thereof comprising administering to the subject a compound of Formula I-A, I, II-A, or II, or a pharmaceutically acceptable salt thereof.

In another aspect, disclosed herein is a method of treating cataplexy in a subject in need thereof comprising administering to the subject a compound of Formula I-A, I, II-A, or II, or a pharmaceutically acceptable salt thereof.

### DETAILED DESCRIPTION OF THE INVENTION

Disclosed herein are compounds, e.g., the compounds of Formula I-A, I, II-A, or II, or pharmaceutically acceptable salts thereof, that are useful in the treatment of narcolepsy or cataplexy in a subject.

In a non-limiting aspect, these compounds may modulate the orexin-2 receptor. In a particular embodiment, the compounds provided herein are considered orexin-2 agonists. As such, in one aspect, the compounds provided herein are useful in treatment of narcolepsy in a subject by acting as an agonist of the orexin-2 receptor.

### Definitions

Listed below are definitions of various terms used to describe this invention. These definitions apply to the terms as they are used throughout this specification and claims, unless otherwise limited in specific instances, either individually or as part of a larger group.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e., to at least one) of the grammatical object of the article. By way of example, "an element" means one element or more than one element. Furthermore, use of the term "including" as well as other forms, such as "include," "includes," and "included," is not limiting.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, including ±5%, ±1%, and ±0.1% from the specified value, as such variations are appropriate to perform the disclosed methods.

As used to herein, the term "EC₅₀" refers to the concentration of a compound required to achieve an effect that is 50% of the maximal observed effect of a compound.

The term "agonist," as used herein, refers to a compound that, when contacted with a target of interest (e.g., the orexin-2 receptor), causes an increase in the magnitude of a certain activity or function of the target compared to the magnitude of the activity or function observed in the absence of the agonist.

The term "treat," "treated," "treating," or "treatment" includes the diminishment or alleviation of at least one symptom associated or caused by the state, disorder or disease being treated. In certain embodiments, the treatment comprises bringing into contact with the orexin-2 receptor an effective amount of a compound of the invention for conditions related to narcolepsy or cataplexy.

As used herein, the term "prevent" or "prevention" means no disorder or disease development if none had occurred, or no further disorder or disease development if there had already been development of the disorder or disease. Also considered is the ability of one to prevent some or all of the symptoms associated with the disorder or disease.

As used herein, the term "patient," "individual" or "subject" refers to a human or a non-human mammal. Non-human mammals include, for example, livestock and pets, such as ovine, bovine, porcine, canine, feline and murine mammals. Preferably, the patient, subject, or individual is human.

As used herein, the terms "effective amount," "pharmaceutically effective amount," and "therapeutically effective amount" refer to a nontoxic but sufficient amount of an agent to provide the desired biological result. That result may be reduction or alleviation of the signs, symptoms, or causes of a disease, or any other desired alteration of a biological system. An appropriate therapeutic amount in any individual case may be determined by one of ordinary skill in the art using routine experimentation.

As used herein, the term "pharmaceutically acceptable" refers to a material, such as a carrier or diluent, which does not abrogate the biological activity or properties of the compound, and is relatively non-toxic, i.e., the material may be administered to an individual without causing undesirable biological effects or interacting in a deleterious manner with any of the components of the composition in which it is contained.

As used herein, the term "pharmaceutically acceptable salt" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the conventional non-toxic salts of the parent compound formed, for example, from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. The phrase "pharmaceutically acceptable salt" is not limited to a mono, or 1:1, salt. For example, "pharmaceutically acceptable salt" also includes bis-salts, such as a bis-hydrochloride salt. Lists of suitable salts are found in Remington's Pharmaceutical Sciences, 17th ed., Mack Publishing Company, Easton, Pa., 1985, p. 1418 and Journal of Pharmaceutical Science, 66, 22 (1977).

As used herein, the term "composition" or "pharmaceutical composition" refers to a mixture of at least one compound useful within the invention with a pharmaceutically acceptable carrier. The pharmaceutical composition facilitates administration of the compound to a patient or subject. Multiple techniques of administering a compound exist in the art including, but not limited to, intravenous, oral, aerosol, parenteral, ophthalmic, pulmonary, and topical administration.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations.

As used herein, "pharmaceutically acceptable carrier" also includes any and all coatings, antibacterial and antifungal agents, and absorption delaying agents, and the like that are compatible with the activity of the compound useful within the invention and are physiologically acceptable to the patient. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA).

As used herein, the term "alkyl," by itself or as part of another substituent means, unless otherwise stated, a straight or branched chain hydrocarbon having the number of carbon atoms designated (*i.e.,* C₁₋₆ alkyl means an alkyl having one to six carbon atoms) and includes straight and branched chains. Examples include methyl, ethyl, propyl, isopropyl, butyl, isobutyl, *tert*-butyl, pentyl, neopentyl, and hexyl. Other examples of C₁-C₆-alkyl include ethyl, methyl, isopropyl, isobutyl, n-pentyl, and n-hexyl.

As used herein, the term "halo" or "halogen" alone or as part of another substituent means, unless otherwise stated, a fluorine, chlorine, bromine, or iodine atom, preferably, fluorine, chlorine, or bromine, more preferably, fluorine or chlorine.

As used herein, the term "alkylene" refers to divalent aliphatic hydrocarbyl groups, for example, having from 1 to 4 carbon atoms that are either straight-chained or branched. This term includes, by way of example, methylene (-CH₂-), ethylene (-CH₂CH₂-), n-propylene (-CH₂CH₂CH₂-), iso-propylene (-CH₂CH(CH₃)-), and the like.

As used herein, the term "alkenyl" denotes a monovalent group derived from a hydrocarbon moiety containing at least two carbon atoms and at least one carbon-carbon double bond. The double bond may or may not be the point of attachment to another group. Alkenyl groups (e.g., C₂-C₈-alkenyl) include, but are not limited to, for example, ethenyl, propenyl, prop-1-en-2-yl, butenyl, 1-methyl-2-buten-1-yl, heptenyl, octenyl and the like.

As used herein, the term "alkynyl" denotes a monovalent group derived from a hydrocarbon moiety containing at least two carbon atoms and at least one carbon-carbon triple bond. The triple bond may or may not be the point of attachment to another group. Alkynyl groups (e.g., C₂-C₈-alkynyl) include, but are not limited to, for example, ethynyl, propynyl, prop-1-yn-2-yl, butynyl, 1-methyl-2-butyn-1-yl, heptynyl, octynyl and the like.

As used herein, the term "alkoxy," refers to the group -O-alkyl, wherein alkyl is as defined herein. Alkoxy includes, by way of example, methoxy, ethoxy, n-propoxy, isopropoxy, n-butoxy, sec-butoxy, t-butoxy and the like.

As used herein, the term "cycloalkyl" means a non-aromatic carbocyclic system that is partially or fully saturated having 1, 2 or 3 rings wherein such rings may be fused. The term "fused" means that a second ring is present *(i.e.,* attached or formed) by having two adjacent atoms in common (*i.e.*, shared) with the first ring. Cycloalkyl also includes bicyclic structures that may be bridged or spirocyclic in nature with each individual ring within the bicycle varying from 3-8 atoms. The term "cycloalkyl" includes, but is not limited to, cyclopropyl, cyclobutyl, cyclopentyl, cyclohexyl, bicyclo[3.1.0]hexyl, spiro[3.3]heptanyl, and bicyclo[1.1.1]pentyl.

As used herein, the term "heterocyclyl" means a non-aromatic carbocyclic system containing 1, 2, 3 or 4 heteroatoms selected independently from N, O, and S and having 1, 2 or 3 rings wherein such rings may be fused, wherein fused is defined above. Heterocyclyl also includes bicyclic structures that may be bridged or spirocyclic in nature with each individual ring within the bicycle varying from 3-8 atoms, and containing 0, 1, or 2 N, O, or S atoms. The term "heterocyclyl" includes cyclic esters (*i.e.,* lactones) and cyclic amides (*i.e.,* lactams) and also specifically includes, but is not limited to, epoxidyl, oxetanyl, tetrahydrofuranyl, tetrahydropyranyl (*i.e.,* oxanyl), pyranyl, dioxanyl, aziridinyl, azetidinyl, pyrrolidinyl, 2,5-dihydro-1H-pyrrolyl, oxazolidinyl, thiazolidinyl, piperidinyl, morpholinyl, piperazinyl, thiomorpholinyl, 1,3-oxazinanyl, 1,3-thiazinanyl, and the like. For example, the term "heterocyclyl" can include 4- to 10-membered heterocyclyl, 4- to 7-membered heterocyclyl, 5- to 10-membered heterocyclyl, 6- to 10-membered heterocyclyl, 4- to 6-membered heterocyclyl, 4-membered heterocyclyl, 5-membered heterocyclyl, 6-membered heterocyclyl, 7-membered heterocyclyl, 8-membered heterocyclyl, 9-membered heterocyclyl, or 10-membered heterocyclyl.

As used herein, the term "aromatic" refers to a carbocycle or heterocycle with one or more polyunsaturated rings and having aromatic character, *i.e.,* having (4n + 2) delocalized π (pi) electrons, where n is an integer.

As used herein, the term "aryl" means an aromatic carbocyclic system containing 1, 2 or 3 rings, wherein such rings may be fused, wherein fused is defined above. If the rings are fused, one of the rings must be fully unsaturated and the fused ring(s) may be fully saturated, partially unsaturated or fully unsaturated. The term "aryl" includes, but is not limited to, phenyl, naphthyl, indanyl, and 1,2,3,4-tetrahydronaphthalenyl. For example, the term "aryl" can include C₆-C₁₀ aryl, C₆-C₈ aryl, or C₆ aryl *(i.e.,* phenyl).

As used herein, the term "heteroaryl" means an aromatic carbocyclic system containing 1, 2, 3, or 4 heteroatoms selected independently from N, O, and S and having 1, 2, or 3 rings wherein such rings may be fused, wherein fused is defined above. The term "heteroaryl" includes, but is not limited to, furanyl, thiophenyl, oxazolyl, thiazolyl, imidazolyl, pyrazolyl, triazolyl, tetrazolyl, isoxazolyl, isothiazolyl, oxadiazolyl, thiadiazolyl, pyridinyl, pyridazinyl, pyrimidinyl, pyrazinyl and the like. For example, the term "heteroaryl" can include 5- to 10-membered heteroaryl, 5- to 8-membered heteroaryl, 5- to 6-membered heteroaryl, 6- to 10-membered heteroaryl, 6- to 8-membered heteroaryl, 5-membered heteroaryl, 6-membered heteroaryl, 7-membered heteroaryl, 8-membered heteroaryl, 9-membered heteroaryl, or 10-membered heteroaryl.

It is to be understood that if an aryl, heteroaryl, cycloalkyl, or heterocyclyl moiety may be bonded or otherwise attached to a designated moiety through differing ring atoms *(i.e.,* shown or described without denotation of a specific point of attachment), then all possible points are intended, whether through a carbon atom or, for example, a trivalent nitrogen atom. For example, the term "pyridinyl" means 2-, 3- or 4-pyridinyl, the term "thiophenyl" means 2- or 3-thiophenyl, and so forth.

As used herein, the term "substituted" means that an atom or group of atoms has replaced hydrogen as the substituent attached to another group.

### Compounds of the Invention

Accordingly, in an initial aspect, the present disclosure provides a compound represented by Formula I-A or a pharmaceutically acceptable salt thereof: 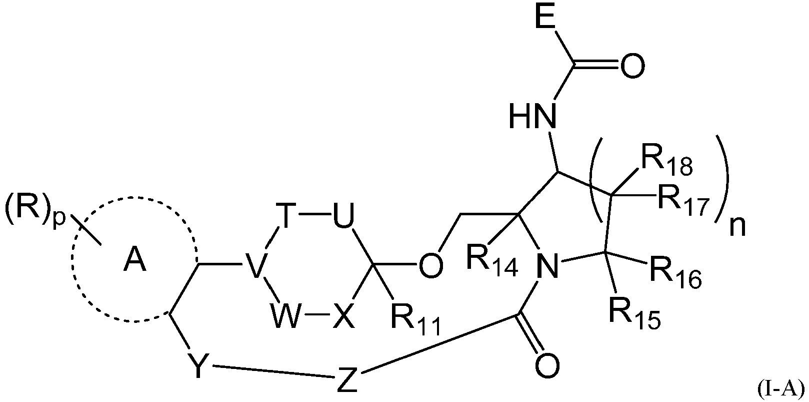 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 1, 2, 3, or 4;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment, provided herein is a compound of Formula I-A having the structure of Formula I or a pharmaceutically acceptable salt thereof: 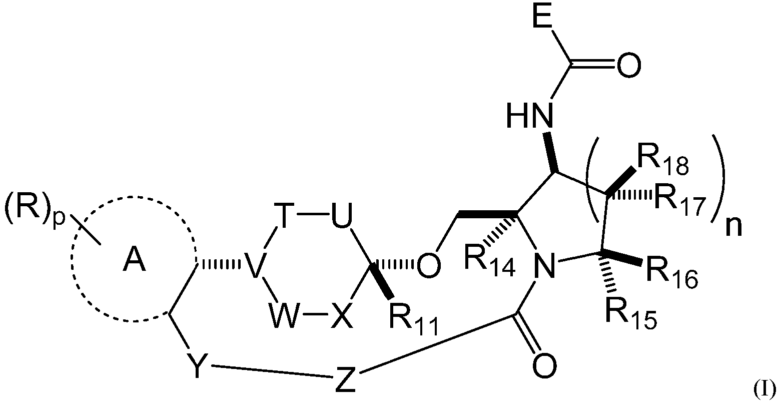 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 1, 2, 3, or 4;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment of Formula (I), n is 1. In another embodiment of Formula (I), n is 2. In another embodiment of Formula (I), n is 3.

In another embodiment of Formula (I), ring A is phenyl. In another embodiment of Formula (I), ring A is pyridinyl. In another embodiment of Formula (I), ring A is pyridazinyl. In another embodiment of Formula (I), ring A is pyrimidinyl. In another embodiment of Formula (I), ring A is pyrazinyl. In another embodiment of Formula (I), ring A is triazinyl.

In another embodiment of Formula (I), Y is NR₁₀. In another embodiment of Formula (I), Y is O. In another embodiment of Formula (I), Y is absent. In another embodiment of Formula (I), ring A is phenyl and Y is NR₁₀. In another embodiment of Formula (I), ring A is phenyl and Y is O. In another embodiment of Formula (I), ring A is phenyl and Y is absent. In another embodiment of Formula (I), ring A is pyridinyl and Y is NR₁₀. In another embodiment of Formula (I), ring A is pyridinyl and Y is O. In another embodiment of Formula (I), ring A is pyridinyl and Y is absent. In another embodiment of Formula (I), ring A is pyridazinyl and Y is NR₁₀. In another embodiment of Formula (I), ring A is pyridazinyl and Y is O. In another embodiment of Formula (I), ring A is pyridazinyl and Y is absent. In another embodiment of Formula (I), ring A is pyrimidinyl and Y is NR₁₀. In another embodiment of Formula (I), ring A is pyrimidinyl and Y is O. In another embodiment of Formula (I), ring A is pyrimidinyl and Y is absent. In another embodiment of Formula (I), ring A is pyrazinyl and Y is NR₁₀. In another embodiment of Formula (I), ring A is pyrazinyl and Y is O. In another embodiment of Formula (I), ring A is pyrazinyl and Y is absent. In another embodiment of Formula (I), ring A is triazinyl and Y is NR₁₀. In another embodiment of Formula (I), ring A is triazinyl and Y is O. In another embodiment of Formula (I), ring A is triazinyl and Y is absent.

In another embodiment of Formula (I), T is CR₁R₂. In another embodiment of Formula (I), T is O. In another embodiment of Formula (I), W is CR₄R₅. In another embodiment of Formula (I), W is O. In another embodiment of Formula (I), T is CR₁R₂ and W is CR₄R₅. In another embodiment of Formula (I), T is O and W is CR₄R₅. In another embodiment of Formula (I), T is CR₁R₂ and W is O.

In another embodiment of Formula (I), V is CR₃. In another embodiment of Formula (I), V is N.

In another embodiment of Formula (I), T is CR₁R₂ and V is CR₃. In another embodiment of Formula (I), T is O and V is CR₃. In another embodiment of Formula (I), T is CR₁R₂ and V is N. In another embodiment of Formula (I), T is O and V is N.

In another embodiment of Formula (I), W is CR₄R₅ and V is CR₃. In another embodiment of Formula (I), W is O and V is CR₃. In another embodiment of Formula (I), W is CR₄R₅ and V is N. In another embodiment of Formula (I), W is O and V is N.

In another embodiment of Formula (I), T is CR₁R₂, W is CR₄R₅, and V is CR₃. In another embodiment of Formula (I), T is CR₁R₂, W is O, and V is CR₃. In another embodiment of Formula (I), T is CR₁R₂, W is CR₄R₅, and V is N. In another embodiment of Formula (I), T is CR₁R₂, W is O, and V is N. In another embodiment of Formula (I), T is O, W is CR₄R₅, and V is CR₃.

In another embodiment of Formula (I), E is NRₐR_{b}. In another embodiment of Formula (I), E is C(=O)NRₐR_{b}. In another embodiment of Formula (I), E is C₁-C₃ alkylene-NRₐR_{b}. In another embodiment of Formula (I), E is unsubstituted C₁-C₃ alkyl, unsubstituted C₂-C₄ alkenyl or unsubstituted C₂-C₄ alkynyl. In another embodiment of Formula (I), E is C₁-C₃ alkyl, C₂-C₄ alkenyl or C₂-C₄ alkynyl substituted with one or more halogen, hydroxyl, C₁-C₃ alkyl, or C₁-C₃ alkoxyl. In another embodiment of Formula (I), E is unsubstituted C₁-C₃ alkyl. In another embodiment of Formula (I), E is C₁-C₃ alkyl substituted with one or more halogen, hydroxyl, C₁-C₃ alkyl, or C₁-C₃ alkoxyl. In another embodiment of Formula (I), E is unsubstituted C₃-C₈ cycloalkyl. In another embodiment of Formula (I), E is C₃-C₈ cycloalkyl substituted with one or more halogen, hydroxyl, C₁-C₃ alkyl, or C₁-C₃ alkoxyl. In another embodiment of Formula (I), E is unsubstituted C₁-C₃ alkylene-(C₃-C₈ cycloalkyl). In another embodiment of Formula (I), E is C₁-C₃ alkylene-(C₃-C₈ cycloalkyl) substituted with one or more halogen, hydroxyl, C₁-C₃ alkyl, or C₁-C₃ alkoxyl. In another embodiment of Formula (I), E is unsubstituted 4- to 10-membered heterocyclyl. In another embodiment of Formula (I), E is 4- to 10-membered heterocyclyl substituted with one or more halogen, hydroxyl, C₁-C₃ alkyl, or C₁-C₃ alkoxyl. In another embodiment of Formula (I), E is unsubstituted C₁-C₃ alkylene-(4- to 10-membered heterocyclyl). In another embodiment of Formula (I), E is C₁-C₃ alkylene-(4- to 10-membered heterocyclyl) substituted with one or more halogen, hydroxyl, C₁-C₃ alkyl, or C₁-C₃ alkoxyl. In another embodiment of Formula (I), E is unsubstituted C₆-C₁₀ aryl. In another embodiment of Formula (I), E is C₆-C₁₀ aryl substituted with one or more halogen, hydroxyl, C₁-C₃ alkyl, or C₁-C₃ alkoxyl. In another embodiment of Formula (I), E is unsubstituted C₁-C₃ alkylene-(C₆-C₁₀ aryl). In another embodiment of Formula (I), E is C₁-C₃ alkylene-(C₆-C₁₀ aryl) substituted with one or more halogen, hydroxyl, C₁-C₃ alkyl, or C₁-C₃ alkoxyl. In another embodiment of Formula (I), E is unsubstituted 5- to 10-membered heteroaryl. In another embodiment of Formula (I), E is 5-to 10-membered heteroaryl substituted with one or more halogen, hydroxyl, C₁-C₃ alkyl, or C₁-C₃ alkoxyl.

In another embodiment of Formula (I), E is unsubstituted 4- to 7-membered heterocyclyl. In another embodiment of Formula (I), E is 4- to 7-membered heterocyclyl substituted with one or more halogen, hydroxyl, C₁-C₃ alkyl, or C₁-C₃ alkoxyl. In another embodiment of Formula (I), E is unsubstituted 4- to 6-membered heterocyclyl. In another embodiment of Formula (I), E is 4- to 6-membered heterocyclyl substituted with one or more halogen, hydroxyl, C₁-C₃ alkyl, or C₁-C₃ alkoxyl. In another embodiment of Formula (I), E is unsubstituted 4-membered heterocyclyl. In another embodiment of Formula (I), E is 4-membered heterocyclyl substituted with one or more halogen, hydroxyl, C₁-C₃ alkyl, or C₁-C₃ alkoxyl. In another embodiment of Formula (I), E is unsubstituted 5-membered heterocyclyl. In another embodiment of Formula (I), E is 5-membered heterocyclyl substituted with one or more halogen, hydroxyl, C₁-C₃ alkyl, or C₁-C₃ alkoxyl. In another embodiment of Formula (I), E is unsubstituted 6-membered heterocyclyl. In another embodiment of Formula (I), E is 6-membered heterocyclyl substituted with one or more halogen, hydroxyl, C₁-C₃ alkyl, or C₁-C₃ alkoxyl.

In another embodiment of Formula (I), E is NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (I), E is C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (I), E is C₁-C₃ alkyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃alkyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (I), E is C₁-C₃ alkyl, C₃-C₈ cycloalkyl, C₁-C₃ alkylene-(C₃-C₈ cycloalkyl), 4- to 10-membered heterocyclyl, or C₁-C₃ alkylene-(4- to 10-membered heterocyclyl), wherein the C₁-C₃alkyl, C₃-C₈ cycloalkyl, C₁-C₃ alkylene-(C₃-C₈ cycloalkyl), 4- to 10-membered heterocyclyl, or C₁-C₃ alkylene-(4- to 10-membered heterocyclyl) is unsubstituted or substituted with one or more halogen, hydroxyl, C₁-C₃ alkyl, or C₁-C₃ alkoxyl.

In another embodiment of Formula (I), E is C₁-C₃ alkyl, C₃-C₈ cycloalkyl, or C₁-C₃ alkylene-(C₃-C₈ cycloalkyl), wherein the C₁-C₃alkyl, C₃-C₈ cycloalkyl, or C₁-C₃ alkylene-(C₃-C₈ cycloalkyl) is unsubstituted or substituted with one or more halogen, hydroxyl, C₁-C₃ alkyl, or C₁-C₃ alkoxyl.

In another embodiment of Formula (I), E is methyl, wherein the methyl is unsubstituted or substituted with one or more halogen, hydroxyl, C₁-C₃ alkyl, or C₁-C₃ alkoxyl. In another embodiment of Formula (I), E is methyl. In another embodiment of Formula (I), E is trifluoromethyl. In another embodiment of Formula (I), E is dioxanyl, wherein the dioxanyl is unsubstituted or substituted with one or more halogen, hydroxyl, C₁-C₃ alkyl, or C₁-C₃ alkoxyl. In another embodiment of Formula (I), E is tetrahydropyranyl, wherein the tetrahydropyranyl is unsubstituted or substituted with one or more halogen, hydroxyl, C₁-C₃ alkyl, or C₁-C₃ alkoxyl. In another embodiment of Formula (I), E is tetrahydrofuranyl, wherein the tetrahydrofuranyl is unsubstituted or substituted with one or more halogen, hydroxyl, C₁-C₃ alkyl, or C₁-C₃ alkoxyl. In another embodiment of Formula (I), E is azetidinyl, wherein the azetidinyl is unsubstituted or substituted with one or more halogen, hydroxyl, C₁-C₃ alkyl, or C₁-C₃ alkoxyl. In another embodiment of Formula (I), E is oxetanyl, wherein the oxetanyl is unsubstituted or substituted with one or more halogen, hydroxyl, C₁-C₃ alkyl, or C₁-C₃ alkoxyl. In another embodiment of Formula (I), E is morpholinyl, wherein the morpholinyl is unsubstituted or substituted with one or more halogen, hydroxyl, C₁-C₃ alkyl, or C₁-C₃ alkoxyl.

In another embodiment of Formula (I), R₁₄ is H. In another embodiment of Formula (I), R₁₄ is unsubstituted C₁-C₃ alkyl. In another embodiment of Formula (I), R₁₅ and R₁₆ are each H. In another embodiment of Formula (I), R₁₅ is unsubstituted C₁-C₃ alkyl and R₁₆ is H. In another embodiment of Formula (I), R₁₆ is unsubstituted C₁-C₃ alkyl and R₁₅ is H. In another embodiment of Formula (I), each R₁₇ and R₁₈ is H. In another embodiment of Formula (I), R₁₇ is unsubstituted C₁-C₃ alkyl and R₁₈ is H. In another embodiment of Formula (I), R₁₈ is unsubstituted C₁-C₃ alkyl and R₁₇ is H. In another embodiment of Formula (I), one of R₁₄, R₁₅, R₁₆, R₁₇ and R₁₈ is unsubstituted C₁-C₃ alkyl and the others are each H.

In another embodiment of Formula (I), m is 1. In another embodiment of Formula (I), m is 2. In another embodiment of Formula (I), m is 3. In another embodiment of Formula (I), m is 4. In another embodiment of Formula (I), m is 1, 2 or 3. In another embodiment of Formula (I), m is 2, 3, or 4. In another embodiment of Formula (I), m is 1 or 2. In another embodiment of Formula (I), m is 3 or 4.

In another embodiment of Formula (I), Y is O and m is 1. In another embodiment of Formula (I), Y is O and m is 2. In another embodiment of Formula (I), Y is O and m is 3. In another embodiment of Formula (I), Y is O and m is 4. In another embodiment of Formula (I), Y is O and m is 1, 2, or 3. In another embodiment of Formula (I), Y is O and m is 2, 3, or 4. In another embodiment of Formula (I), Y is O and m is 1 or 2. In another embodiment of Formula (I), Y is O and m is 3 or 4.

In another embodiment of Formula (I), Y is absent and m is 1. In another embodiment of Formula (I), Y is absent and m is 2. In another embodiment of Formula (I), Y is absent and m is 3. In another embodiment of Formula (I), Y is absent and m is 4. In another embodiment of Formula (I), Y is absent and m is 1, 2, or 3. In another embodiment of Formula (I), Y is absent and m is 2, 3, or 4. In another embodiment of Formula (I), Y is absent and m is 1 or 2. In another embodiment of Formula (I), Y is absent and m is 3 or 4.

In another embodiment of Formula (I), Y is NR₁₀ and m is 1. In another embodiment of Formula (I), Y is NR₁₀ and m is 2. In another embodiment of Formula (I), Y is NR₁₀ and m is 3. In another embodiment of Formula (I), Y is NR₁₀ and m is 4. In another embodiment of Formula (I), Y is NR₁₀ and m is 1, 2, or 3. In another embodiment of Formula (I), Y is NR₁₀ and m is 2, 3, or 4. In another embodiment of Formula (I), Y is NR₁₀ and m is 1 or 2. In another embodiment of Formula (I), Y is NR₁₀ and m is 3 or 4.

In another embodiment of Formula (I), ring A is phenyl and n is 1. In another embodiment of Formula (I), ring A is phenyl and n is 2. In another embodiment of Formula (I), ring A is phenyl and n is 3. In another embodiment of Formula (I), ring A is pyridinyl and n is 1. In another embodiment of Formula (I), ring A is pyridinyl and n is 2. In another embodiment of Formula (I), ring A is pyridinyl and n is 3. In another embodiment of Formula (I), ring A is pyridazinyl and n is 1. In another embodiment of Formula (I), ring A is pyridazinyl and n is 2. In another embodiment of Formula (I), ring A is pyridazinyl and n is 3. In another embodiment of Formula (I), ring A is pyrimidinyl and n is 1. In another embodiment of Formula (I), ring A is pyrimidinyl and n is 2. In another embodiment of Formula (I), ring A is pyrimidinyl and n is 3. In another embodiment of Formula (I), ring A is pyrazinyl and n is 1. In another embodiment of Formula (I), ring A is pyrazinyl and n is 2. In another embodiment of Formula (I), ring A is pyrazinyl and n is 3. In another embodiment of Formula (I), ring A is triazinyl and n is 1. In another embodiment of Formula (I), ring A is triazinyl and n is 2. In another embodiment of Formula (I), ring A is triazinyl and n is 3.

In another embodiment of Formula (I), ring A is phenyl, n is 1, and Y is NR₁₀. In another embodiment of Formula (I), ring A is phenyl, n is 2, and Y is NR₁₀. In another embodiment of Formula (I), ring A is phenyl, n is 3, and Y is NR₁₀. In another embodiment of Formula (I), ring A is phenyl, n is 1, and Y is O. In another embodiment of Formula (I), ring A is phenyl, n is 2, and Y is O. In another embodiment of Formula (I), ring A is phenyl, n is 3, and Y is O. In another embodiment of Formula (I), ring A is phenyl, n is 1, and Y is absent. In another embodiment of Formula (I), ring A is phenyl, n is 2, and Y is absent. In another embodiment of Formula (I), ring A is phenyl, n is 3, and Y is absent.

In another embodiment of Formula (I), ring A is phenyl, n is 1, Y is NR₁₀, and m is 1 or 2. In another embodiment of Formula (I), ring A is phenyl, n is 2, Y is NR₁₀, and m is 1 or 2. In another embodiment of Formula (I), ring A is phenyl, n is 3, Y is NR₁₀, and m is 1 or 2. In another embodiment of Formula (I), ring A is phenyl, n is 1, Y is O, and m is 1 or 2. In another embodiment of Formula (I), ring A is phenyl, n is 2, Y is O, and m is 1 or 2. In another embodiment of Formula (I), ring A is phenyl, n is 3, Y is O, and m is 1 or 2. In another embodiment of Formula (I), ring A is phenyl, n is 1, Y is absent, and m is 1 or 2. In another embodiment of Formula (I), ring A is phenyl, n is 2, Y is absent, and m is 1 or 2. In another embodiment of Formula (I), ring A is phenyl, n is 3, Y is absent, and m is 1 or 2. In another embodiment of Formula (I), ring A is phenyl, n is 1, Y is NR₁₀, and m is 3 or 4. In another embodiment of Formula (I), ring A is phenyl, n is 2, Y is NR₁₀, and m is 3 or 4. In another embodiment of Formula (I), ring A is phenyl, n is 3, Y is NR₁₀, and m is 3 or 4. In another embodiment of Formula (I), ring A is phenyl, n is 1, Y is O, and m is 3 or 4. In another embodiment of Formula (I), ring A is phenyl, n is 2, Y is O, and m is 3 or 4. In another embodiment of Formula (I), ring A is phenyl, n is 3, Y is O, and m is 3 or 4. In another embodiment of Formula (I), ring A is phenyl, n is 1, Y is absent, and m is 3 or 4. In another embodiment of Formula (I), ring A is phenyl, n is 2, Y is absent, and m is 3 or 4. In another embodiment of Formula (I), ring A is phenyl, n is 3, Y is absent, and m is 3 or 4.

In another embodiment of Formula (I), ring A is pyridinyl, n is 1, and Y is NR₁₀. In another embodiment of Formula (I), ring A is pyridinyl, n is 2, and Y is NR₁₀. In another embodiment of Formula (I), ring A is pyridinyl, n is 3, and Y is NR₁₀. In another embodiment of Formula (I), ring A is pyridinyl, n is 1, and Y is O. In another embodiment of Formula (I), ring A is pyridinyl, n is 2, and Y is O. In another embodiment of Formula (I), ring A is pyridinyl, n is 3, and Y is O. In another embodiment of Formula (I), ring A is pyridinyl, n is 1, and Y is absent. In another embodiment of Formula (I), ring A is pyridinyl, n is 2, and Y is absent. In another embodiment of Formula (I), ring A is pyridinyl, n is 3, and Y is absent.

In another embodiment of Formula (I), ring A is pyridinyl, n is 1, Y is NR₁₀, and m is 1 or 2. In another embodiment of Formula (I), ring A is pyridinyl, n is 2, Y is NR₁₀, and m is 1 or 2. In another embodiment of Formula (I), ring A is pyridinyl, n is 3, Y is NR₁₀, and m is 1 or 2. In another embodiment of Formula (I), ring A is pyridinyl, n is 1, Y is O, and m is 1 or 2. In another embodiment of Formula (I), ring A is pyridinyl, n is 2, Y is O, and m is 1 or 2. In another embodiment of Formula (I), ring A is pyridinyl, n is 3, Y is O, and m is 1 or 2. In another embodiment of Formula (I), ring A is pyridinyl, n is 1, Y is absent, and m is 1 or 2. In another embodiment of Formula (I), ring A is pyridinyl, n is 2, Y is absent, and m is 1 or 2. In another embodiment of Formula (I), ring A is pyridinyl, n is 3, Y is absent, and m is 1 or 2. In another embodiment of Formula (I), ring A is pyridinyl, n is 1, Y is NR₁₀, and m is 3 or 4. In another embodiment of Formula (I), ring A is pyridinyl, n is 2, Y is NR₁₀, and m is 3 or 4. In another embodiment of Formula (I), ring A is pyridinyl, n is 3, Y is NR₁₀, and m is 3 or 4. In another embodiment of Formula (I), ring A is pyridinyl, n is 1, Y is O, and m is 3 or 4. In another embodiment of Formula (I), ring A is pyridinyl, n is 2, Y is O, and m is 3 or 4. In another embodiment of Formula (I), ring A is pyridinyl, n is 3, Y is O, and m is 3 or 4. In another embodiment of Formula (I), ring A is pyridinyl, n is 1, Y is absent, and m is 3 or 4. In another embodiment of Formula (I), ring A is pyridinyl, n is 2, Y is absent, and m is 3 or 4. In another embodiment of Formula (I), ring A is pyridinyl, n is 3, Y is absent, and m is 3 or 4.

In another embodiment of Formula (I), ring A is pyridazinyl, n is 1, and Y is NR₁₀. In another embodiment of Formula (I), ring A is pyridazinyl, n is 2, and Y is NR₁₀. In another embodiment of Formula (I), ring A is pyridazinyl, n is 3, and Y is NR₁₀. In another embodiment of Formula (I), ring A is pyridazinyl, n is 1, and Y is O. In another embodiment of Formula (I), ring A is pyridazinyl, n is 2, and Y is O. In another embodiment of Formula (I), ring A is pyridazinyl, n is 3, and Y is O. In another embodiment of Formula (I), ring A is pyridazinyl, n is 1, and Y is absent. In another embodiment of Formula (I), ring A is pyridazinyl, n is 2, and Y is absent. In another embodiment of Formula (I), ring A is pyridazinyl, n is 3, and Y is absent.

In another embodiment of Formula (I), ring A is pyridazinyl, n is 1, Y is NR₁₀, and m is 1 or 2. In another embodiment of Formula (I), ring A is pyridazinyl, n is 2, Y is NR₁₀, and m is 1 or 2. In another embodiment of Formula (I), ring A is pyridazinyl, n is 3, Y is NR₁₀, and m is 1 or 2. In another embodiment of Formula (I), ring A is pyridazinyl, n is 1, Y is O, and m is 1 or 2. In another embodiment of Formula (I), ring A is pyridazinyl, n is 2, Y is O, and m is 1 or 2. In another embodiment of Formula (I), ring A is pyridazinyl, n is 3, Y is O, and m is 1 or 2. In another embodiment of Formula (I), ring A is pyridazinyl, n is 1, Y is absent, and m is 1 or 2. In another embodiment of Formula (I), ring A is pyridazinyl, n is 2, Y is absent, and m is 1 or 2. In another embodiment of Formula (I), ring A is pyridazinyl, n is 3, Y is absent, and m is 1 or 2. In another embodiment of Formula (I), ring A is pyridazinyl, n is 1, Y is NR₁₀, and m is 3 or 4. In another embodiment of Formula (I), ring A is pyridazinyl, n is 2, Y is NR₁₀, and m is 3 or 4. In another embodiment of Formula (I), ring A is pyridazinyl, n is 3, Y is NR₁₀, and m is 3 or 4. In another embodiment of Formula (I), ring A is pyridazinyl, n is 1, Y is O, and m is 3 or 4. In another embodiment of Formula (I), ring A is pyridazinyl, n is 2, Y is O, and m is 3 or 4. In another embodiment of Formula (I), ring A is pyridazinyl, n is 3, Y is O, and m is 3 or 4. In another embodiment of Formula (I), ring A is pyridazinyl, n is 1, Y is absent, and m is 3 or 4. In another embodiment of Formula (I), ring A is pyridazinyl, n is 2, Y is absent, and m is 3 or 4. In another embodiment of Formula (I), ring A is pyridazinyl, n is 3, Y is absent, and m is 3 or 4.

In another embodiment of Formula (I), ring A is pyrimidinyl, n is 1, and Y is NR₁₀. In another embodiment of Formula (I), ring A is pyrimidinyl, n is 2, and Y is NR₁₀. In another embodiment of Formula (I), ring A is pyrimidinyl, n is 3, and Y is NR₁₀. In another embodiment of Formula (I), ring A is pyrimidinyl, n is 1, and Y is O. In another embodiment of Formula (I), ring A is pyrimidinyl, n is 2, and Y is O. In another embodiment of Formula (I), ring A is pyrimidinyl, n is 3, and Y is O. In another embodiment of Formula (I), ring A is pyrimidinyl, n is 1, and Y is absent. In another embodiment of Formula (I), ring A is pyrimidinyl, n is 2, and Y is absent. In another embodiment of Formula (I), ring A is pyrimidinyl, n is 3, and Y is absent.

In another embodiment of Formula (I), ring A is pyrimidinyl, n is 1, Y is NR₁₀, and m is 1 or 2. In another embodiment of Formula (I), ring A is pyrimidinyl, n is 2, Y is NR₁₀, and m is 1 or 2. In another embodiment of Formula (I), ring A is pyrimidinyl, n is 3, Y is NR₁₀, and m is 1 or 2. In another embodiment of Formula (I), ring A is pyrimidinyl, n is 1, Y is O, and m is 1 or 2. In another embodiment of Formula (I), ring A is pyrimidinyl, n is 2, Y is O, and m is 1 or 2. In another embodiment of Formula (I), ring A is pyrimidinyl, n is 3, Y is O, and m is 1 or 2. In another embodiment of Formula (I), ring A is pyrimidinyl, n is 1, Y is absent, and m is 1 or 2. In another embodiment of Formula (I), ring A is pyrimidinyl, n is 2, Y is absent, and m is 1 or 2. In another embodiment of Formula (I), ring A is pyrimidinyl, n is 3, Y is absent, and m is 1 or 2. In another embodiment of Formula (I), ring A is pyrimidinyl, n is 1, Y is NR₁₀, and m is 3 or 4. In another embodiment of Formula (I), ring A is pyrimidinyl, n is 2, Y is NR₁₀, and m is 3 or 4. In another embodiment of Formula (I), ring A is pyrimidinyl, n is 3, Y is NR₁₀, and m is 3 or 4. In another embodiment of Formula (I), ring A is pyrimidinyl, n is 1, Y is O, and m is 3 or 4. In another embodiment of Formula (I), ring A is pyrimidinyl, n is 2, Y is O, and m is 3 or 4. In another embodiment of Formula (I), ring A is pyrimidinyl, n is 3, Y is O, and m is 3 or 4. In another embodiment of Formula (I), ring A is pyrimidinyl, n is 1, Y is absent, and m is 3 or 4. In another embodiment of Formula (I), ring A is pyrimidinyl, n is 2, Y is absent, and m is 3 or 4. In another embodiment of Formula (I), ring A is pyrimidinyl, n is 3, Y is absent, and m is 3 or 4.

In another embodiment of Formula (I), ring A is pyrazinyl, n is 1, and Y is NR₁₀. In another embodiment of Formula (I), ring A is pyrazinyl, n is 2, and Y is NR₁₀. In another embodiment of Formula (I), ring A is pyrazinyl, n is 3, and Y is NR₁₀. In another embodiment of Formula (I), ring A is pyrazinyl, n is 1, and Y is O. In another embodiment of Formula (I), ring A is pyrazinyl, n is 2, and Y is O. In another embodiment of Formula (I), ring A is pyrazinyl, n is 3, and Y is O. In another embodiment of Formula (I), ring A is pyrazinyl, n is 1, and Y is absent. In another embodiment of Formula (I), ring A is pyrazinyl, n is 2, and Y is absent. In another embodiment of Formula (I), ring A is pyrazinyl, n is 3, and Y is absent.

In another embodiment of Formula (I), ring A is pyrazinyl, n is 1, Y is NR₁₀, and m is 1 or 2. In another embodiment of Formula (I), ring A is pyrazinyl, n is 2, Y is NR₁₀, and m is 1 or 2. In another embodiment of Formula (I), ring A is pyrazinyl, n is 3, Y is NR₁₀, and m is 1 or 2. In another embodiment of Formula (I), ring A is pyrazinyl, n is 1, Y is O, and m is 1 or 2. In another embodiment of Formula (I), ring A is pyrazinyl, n is 2, Y is O, and m is 1 or 2. In another embodiment of Formula (I), ring A is pyrazinyl, n is 3, Y is O, and m is 1 or 2. In another embodiment of Formula (I), ring A is pyrazinyl, n is 1, Y is absent, and m is 1 or 2. In another embodiment of Formula (I), ring A is pyrazinyl, n is 2, Y is absent, and m is 1 or 2. In another embodiment of Formula (I), ring A is pyrazinyl, n is 3, Y is absent, and m is 1 or 2. In another embodiment of Formula (I), ring A is pyrazinyl, n is 1, Y is NR₁₀, and m is 3 or 4. In another embodiment of Formula (I), ring A is pyrazinyl, n is 2, Y is NR₁₀, and m is 3 or 4. In another embodiment of Formula (I), ring A is pyrazinyl, n is 3, Y is NR₁₀, and m is 3 or 4. In another embodiment of Formula (I), ring A is pyrazinyl, n is 1, Y is O, and m is 3 or 4. In another embodiment of Formula (I), ring A is pyrazinyl, n is 2, Y is O, and m is 3 or 4. In another embodiment of Formula (I), ring A is pyrazinyl, n is 3, Y is O, and m is 3 or 4. In another embodiment of Formula (I), ring A is pyrazinyl, n is 1, Y is absent, and m is 3 or 4. In another embodiment of Formula (I), ring A is pyrazinyl, n is 2, Y is absent, and m is 3 or 4. In another embodiment of Formula (I), ring A is pyrazinyl, n is 3, Y is absent, and m is 3 or 4.

In another embodiment of Formula (I), ring A is triazinyl, n is 1, and Y is NR₁₀. In another embodiment of Formula (I), ring A is triazinyl, n is 2, and Y is NR₁₀. In another embodiment of Formula (I), ring A is triazinyl, n is 3, and Y is NR₁₀. In another embodiment of Formula (I), ring A is triazinyl, n is 1, and Y is O. In another embodiment of Formula (I), ring A is triazinyl, n is 2, and Y is O. In another embodiment of Formula (I), ring A is triazinyl, n is 3, and Y is O. In another embodiment of Formula (I), ring A is triazinyl, n is 1, and Y is absent. In another embodiment of Formula (I), ring A is triazinyl, n is 2, and Y is absent. In another embodiment of Formula (I), ring A is triazinyl, n is 3, and Y is absent.

In another embodiment of Formula (I), ring A is triazinyl, n is 1, Y is NR₁₀, and m is 1 or 2. In another embodiment of Formula (I), ring A is triazinyl, n is 2, Y is NR₁₀, and m is 1 or 2. In another embodiment of Formula (I), ring A is triazinyl, n is 3, Y is NR₁₀, and m is 1 or 2. In another embodiment of Formula (I), ring A is triazinyl, n is 1, Y is O, and m is 1 or 2. In another embodiment of Formula (I), ring A is triazinyl, n is 2, Y is O, and m is 1 or 2. In another embodiment of Formula (I), ring A is triazinyl, n is 3, Y is O, and m is 1 or 2. In another embodiment of Formula (I), ring A is triazinyl, n is 1, Y is absent, and m is 1 or 2. In another embodiment of Formula (I), ring A is triazinyl, n is 2, Y is absent, and m is 1 or 2. In another embodiment of Formula (I), ring A is triazinyl, n is 3, Y is absent, and m is 1 or 2. In another embodiment of Formula (I), ring A is triazinyl, n is 1, Y is NR₁₀, and m is 3 or 4. In another embodiment of Formula (I), ring A is triazinyl, n is 2, Y is NR₁₀, and m is 3 or 4. In another embodiment of Formula (I), ring A is triazinyl, n is 3, Y is NR₁₀, and m is 3 or 4. In another embodiment of Formula (I), ring A is triazinyl, n is 1, Y is O, and m is 3 or 4. In another embodiment of Formula (I), ring A is triazinyl, n is 2, Y is O, and m is 3 or 4. In another embodiment of Formula (I), ring A is triazinyl, n is 3, Y is O, and m is 3 or 4. In another embodiment of Formula (I), ring A is triazinyl, n is 1, Y is absent, and m is 3 or 4. In another embodiment of Formula (I), ring A is triazinyl, n is 2, Y is absent, and m is 3 or 4. In another embodiment of Formula (I), ring A is triazinyl, n is 3, Y is absent, and m is 3 or 4.

In another embodiment of Formula (I), ring A is phenyl, T is CR₁R₂, W is CR₄R₅, and V is CR₃. In another embodiment of Formula (I), ring A is phenyl, p is 0, T is CR₁R₂, W is CR₄R₅, and V is CR₃. In another embodiment of Formula (I), ring A is phenyl, T is CR₁R₂, W is CR₄R₅, V is CR₃, and n is 1. In another embodiment of Formula (I), ring A is phenyl, T is CR₁R₂, W is CR₄R₅, V is CR₃, and n is 2. In another embodiment of Formula (I), ring A is phenyl, T is CR₁R₂, W is CR₄R₅, V is CR₃, and n is 3. In another embodiment of Formula (I), ring A is phenyl, p is 0, T is CR₁R₂, W is CR₄R₅, V is CR₃, and n is 1. In another embodiment of Formula (I), ring A is phenyl, p is 0, T is CR₁R₂, W is CR₄R₅, V is CR₃, and n is 2. In another embodiment of Formula (I), ring A is phenyl, p is 0, T is CR₁R₂, W is CR₄R₅, V is CR₃, and n is 3.

In another embodiment of Formula (I), ring A is phenyl, T is CR₁R₂, W is CR₄R₅, V is CR₃, and Y is O. In another embodiment of Formula (I), ring A is phenyl, p is 0, T is CR₁R₂, W is CR₄R₅, V is CR₃, and Y is O. In another embodiment of Formula (I), ring A is phenyl, T is CR₁R₂, W is CR₄R₅, V is CR₃, Y is O, and n is 1. In another embodiment of Formula (I), ring A is phenyl, T is CR₁R₂, W is CR₄R₅, V is CR₃, Y is O, and n is 2. In another embodiment of Formula (I), ring A is phenyl, T is CR₁R₂, W is CR₄R₅, V is CR₃, Y is O, and n is 3. In another embodiment of Formula (I), ring A is phenyl, p is 0, T is CR₁R₂, W is CR₄R₅, V is CR₃, Y is O, and n is 1. In another embodiment of Formula (I), ring A is phenyl, p is 0, T is CR₁R₂, W is CR₄R₅, V is CR₃, Y is O, and n is 2. In another embodiment of Formula (I), ring A is phenyl, p is 0, T is CR₁R₂, W is CR₄R₅, V is CR₃, Y is O, and n is 3.

In another embodiment of Formula (I), ring A is phenyl, T is CR₁R₂, W is CR₄R₅, V is CR₃, Y is O, and m is 1 or 2. In another embodiment of Formula (I), ring A is phenyl, p is 0, T is CR₁R₂, W is CR₄R₅, V is CR₃, Y is O, and m is 1 or 2. In another embodiment of Formula (I), ring A is phenyl, T is CR₁R₂, W is CR₄R₅, V is CR₃, Y is O, n is 1, and m is 1 or 2. In another embodiment of Formula (I), ring A is phenyl, T is CR₁R₂, W is CR₄R₅, V is CR₃, Y is O, n is 2, and m is 1 or 2. In another embodiment of Formula (I), ring A is phenyl, T is CR₁R₂, W is CR₄R₅, V is CR₃, Y is O, n is 3, and m is 1 or 2. In another embodiment of Formula (I), ring A is phenyl, p is 0, T is CR₁R₂, W is CR₄R₅, V is CR₃, Y is O, n is 1, and m is 1 or 2. In another embodiment of Formula (I), ring A is phenyl, p is 0, T is CR₁R₂, W is CR₄R₅, V is CR₃, Y is O, n is 2, and m is 1 or 2. In another embodiment of Formula (I), ring A is phenyl, p is 0, T is CR₁R₂, W is CR₄R₅, V is CR₃, Y is O, n is 3, and m is 1 or 2. In another embodiment of Formula (I), ring A is phenyl, T is CR₁R₂, W is CR₄R₅, V is CR₃, Y is O, and m is 3 or 4. In another embodiment of Formula (I), ring A is phenyl, p is 0, T is CR₁R₂, W is CR₄R₅, V is CR₃, Y is O, and m is 3 or 4. In another embodiment of Formula (I), ring A is phenyl, T is CR₁R₂, W is CR₄R₅, V is CR₃, Y is O, n is 1, and m is 3 or 4. In another embodiment of Formula (I), ring A is phenyl, T is CR₁R₂, W is CR₄R₅, V is CR₃, Y is O, n is 2, and m is 3 or 4. In another embodiment of Formula (I), ring A is phenyl, T is CR₁R₂, W is CR₄R₅, V is CR₃, Y is O, n is 3, and m is 3 or 4. In another embodiment of Formula (I), ring A is phenyl, p is 0, T is CR₁R₂, W is CR₄R₅, V is CR₃, Y is O, n is 1, and m is 3 or 4. In another embodiment of Formula (I), ring A is phenyl, p is 0, T is CR₁R₂, W is CR₄R₅, V is CR₃, Y is O, n is 2, and m is 3 or 4. In another embodiment of Formula (I), ring A is phenyl, p is 0, T is CR₁R₂, W is CR₄R₅, V is CR₃, Y is O, n is 3, and m is 3 or 4.

In another embodiment of Formula (I), p is 0 and R₁, R₂, R₄, and R₅ are each H. In another embodiment of Formula (I), p is 0; R₁, R₂, R₄, and R₅ are each H; and R₃ is H. In another embodiment of Formula (I), p is 0; R₁, R₂, R₄, and R₅ are each H; R₃ is H; and R₆, R₇, R₈, R₉, and R₁₁ are each H. In another embodiment of Formula (I), p is 0; R₁, R₂, R₄, and R₅ are each H; R₃ is H; R₆, R₇, R₈, R₉, and R₁₁ are each H; and R₁₂ and R₁₃ are each H.

In another embodiment of Formula (I), p is 1 and R₁, R₂, R₄, and R₅ are each H. In another embodiment of Formula (I), p is 1; R₁, R₂, R₄, and R₅ are each H; and R₃ is H. In another embodiment of Formula (I), p is 1; R₁, R₂, R₄, and R₅ are each H; R₃ is H; and R₆, R₇, R₈, R₉, and R₁₁ are each H. In another embodiment of Formula (I), p is 1; R₁, R₂, R₄, and R₅ are each H; R₃ is H; R₆, R₇, R₈, R₉, and R₁₁ are each H; and R₁₂ and R₁₃ are each H.

In another embodiment of Formula (I), p is 2 and R₁, R₂, R₄, and R₅ are each H. In another embodiment of Formula (I), p is 2; R₁, R₂, R₄, and R₅ are each H; and R₃ is H. In another embodiment of Formula (I), p is 2; R₁, R₂, R₄, and R₅ are each H; R₃ is H; and R₆, R₇, R₈, R₉, and R₁₁ are each H. In another embodiment of Formula (I), p is 2; R₁, R₂, R₄, and R₅ are each H; R₃ is H; R₆, R₇, R₈, R₉, and R₁₁ are each H; and R₁₂ and R₁₃ are each H.

In another embodiment of Formula (I), p is 1, 2, 3, or 4 and R is fluorine. In another embodiment of Formula (I), p is 1, 2, 3, or 4 and R is deuterium. In another embodiment of Formula (I), p is 1, 2, 3, or 4 and each R is, independently, selected from the group consisting of hydroxyl, cyano, unsubstituted C₁-C₃ alkyl, and C₁-C₃ alkyl substituted with one or more halogen or deuterium. In another embodiment of Formula (I), p is 1, 2, 3, or 4 and each R is, independently, selected from the group consisting of cyano, unsubstituted C₁-C₃ alkyl, and C₁-C₃ alkyl substituted with one or more halogen or deuterium. In another embodiment of Formula (I), p is 1 and R is unsubstituted C₁-C₃ alkyl. In another embodiment of Formula (I), p is 1 and R is methyl. In another embodiment of Formula (I), p is 1 or 2 and each R is methyl. In another embodiment of Formula (I), p is 1 and R is C₁-C₃ alkyl substituted with one or more halogen. In another embodiment of Formula (I), p is 1 and R is CF₃. In another embodiment of Formula (I), p is 1 or 2 and each R is CF₃.

In another embodiment of Formula (I), one or more of R₁, R₂, R₄, and R₅ is fluorine. In another embodiment of Formula (I), one or more of R₁, R₂, R₄, and R₅ is deuterium. In another embodiment of Formula (I), one or more of R₆, R₇, R₈, R₉, and R₁₁ is fluorine. In another embodiment of Formula (I), one or more of R₆, R₇, R₈, R₉, and R₁₁ is deuterium. In another embodiment of Formula (I), one or more of each R₁₂ and R₁₃ is fluorine. In another embodiment of Formula (I), one or more of each R₁₂ and R₁₃ is deuterium.

In another embodiment of Formula (I), Y is O, T is CR₁R₂, V is CR₃, W is CR₄R₅, and R₁₁ is H. In another embodiment of Formula (I), Y is O, T is CR₁R₂, V is CR₃, W is CR₄R₅, R₁₁ is H, and m is 1. In another embodiment of Formula (I), Y is O, T is CR₁R₂, V is CR₃, W is CR₄R₅, and each of R₁₁, R₁₄, R₁₅, R₁₆, R₁₇, and R₁₈ is H. In another embodiment of Formula (I), Y is O, T is CR₁R₂, V is CR₃, W is CR₄R₅, each of R₁₁, R₁₄, R₁₅, R₁₆, R₁₇, and R₁₈ is H, and m is 1. In another embodiment of Formula (I), Y is O, T is CR₁R₂, V is CR₃, W is CR₄R₅, and each of R₁₁, R₁₂, R₁₃, R₁₄, R₁₅, R₁₆, R₁₇, and R₁₈ is H. In another embodiment of Formula (I), Y is O, T is CR₁R₂, V is CR₃, W is CR₄R₅, each of R₁₁, R₁₂, R₁₃, R₁₄, R₁₅, R₁₆, R₁₇, and R₁₈ is H, and m is 1.

Each of the embodiments described herein with respect to compounds of Formula I also applies to compounds of Formula I-A.

Also disclosed herein is a compound having the structure of Formula II-A or a pharmaceutically acceptable salt thereof: 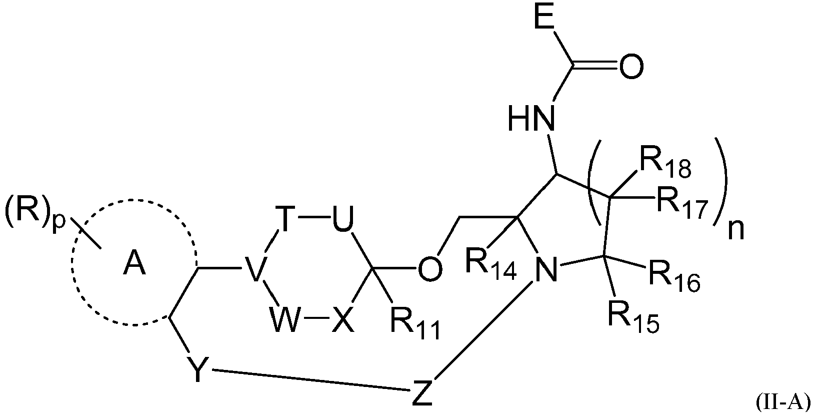 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 2, 3, 4, or 5 when Y is absent; or
m is 1, 2, 3, or 4 when Y is NR₁₀ or O;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment, provided herein are compounds of Formula II-A having the structure of Formula II or a pharmaceutically acceptable salt thereof: 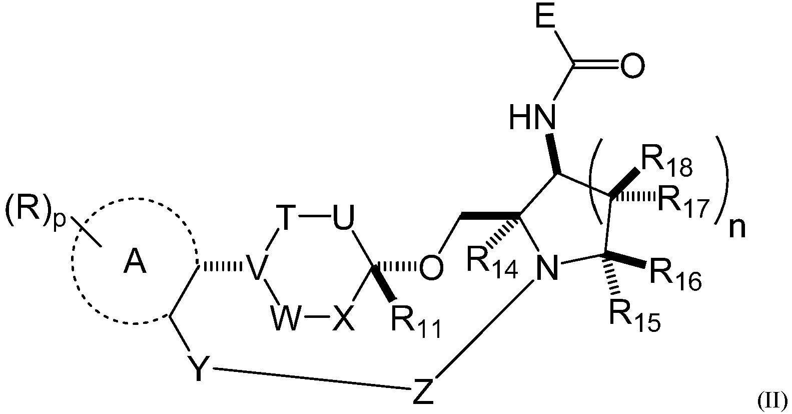 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 2, 3, 4, or 5 when Y is absent; or
m is 1, 2, 3, or 4 when Y is NR₁₀ or O;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

In one embodiment of Formula (II), n is 1. In another embodiment of Formula (II), n is 2. In another embodiment of Formula (II), n is 3.

In another embodiment of Formula (II), ring A is phenyl. In another embodiment of Formula (II), ring A is pyridinyl. In another embodiment of Formula (II), ring A is pyridazinyl. In another embodiment of Formula (II), ring A is pyrimidinyl. In another embodiment of Formula (II), ring A is pyrazinyl. In another embodiment of Formula (II), ring A is triazinyl.

In another embodiment of Formula (II), Y is NR₁₀. In another embodiment of Formula (II), Y is O. In another embodiment of Formula (II), Y is absent. In another embodiment of Formula (II), ring A is phenyl and Y is NR₁₀. In another embodiment of Formula (II), ring A is phenyl and Y is O. In another embodiment of Formula (II), ring A is phenyl and Y is absent. In another embodiment of Formula (II), ring A is pyridinyl and Y is NR₁₀. In another embodiment of Formula (II), ring A is pyridinyl and Y is O. In another embodiment of Formula (II), ring A is pyridinyl and Y is absent. In another embodiment of Formula (II), ring A is pyridazinyl and Y is NR₁₀. In another embodiment of Formula (II), ring A is pyridazinyl and Y is O. In another embodiment of Formula (II), ring A is pyridazinyl and Y is absent. In another embodiment of Formula (II), ring A is pyrimidinyl and Y is NR₁₀. In another embodiment of Formula (II), ring A is pyrimidinyl and Y is O. In another embodiment of Formula (II), ring A is pyrimidinyl and Y is absent. In another embodiment of Formula (II), ring A is pyrazinyl and Y is NR₁₀. In another embodiment of Formula (II), ring A is pyrazinyl and Y is O. In another embodiment of Formula (II), ring A is pyrazinyl and Y is absent. In another embodiment of Formula (II), ring A is triazinyl and Y is NR₁₀. In another embodiment of Formula (II), ring A is triazinyl and Y is O. In another embodiment of Formula (II), ring A is triazinyl and Y is absent.

In another embodiment of Formula (II), T is CR₁R₂. In another embodiment of Formula (II), T is O. In another embodiment of Formula (II), W is CR₄R₅. In another embodiment of Formula (II), W is O. In another embodiment of Formula (II), T is CR₁R₂ and W is CR₄R₅. In another embodiment of Formula (II), T is O and W is CR₄R₅. In another embodiment of Formula (II), T is CR₁R₂ and W is O.

In another embodiment of Formula (II), V is CR₃. In another embodiment of Formula (II), V is N.

In another embodiment of Formula (II), T is CR₁R₂ and V is CR₃. In another embodiment of Formula (II), T is O and V is CR₃. In another embodiment of Formula (II), T is CR₁R₂ and V is N. In another embodiment of Formula (II), T is O and V is N.

In another embodiment of Formula (II), W is CR₄R₅ and V is CR₃. In another embodiment of Formula (II), W is O and V is CR₃. In another embodiment of Formula (II), W is CR₄R₅ and V is N. In another embodiment of Formula (II), W is O and V is N.

In another embodiment of Formula (II), T is CR₁R₂, W is CR₄R₅, and V is CR₃. In another embodiment of Formula (II), T is CR₁R₂, W is O, and V is CR₃. In another embodiment of Formula (II), T is CR₁R₂, W is CR₄R₅, and V is N. In another embodiment of Formula (II), T is CR₁R₂, W is O, and V is N. In another embodiment of Formula (II), T is O, W is CR₄R₅, and V is CR₃.

In another embodiment of Formula (II), E is NRₐR_{b}. In another embodiment of Formula (II), E is C(=O)NRₐR_{b}. In another embodiment of Formula (II), E is C₁-C₃ alkylene-NRₐR_{b}. In another embodiment of Formula (II), E is unsubstituted C₁-C₃ alkyl, unsubstituted C₂-C₄ alkenyl or unsubstituted C₂-C₄ alkynyl. In another embodiment of Formula (II), E is C₁-C₃ alkyl, C₂-C₄ alkenyl or C₂-C₄ alkynyl substituted with one or more halogen, hydroxyl, C₁-C₃ alkyl, or C₁-C₃ alkoxyl. In another embodiment of Formula (II), E is unsubstituted C₁-C₃ alkyl. In another embodiment of Formula (II), E is C₁-C₃ alkyl substituted with one or more halogen, hydroxyl, C₁-C₃ alkyl, or C₁-C₃ alkoxyl. In another embodiment of Formula (II), E is unsubstituted C₃-C₈ cycloalkyl. In another embodiment of Formula (II), E is C₃-C₈ cycloalkyl substituted with one or more halogen, hydroxyl, C₁-C₃ alkyl, or C₁-C₃ alkoxyl. In another embodiment of Formula (II), E is unsubstituted C₁-C₃ alkylene-(C₃-C₈ cycloalkyl). In another embodiment of Formula (II), E is C₁-C₃ alkylene-(C₃-C₈ cycloalkyl) substituted with one or more halogen, hydroxyl, C₁-C₃ alkyl, or C₁-C₃ alkoxyl. In another embodiment of Formula (II), E is unsubstituted 4- to 10-membered heterocyclyl. In another embodiment of Formula (II), E is 4- to 10-membered heterocyclyl substituted with one or more halogen, hydroxyl, C₁-C₃ alkyl, or C₁-C₃ alkoxyl. In another embodiment of Formula (II), E is unsubstituted C₁-C₃ alkylene-(4- to 10-membered heterocyclyl). In another embodiment of Formula (II), E is C₁-C₃ alkylene-(4- to 10-membered heterocyclyl) substituted with one or more halogen, hydroxyl, C₁-C₃ alkyl, or C₁-C₃ alkoxyl. In another embodiment of Formula (II), E is unsubstituted C₆-C₁₀ aryl. In another embodiment of Formula (II), E is C₆-C₁₀ aryl substituted with one or more halogen, hydroxyl, C₁-C₃ alkyl, or C₁-C₃ alkoxyl. In another embodiment of Formula (II), E is unsubstituted C₁-C₃ alkylene-(C₆-C₁₀ aryl). In another embodiment of Formula (II), E is C₁-C₃ alkylene-(C₆-C₁₀ aryl) substituted with one or more halogen, hydroxyl, C₁-C₃ alkyl, or C₁-C₃ alkoxyl. In another embodiment of Formula (II), E is unsubstituted 5- to 10-membered heteroaryl. In another embodiment of Formula (II), E is 5- to 10-membered heteroaryl substituted with one or more halogen, hydroxyl, C₁-C₃ alkyl, or C₁-C₃ alkoxyl.

In another embodiment of Formula (II), E is unsubstituted 4- to 7-membered heterocyclyl. In another embodiment of Formula (II), E is 4- to 7-membered heterocyclyl substituted with one or more halogen, hydroxyl, C₁-C₃ alkyl, or C₁-C₃ alkoxyl. In another embodiment of Formula (II), E is unsubstituted 4- to 6-membered heterocyclyl. In another embodiment of Formula (II), E is 4- to 6-membered heterocyclyl substituted with one or more halogen, hydroxyl, C₁-C₃ alkyl, or C₁-C₃ alkoxyl. In another embodiment of Formula (II), E is unsubstituted 4-membered heterocyclyl. In another embodiment of Formula (II), E is 4-membered heterocyclyl substituted with one or more halogen, hydroxyl, C₁-C₃ alkyl, or C₁-C₃ alkoxyl. In another embodiment of Formula (II), E is unsubstituted 5-membered heterocyclyl. In another embodiment of Formula (II), E is 5-membered heterocyclyl substituted with one or more halogen, hydroxyl, C₁-C₃ alkyl, or C₁-C₃ alkoxyl. In another embodiment of Formula (II), E is unsubstituted 6-membered heterocyclyl. In another embodiment of Formula (II), E is 6-membered heterocyclyl substituted with one or more halogen, hydroxyl, C₁-C₃ alkyl, or C₁-C₃ alkoxyl.

In another embodiment of Formula (II), E is NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (II), E is C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (II), E is C₁-C₃ alkyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl), wherein the C₁-C₃alkyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, or C₁-C₃ alkylene-(C₆-C₁₀ aryl) is unsubstituted or substituted with one or more halogen, hydroxyl, C₁-C₃ alkyl, or C₁-C₃ alkoxyl.

In another embodiment of Formula (II), E is C₁-C₃ alkyl, C₃-C₈ cycloalkyl, C₁-C₃ alkylene-(C₃-C₈ cycloalkyl), 4- to 10-membered heterocyclyl, or C₁-C₃ alkylene-(4- to 10-membered heterocyclyl), wherein the C₁-C₃alkyl, C₃-C₈ cycloalkyl, C₁-C₃ alkylene-(C₃-C₈ cycloalkyl), 4- to 10-membered heterocyclyl, or C₁-C₃ alkylene-(4- to 10-membered heterocyclyl) is unsubstituted or substituted with one or more halogen, hydroxyl, C₁-C₃ alkyl, or C₁-C₃ alkoxyl.

In another embodiment of Formula (II), E is C₁-C₃ alkyl, C₃-C₈ cycloalkyl, or C₁-C₃ alkylene-(C₃-C₈ cycloalkyl), wherein the C₁-C₃alkyl, C₃-C₈ cycloalkyl, or C₁-C₃ alkylene-(C₃-C₈ cycloalkyl) is unsubstituted or substituted with one or more halogen, hydroxyl, C₁-C₃ alkyl, or C₁-C₃ alkoxyl.

In another embodiment of Formula (II), E is methyl, wherein the methyl is unsubstituted or substituted with one or more halogen, hydroxyl, C₁-C₃ alkyl, or C₁-C₃ alkoxyl. In another embodiment of Formula (II), E is methyl. In another embodiment of Formula (II), E is trifluoromethyl. In another embodiment of Formula (II), E is dioxanyl, wherein the dioxanyl is unsubstituted or substituted with one or more halogen, hydroxyl, C₁-C₃ alkyl, or C₁-C₃ alkoxyl. In another embodiment of Formula (II), E is tetrahydropyranyl, wherein the tetrahydropyranyl is unsubstituted or substituted with one or more halogen, hydroxyl, C₁-C₃ alkyl, or C₁-C₃ alkoxyl. In another embodiment of Formula (II), E is tetrahydrofuranyl, wherein the tetrahydrofuranyl is unsubstituted or substituted with one or more halogen, hydroxyl, C₁-C₃ alkyl, or C₁-C₃ alkoxyl. In another embodiment of Formula (II), E is azetidinyl, wherein the azetidinyl is unsubstituted or substituted with one or more halogen, hydroxyl, C₁-C₃ alkyl, or C₁-C₃ alkoxyl. In another embodiment of Formula (II), E is oxetanyl, wherein the oxetanyl is unsubstituted or substituted with one or more halogen, hydroxyl, C₁-C₃ alkyl, or C₁-C₃ alkoxyl. In another embodiment of Formula (II), E is morpholinyl, wherein the morpholinyl is unsubstituted or substituted with one or more halogen, hydroxyl, C₁-C₃ alkyl, or C₁-C₃ alkoxyl.

In another embodiment of Formula (II), R₁₄ is H. In another embodiment of Formula (II), R₁₄ is unsubstituted C₁-C₃ alkyl. In another embodiment of Formula (II), R₁₅ and R₁₆ are each H. In another embodiment of Formula (II), R₁₅ is unsubstituted C₁-C₃ alkyl and R₁₆ is H. In another embodiment of Formula (II), R₁₆ is unsubstituted C₁-C₃ alkyl and R₁₅ is H. In another embodiment of Formula (II), each R₁₇ and R₁₈ is H. In another embodiment of Formula (II), R₁₇ is unsubstituted C₁-C₃ alkyl and R₁₈ is H. In another embodiment of Formula (II), R₁₈ is unsubstituted C₁-C₃ alkyl and R₁₇ is H. In another embodiment of Formula (II), one of R₁₄, R₁₅, R₁₆, R₁₇ and R₁₈ is unsubstituted C₁-C₃ alkyl and the others are each H.

In another embodiment of Formula (II), m is 1. In another embodiment of Formula (II), m is 2. In another embodiment of Formula (II), m is 3. In another embodiment of Formula (II), m is 4. In another embodiment of Formula (II), m is 5. In another embodiment of Formula (II), m is 1, 2 or 3. In another embodiment of Formula (II), m is 2, 3, or 4. In another embodiment of Formula (II), m is 1 or 2. In another embodiment of Formula (II), m is 3 or 4.

In another embodiment of Formula (II), Y is O and m is 1. In another embodiment of Formula (II), Y is O and m is 2. In another embodiment of Formula (II), Y is O and m is 3. In another embodiment of Formula (II), Y is O and m is 4. In another embodiment of Formula (II), Y is O and m is 1, 2, or 3. In another embodiment of Formula (II), Y is O and m is 2, 3, or 4. In another embodiment of Formula (II), Y is O and m is 1 or 2. In another embodiment of Formula (II), Y is O and m is 3 or 4.

In another embodiment of Formula (II), Y is absent and m is 1. In another embodiment of Formula (II), Y is absent and m is 2. In another embodiment of Formula (II), Y is absent and m is 3. In another embodiment of Formula (II), Y is absent and m is 4. In another embodiment of Formula (II), Y is absent and m is 1, 2, or 3. In another embodiment of Formula (II), Y is absent and m is 2, 3, or 4. In another embodiment of Formula (II), Y is absent and m is 1 or 2. In another embodiment of Formula (II), Y is absent and m is 3 or 4.

In another embodiment of Formula (II), Y is NR₁₀ and m is 1. In another embodiment of Formula (II), Y is NR₁₀ and m is 2. In another embodiment of Formula (II), Y is NR₁₀ and m is 3. In another embodiment of Formula (II), Y is NR₁₀ and m is 4. In another embodiment of Formula (II), Y is NR₁₀ and m is 1, 2, or 3. In another embodiment of Formula (II), Y is NR₁₀ and m is 2, 3, or 4. In another embodiment of Formula (II), Y is NR₁₀ and m is 1 or 2. In another embodiment of Formula (II), Y is NR₁₀ and m is 3 or 4.

In another embodiment of Formula (II), ring A is phenyl and n is 1. In another embodiment of Formula (II), ring A is phenyl and n is 2. In another embodiment of Formula (II), ring A is phenyl and n is 3. In another embodiment of Formula (II), ring A is pyridinyl and n is 1. In another embodiment of Formula (II), ring A is pyridinyl and n is 2. In another embodiment of Formula (II), ring A is pyridinyl and n is 3. In another embodiment of Formula (II), ring A is pyridazinyl and n is 1. In another embodiment of Formula (II), ring A is pyridazinyl and n is 2. In another embodiment of Formula (II), ring A is pyridazinyl and n is 3. In another embodiment of Formula (II), ring A is pyrimidinyl and n is 1. In another embodiment of Formula (II), ring A is pyrimidinyl and n is 2. In another embodiment of Formula (II), ring A is pyrimidinyl and n is 3. In another embodiment of Formula (II), ring A is pyrazinyl and n is 1. In another embodiment of Formula (II), ring A is pyrazinyl and n is 2. In another embodiment of Formula (II), ring A is pyrazinyl and n is 3. In another embodiment of Formula (II), ring A is triazinyl and n is 1. In another embodiment of Formula (II), ring A is triazinyl and n is 2. In another embodiment of Formula (II), ring A is triazinyl and n is 3.

In another embodiment of Formula (II), ring A is phenyl, n is 1, and Y is NR₁₀. In another embodiment of Formula (II), ring A is phenyl, n is 2, and Y is NR₁₀. In another embodiment of Formula (II), ring A is phenyl, n is 3, and Y is NR₁₀. In another embodiment of Formula (II), ring A is phenyl, n is 1, and Y is O. In another embodiment of Formula (II), ring A is phenyl, n is 2, and Y is O. In another embodiment of Formula (II), ring A is phenyl, n is 3, and Y is O. In another embodiment of Formula (II), ring A is phenyl, n is 1, and Y is absent. In another embodiment of Formula (II), ring A is phenyl, n is 2, and Y is absent. In another embodiment of Formula (II), ring A is phenyl, n is 3, and Y is absent.

In another embodiment of Formula (II), ring A is phenyl, n is 1, Y is NR₁₀, and m is 1 or 2. In another embodiment of Formula (II), ring A is phenyl, n is 2, Y is NR₁₀, and m is 1 or 2. In another embodiment of Formula (II), ring A is phenyl, n is 3, Y is NR₁₀, and m is 1 or 2. In another embodiment of Formula (II), ring A is phenyl, n is 1, Y is O, and m is 1 or 2. In another embodiment of Formula (II), ring A is phenyl, n is 2, Y is O, and m is 1 or 2. In another embodiment of Formula (II), ring A is phenyl, n is 3, Y is O, and m is 1 or 2. In another embodiment of Formula (II), ring A is phenyl, n is 1, Y is absent, and m is 1 or 2. In another embodiment of Formula (II), ring A is phenyl, n is 2, Y is absent, and m is 1 or 2. In another embodiment of Formula (II), ring A is phenyl, n is 3, Y is absent, and m is 1 or 2. In another embodiment of Formula (II), ring A is phenyl, n is 1, Y is NR₁₀, and m is 3 or 4. In another embodiment of Formula (II), ring A is phenyl, n is 2, Y is NR₁₀, and m is 3 or 4. In another embodiment of Formula (II), ring A is phenyl, n is 3, Y is NR₁₀, and m is 3 or 4. In another embodiment of Formula (II), ring A is phenyl, n is 1, Y is O, and m is 3 or 4. In another embodiment of Formula (II), ring A is phenyl, n is 2, Y is O, and m is 3 or 4. In another embodiment of Formula (II), ring A is phenyl, n is 3, Y is O, and m is 3 or 4. In another embodiment of Formula (II), ring A is phenyl, n is 1, Y is absent, and m is 3 or 4. In another embodiment of Formula (II), ring A is phenyl, n is 2, Y is absent, and m is 3 or 4. In another embodiment of Formula (II), ring A is phenyl, n is 3, Y is absent, and m is 3 or 4.

In another embodiment of Formula (II), ring A is pyridinyl, n is 1, and Y is NR₁₀. In another embodiment of Formula (II), ring A is pyridinyl, n is 2, and Y is NR₁₀. In another embodiment of Formula (II), ring A is pyridinyl, n is 3, and Y is NR₁₀. In another embodiment of Formula (II), ring A is pyridinyl, n is 1, and Y is O. In another embodiment of Formula (II), ring A is pyridinyl, n is 2, and Y is O. In another embodiment of Formula (II), ring A is pyridinyl, n is 3, and Y is O. In another embodiment of Formula (II), ring A is pyridinyl, n is 1, and Y is absent. In another embodiment of Formula (II), ring A is pyridinyl, n is 2, and Y is absent. In another embodiment of Formula (II), ring A is pyridinyl, n is 3, and Y is absent.

In another embodiment of Formula (II), ring A is pyridinyl, n is 1, Y is NR₁₀, and m is 1 or 2. In another embodiment of Formula (II), ring A is pyridinyl, n is 2, Y is NR₁₀, and m is 1 or 2. In another embodiment of Formula (II), ring A is pyridinyl, n is 3, Y is NR₁₀, and m is 1 or 2. In another embodiment of Formula (II), ring A is pyridinyl, n is 1, Y is O, and m is 1 or 2. In another embodiment of Formula (II), ring A is pyridinyl, n is 2, Y is O, and m is 1 or 2. In another embodiment of Formula (II), ring A is pyridinyl, n is 3, Y is O, and m is 1 or 2. In another embodiment of Formula (II), ring A is pyridinyl, n is 1, Y is absent, and m is 1 or 2. In another embodiment of Formula (II), ring A is pyridinyl, n is 2, Y is absent, and m is 1 or 2. In another embodiment of Formula (II), ring A is pyridinyl, n is 3, Y is absent, and m is 1 or 2. In another embodiment of Formula (II), ring A is pyridinyl, n is 1, Y is NR₁₀, and m is 3 or 4. In another embodiment of Formula (II), ring A is pyridinyl, n is 2, Y is NR₁₀, and m is 3 or 4. In another embodiment of Formula (II), ring A is pyridinyl, n is 3, Y is NR₁₀, and m is 3 or 4. In another embodiment of Formula (II), ring A is pyridinyl, n is 1, Y is O, and m is 3 or 4. In another embodiment of Formula (II), ring A is pyridinyl, n is 2, Y is O, and m is 3 or 4. In another embodiment of Formula (II), ring A is pyridinyl, n is 3, Y is O, and m is 3 or 4. In another embodiment of Formula (II), ring A is pyridinyl, n is 1, Y is absent, and m is 3 or 4. In another embodiment of Formula (II), ring A is pyridinyl, n is 2, Y is absent, and m is 3 or 4. In another embodiment of Formula (II), ring A is pyridinyl, n is 3, Y is absent, and m is 3 or 4.

In another embodiment of Formula (II), ring A is pyridazinyl, n is 1, and Y is NR₁₀. In another embodiment of Formula (II), ring A is pyridazinyl, n is 2, and Y is NR₁₀. In another embodiment of Formula (II), ring A is pyridazinyl, n is 3, and Y is NR₁₀. In another embodiment of Formula (II), ring A is pyridazinyl, n is 1, and Y is O. In another embodiment of Formula (II), ring A is pyridazinyl, n is 2, and Y is O. In another embodiment of Formula (II), ring A is pyridazinyl, n is 3, and Y is O. In another embodiment of Formula (II), ring A is pyridazinyl, n is 1, and Y is absent. In another embodiment of Formula (II), ring A is pyridazinyl, n is 2, and Y is absent. In another embodiment of Formula (II), ring A is pyridazinyl, n is 3, and Y is absent.

In another embodiment of Formula (II), ring A is pyridazinyl, n is 1, Y is NR₁₀, and m is 1 or 2. In another embodiment of Formula (II), ring A is pyridazinyl, n is 2, Y is NR₁₀, and m is 1 or 2. In another embodiment of Formula (II), ring A is pyridazinyl, n is 3, Y is NR₁₀, and m is 1 or 2. In another embodiment of Formula (II), ring A is pyridazinyl, n is 1, Y is O, and m is 1 or 2. In another embodiment of Formula (II), ring A is pyridazinyl, n is 2, Y is O, and m is 1 or 2. In another embodiment of Formula (II), ring A is pyridazinyl, n is 3, Y is O, and m is 1 or 2. In another embodiment of Formula (II), ring A is pyridazinyl, n is 1, Y is absent, and m is 1 or 2. In another embodiment of Formula (II), ring A is pyridazinyl, n is 2, Y is absent, and m is 1 or 2. In another embodiment of Formula (II), ring A is pyridazinyl, n is 3, Y is absent, and m is 1 or 2. In another embodiment of Formula (II), ring A is pyridazinyl, n is 1, Y is NR₁₀, and m is 3 or 4. In another embodiment of Formula (II), ring A is pyridazinyl, n is 2, Y is NR₁₀, and m is 3 or 4. In another embodiment of Formula (II), ring A is pyridazinyl, n is 3, Y is NR₁₀, and m is 3 or 4. In another embodiment of Formula (II), ring A is pyridazinyl, n is 1, Y is O, and m is 3 or 4. In another embodiment of Formula (II), ring A is pyridazinyl, n is 2, Y is O, and m is 3 or 4. In another embodiment of Formula (II), ring A is pyridazinyl, n is 3, Y is O, and m is 3 or 4. In another embodiment of Formula (II), ring A is pyridazinyl, n is 1, Y is absent, and m is 3 or 4. In another embodiment of Formula (II), ring A is pyridazinyl, n is 2, Y is absent, and m is 3 or 4. In another embodiment of Formula (II), ring A is pyridazinyl, n is 3, Y is absent, and m is 3 or 4.

In another embodiment of Formula (II), ring A is pyrimidinyl, n is 1, and Y is NR₁₀. In another embodiment of Formula (II), ring A is pyrimidinyl, n is 2, and Y is NR₁₀. In another embodiment of Formula (II), ring A is pyrimidinyl, n is 3, and Y is NR₁₀. In another embodiment of Formula (II), ring A is pyrimidinyl, n is 1, and Y is O. In another embodiment of Formula (II), ring A is pyrimidinyl, n is 2, and Y is O. In another embodiment of Formula (II), ring A is pyrimidinyl, n is 3, and Y is O. In another embodiment of Formula (II), ring A is pyrimidinyl, n is 1, and Y is absent. In another embodiment of Formula (II), ring A is pyrimidinyl, n is 2, and Y is absent. In another embodiment of Formula (II), ring A is pyrimidinyl, n is 3, and Y is absent.

In another embodiment of Formula (II), ring A is pyrimidinyl, n is 1, Y is NR₁₀, and m is 1 or 2. In another embodiment of Formula (II), ring A is pyrimidinyl, n is 2, Y is NR₁₀, and m is 1 or 2. In another embodiment of Formula (II), ring A is pyrimidinyl, n is 3, Y is NR₁₀, and m is 1 or 2. In another embodiment of Formula (II), ring A is pyrimidinyl, n is 1, Y is O, and m is 1 or 2. In another embodiment of Formula (II), ring A is pyrimidinyl, n is 2, Y is O, and m is 1 or 2. In another embodiment of Formula (II), ring A is pyrimidinyl, n is 3, Y is O, and m is 1 or 2. In another embodiment of Formula (II), ring A is pyrimidinyl, n is 1, Y is absent, and m is 1 or 2. In another embodiment of Formula (II), ring A is pyrimidinyl, n is 2, Y is absent, and m is 1 or 2. In another embodiment of Formula (II), ring A is pyrimidinyl, n is 3, Y is absent, and m is 1 or 2. In another embodiment of Formula (II), ring A is pyrimidinyl, n is 1, Y is NR₁₀, and m is 3 or 4. In another embodiment of Formula (II), ring A is pyrimidinyl, n is 2, Y is NR₁₀, and m is 3 or 4. In another embodiment of Formula (II), ring A is pyrimidinyl, n is 3, Y is NR₁₀, and m is 3 or 4. In another embodiment of Formula (II), ring A is pyrimidinyl, n is 1, Y is O, and m is 3 or 4. In another embodiment of Formula (II), ring A is pyrimidinyl, n is 2, Y is O, and m is 3 or 4. In another embodiment of Formula (II), ring A is pyrimidinyl, n is 3, Y is O, and m is 3 or 4. In another embodiment of Formula (II), ring A is pyrimidinyl, n is 1, Y is absent, and m is 3 or 4. In another embodiment of Formula (II), ring A is pyrimidinyl, n is 2, Y is absent, and m is 3 or 4. In another embodiment of Formula (II), ring A is pyrimidinyl, n is 3, Y is absent, and m is 3 or 4.

In another embodiment of Formula (II), ring A is pyrazinyl, n is 1, and Y is NR₁₀. In another embodiment of Formula (II), ring A is pyrazinyl, n is 2, and Y is NR₁₀. In another embodiment of Formula (II), ring A is pyrazinyl, n is 3, and Y is NR₁₀. In another embodiment of Formula (II), ring A is pyrazinyl, n is 1, and Y is O. In another embodiment of Formula (II), ring A is pyrazinyl, n is 2, and Y is O. In another embodiment of Formula (II), ring A is pyrazinyl, n is 3, and Y is O. In another embodiment of Formula (II), ring A is pyrazinyl, n is 1, and Y is absent. In another embodiment of Formula (II), ring A is pyrazinyl, n is 2, and Y is absent. In another embodiment of Formula (II), ring A is pyrazinyl, n is 3, and Y is absent.

In another embodiment of Formula (II), ring A is pyrazinyl, n is 1, Y is NR₁₀, and m is 1 or 2. In another embodiment of Formula (II), ring A is pyrazinyl, n is 2, Y is NR₁₀, and m is 1 or 2. In another embodiment of Formula (II), ring A is pyrazinyl, n is 3, Y is NR₁₀, and m is 1 or 2. In another embodiment of Formula (II), ring A is pyrazinyl, n is 1, Y is O, and m is 1 or 2. In another embodiment of Formula (II), ring A is pyrazinyl, n is 2, Y is O, and m is 1 or 2. In another embodiment of Formula (II), ring A is pyrazinyl, n is 3, Y is O, and m is 1 or 2. In another embodiment of Formula (II), ring A is pyrazinyl, n is 1, Y is absent, and m is 1 or 2. In another embodiment of Formula (II), ring A is pyrazinyl, n is 2, Y is absent, and m is 1 or 2. In another embodiment of Formula (II), ring A is pyrazinyl, n is 3, Y is absent, and m is 1 or 2. In another embodiment of Formula (II), ring A is pyrazinyl, n is 1, Y is NR₁₀, and m is 3 or 4. In another embodiment of Formula (II), ring A is pyrazinyl, n is 2, Y is NR₁₀, and m is 3 or 4. In another embodiment of Formula (II), ring A is pyrazinyl, n is 3, Y is NR₁₀, and m is 3 or 4. In another embodiment of Formula (II), ring A is pyrazinyl, n is 1, Y is O, and m is 3 or 4. In another embodiment of Formula (II), ring A is pyrazinyl, n is 2, Y is O, and m is 3 or 4. In another embodiment of Formula (II), ring A is pyrazinyl, n is 3, Y is O, and m is 3 or 4. In another embodiment of Formula (II), ring A is pyrazinyl, n is 1, Y is absent, and m is 3 or 4. In another embodiment of Formula (II), ring A is pyrazinyl, n is 2, Y is absent, and m is 3 or 4. In another embodiment of Formula (II), ring A is pyrazinyl, n is 3, Y is absent, and m is 3 or 4.

In another embodiment of Formula (II), ring A is triazinyl, n is 1, and Y is NR₁₀. In another embodiment of Formula (II), ring A is triazinyl, n is 2, and Y is NR₁₀. In another embodiment of Formula (II), ring A is triazinyl, n is 3, and Y is NR₁₀. In another embodiment of Formula (II), ring A is triazinyl, n is 1, and Y is O. In another embodiment of Formula (II), ring A is triazinyl, n is 2, and Y is O. In another embodiment of Formula (II), ring A is triazinyl, n is 3, and Y is O. In another embodiment of Formula (II), ring A is triazinyl, n is 1, and Y is absent. In another embodiment of Formula (II), ring A is triazinyl, n is 2, and Y is absent. In another embodiment of Formula (II), ring A is triazinyl, n is 3, and Y is absent.

In another embodiment of Formula (II), ring A is triazinyl, n is 1, Y is NR₁₀, and m is 1 or 2. In another embodiment of Formula (II), ring A is triazinyl, n is 2, Y is NR₁₀, and m is 1 or 2. In another embodiment of Formula (II), ring A is triazinyl, n is 3, Y is NR₁₀, and m is 1 or 2. In another embodiment of Formula (II), ring A is triazinyl, n is 1, Y is O, and m is 1 or 2. In another embodiment of Formula (II), ring A is triazinyl, n is 2, Y is O, and m is 1 or 2. In another embodiment of Formula (II), ring A is triazinyl, n is 3, Y is O, and m is 1 or 2. In another embodiment of Formula (II), ring A is triazinyl, n is 1, Y is absent, and m is 1 or 2. In another embodiment of Formula (II), ring A is triazinyl, n is 2, Y is absent, and m is 1 or 2. In another embodiment of Formula (II), ring A is triazinyl, n is 3, Y is absent, and m is 1 or 2. In another embodiment of Formula (II), ring A is triazinyl, n is 1, Y is NR₁₀, and m is 3 or 4. In another embodiment of Formula (II), ring A is triazinyl, n is 2, Y is NR₁₀, and m is 3 or 4. In another embodiment of Formula (II), ring A is triazinyl, n is 3, Y is NR₁₀, and m is 3 or 4. In another embodiment of Formula (II), ring A is triazinyl, n is 1, Y is O, and m is 3 or 4. In another embodiment of Formula (II), ring A is triazinyl, n is 2, Y is O, and m is 3 or 4. In another embodiment of Formula (II), ring A is triazinyl, n is 3, Y is O, and m is 3 or 4. In another embodiment of Formula (II), ring A is triazinyl, n is 1, Y is absent, and m is 3 or 4. In another embodiment of Formula (II), ring A is triazinyl, n is 2, Y is absent, and m is 3 or 4. In another embodiment of Formula (II), ring A is triazinyl, n is 3, Y is absent, and m is 3 or 4.

In another embodiment of Formula (II), ring A is phenyl, T is CR₁R₂, W is CR₄R₅, and V is CR₃. In another embodiment of Formula (II), ring A is phenyl, p is 0, T is CR₁R₂, W is CR₄R₅, and V is CR₃. In another embodiment of Formula (II), ring A is phenyl, T is CR₁R₂, W is CR₄R₅, V is CR₃, and n is 1. In another embodiment of Formula (II), ring A is phenyl, T is CR₁R₂, W is CR₄R₅, V is CR₃, and n is 2. In another embodiment of Formula (II), ring A is phenyl, T is CR₁R₂, W is CR₄R₅, V is CR₃, and n is 3. In another embodiment of Formula (II), ring A is phenyl, p is 0, T is CR₁R₂, W is CR₄R₅, V is CR₃, and n is 1. In another embodiment of Formula (II), ring A is phenyl, p is 0, T is CR₁R₂, W is CR₄R₅, V is CR₃, and n is 2. In another embodiment of Formula (II), ring A is phenyl, p is 0, T is CR₁R₂, W is CR₄R₅, V is CR₃, and n is 3.

In another embodiment of Formula (II), ring A is phenyl, T is CR₁R₂, W is CR₄R₅, V is CR₃, and Y is O. In another embodiment of Formula (II), ring A is phenyl, p is 0, T is CR₁R₂, W is CR₄R₅, V is CR₃, and Y is O. In another embodiment of Formula (II), ring A is phenyl, T is CR₁R₂, W is CR₄R₅, V is CR₃, Y is O, and n is 1. In another embodiment of Formula (II), ring A is phenyl, T is CR₁R₂, W is CR₄R₅, V is CR₃, Y is O, and n is 2. In another embodiment of Formula (II), ring A is phenyl, T is CR₁R₂, W is CR₄R₅, V is CR₃, Y is O, and n is 3. In another embodiment of Formula (II), ring A is phenyl, p is 0, T is CR₁R₂, W is CR₄R₅, V is CR₃, Y is O, and n is 1. In another embodiment of Formula (II), ring A is phenyl, p is 0, T is CR₁R₂, W is CR₄R₅, V is CR₃, Y is O, and n is 2. In another embodiment of Formula (II), ring A is phenyl, p is 0, T is CR₁R₂, W is CR₄R₅, V is CR₃, Y is O, and n is 3.

In another embodiment of Formula (II), ring A is phenyl, T is CR₁R₂, W is CR₄R₅, V is CR₃, Y is O, and m is 1 or 2. In another embodiment of Formula (II), ring A is phenyl, p is 0, T is CR₁R₂, W is CR₄R₅, V is CR₃, Y is O, and m is 1 or 2. In another embodiment of Formula (II), ring A is phenyl, T is CR₁R₂, W is CR₄R₅, V is CR₃, Y is O, n is 1, and m is 1 or 2. In another embodiment of Formula (II), ring A is phenyl, T is CR₁R₂, W is CR₄R₅, V is CR₃, Y is O, n is 2, and m is 1 or 2. In another embodiment of Formula (II), ring A is phenyl, T is CR₁R₂, W is CR₄R₅, V is CR₃, Y is O, n is 3, and m is 1 or 2. In another embodiment of Formula (II), ring A is phenyl, p is 0, T is CR₁R₂, W is CR₄R₅, V is CR₃, Y is O, n is 1, and m is 1 or 2. In another embodiment of Formula (II), ring A is phenyl, p is 0, T is CR₁R₂, W is CR₄R₅, V is CR₃, Y is O, n is 2, and m is 1 or 2. In another embodiment of Formula (II), ring A is phenyl, p is 0, T is CR₁R₂, W is CR₄R₅, V is CR₃, Y is O, n is 3, and m is 1 or 2. In another embodiment of Formula (II), ring A is phenyl, T is CR₁R₂, W is CR₄R₅, V is CR₃, Y is O, and m is 3 or 4. In another embodiment of Formula (II), ring A is phenyl, p is 0, T is CR₁R₂, W is CR₄R₅, V is CR₃, Y is O, and m is 3 or 4. In another embodiment of Formula (II), ring A is phenyl, T is CR₁R₂, W is CR₄R₅, V is CR₃, Y is O, n is 1, and m is 3 or 4. In another embodiment of Formula (II), ring A is phenyl, T is CR₁R₂, W is CR₄R₅, V is CR₃, Y is O, n is 2, and m is 3 or 4. In another embodiment of Formula (II), ring A is phenyl, T is CR₁R₂, W is CR₄R₅, V is CR₃, Y is O, n is 3, and m is 3 or 4. In another embodiment of Formula (II), ring A is phenyl, p is 0, T is CR₁R₂, W is CR₄R₅, V is CR₃, Y is O, n is 1, and m is 3 or 4. In another embodiment of Formula (II), ring A is phenyl, p is 0, T is CR₁R₂, W is CR₄R₅, V is CR₃, Y is O, n is 2, and m is 3 or 4. In another embodiment of Formula (II), ring A is phenyl, p is 0, T is CR₁R₂, W is CR₄R₅, V is CR₃, Y is O, n is 3, and m is 3 or 4. In another embodiment of Formula (II), p is 0 and R₁, R₂, R₄, and R₅ are each H. In another embodiment of Formula (II), p is 0; R₁, R₂, R₄, and R₅ are each H; and R₃ is H. In another embodiment of Formula (II), p is 0; R₁, R₂, R₄, and R₅ are each H; R₃ is H; and R₆, R₇, R₈, R₉, and R₁₁ are each H. In another embodiment of Formula (II), p is 0; R₁, R₂, R₄, and R₅ are each H; R₃ is H; R₆, R₇, R₈, R₉, and R₁₁ are each H; and R₁₂ and R₁₃ are each H.

In another embodiment of Formula (II), p is 1 and R₁, R₂, R₄, and R₅ are each H. In another embodiment of Formula (II), p is 1; R₁, R₂, R₄, and R₅ are each H; and R₃ is H. In another embodiment of Formula (II), p is 1; R₁, R₂, R₄, and R₅ are each H; R₃ is H; and R₆, R₇, R₈, R₉, and R₁₁ are each H. In another embodiment of Formula (II), p is 1; R₁, R₂, R₄, and R₅ are each H; R₃ is H; R₆, R₇, R₈, R₉, and R₁₁ are each H; and R₁₂ and R₁₃ are each H.

In another embodiment of Formula (II), p is 2 and R₁, R₂, R₄, and R₅ are each H. In another embodiment of Formula (II), p is 2; R₁, R₂, R₄, and R₅ are each H; and R₃ is H. In another embodiment of Formula (II), p is 2; R₁, R₂, R₄, and R₅ are each H; R₃ is H; and R₆, R₇, R₈, R₉, and R₁₁ are each H. In another embodiment of Formula (II), p is 2; R₁, R₂, R₄, and R₅ are each H; R₃ is H; R₆, R₇, R₈, R₉, and R₁₁ are each H; and R₁₂ and R₁₃ are each H.

In another embodiment of Formula (II), p is 1, 2, 3, or 4 and R is fluorine. In another embodiment of Formula (II), p is 1, 2, 3, or 4 and R is deuterium. In another embodiment of Formula (II), p is 1, 2, 3, or 4 and each R is, independently, selected from the group consisting of hydroxyl, cyano, unsubstituted C₁-C₃ alkyl, and C₁-C₃ alkyl substituted with one or more halogen or deuterium. In another embodiment of Formula (II), p is 1, 2, 3, or 4 and each R is, independently, selected from the group consisting of cyano, unsubstituted C₁-C₃ alkyl, and C₁-C₃ alkyl substituted with one or more halogen or deuterium. In another embodiment of Formula (II), p is 1 and R is unsubstituted C₁-C₃ alkyl. In another embodiment of Formula (II), p is 1 and R is methyl. In another embodiment of Formula (II), p is 1 or 2 and each R is methyl. In another embodiment of Formula (II), p is 1 and R is C₁-C₃ alkyl substituted with one or more halogen. In another embodiment of Formula (II), p is 1 and R is CF₃. In another embodiment of Formula (II), p is 1 or 2 and each R is CF₃.

In another embodiment of Formula (II), one or more of R₁, R₂, R₄, and R₅ is fluorine. In another embodiment of Formula (II), one or more of R₁, R₂, R₄, and R₅ is deuterium. In another embodiment of Formula (II), one or more of R₆, R₇, R₈, R₉, and R₁₁ is fluorine. In another embodiment of Formula (II), one or more of R₆, R₇, R₈, R₉, and R₁₁ is deuterium. In another embodiment of Formula (II), one or more of each R₁₂ and R₁₃ is fluorine. In another embodiment of Formula (II), one or more of each R₁₂ and R₁₃ is deuterium.

In another embodiment of Formula (II), Y is O, T is CR₁R₂, V is CR₃, W is CR₄R₅, and R₁₁ is H. In another embodiment of Formula (II), Y is O, T is CR₁R₂, V is CR₃, W is CR₄R₅, R₁₁ is H, and m is 2. In another embodiment of Formula (II), Y is O, T is CR₁R₂, V is CR₃, W is CR₄R₅, and each of R₁₁, R₁₄, R₁₅, R₁₆, R₁₇, and R₁₈ is H. In another embodiment of Formula (II), Y is O, T is CR₁R₂, V is CR₃, W is CR₄R₅, each of R₁₁, R₁₄, R₁₅, R₁₆, R₁₇, and R₁₈ is H, and m is 2. In another embodiment of Formula (II), Y is O, T is CR₁R₂, V is CR₃, W is CR₄R₅, and each of R₁₁, R₁₂, R₁₃, R₁₄, R₁₅, R₁₆, R₁₇, and R₁₈ is H. In another embodiment of Formula (II), Y is O, T is CR₁R₂, V is CR₃, W is CR₄R₅, each of R₁₁, R₁₂, R₁₃, R₁₄, R₁₅, R₁₆, R₁₇, and R₁₈ is H, and m is 2.

Each of the embodiments described herein with respect to compounds of Formula II also applies to compounds of Formula II-A.

According to Formula I-A, I, II-A, or II herein, when ring A is pyridinyl, the position of the pyridinyl N atom is specified as shown below:

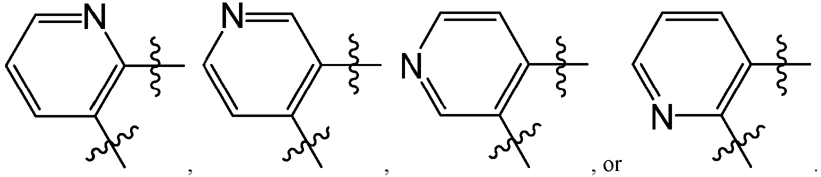

Further, according to Formula I-A, I, II-A, or II herein, when ring A is pyridazinyl, the positions of the pyridazinyl N atoms are specified as shown below:

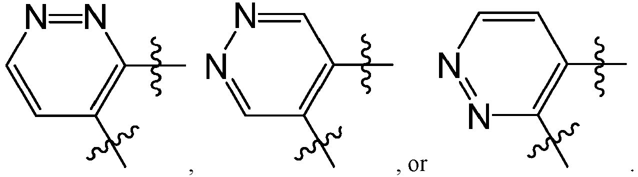

Further, according to Formula I-A, I, II-A, or II herein, when ring A is pyrimidinyl, the positions of the pyrimidinyl N atoms are specified as shown below:

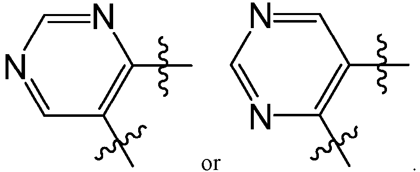

Further, according to Formula I-A, I, II-A, or II herein, when ring A is pyrazinyl, the positions of the pyrazinyl N atoms are specified as shown below:

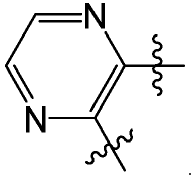

Further, according to Formula I-A, I, II-A, or II herein, when ring A is triazinyl, the positions of the triazinyl N atoms are specified as shown below:

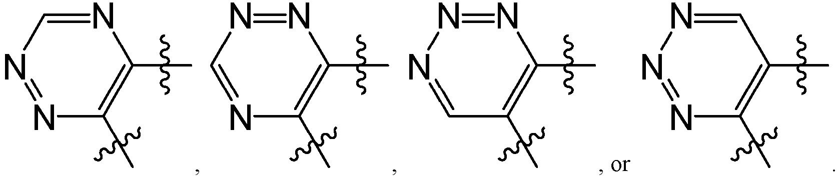

All other variables described in Formula I-A, I. II-A, or II are as defined above.

Certain embodiments of compounds of Formula I-A, I, II-A, II or pharmaceutically acceptable salts thereof, are shown below in Table 1. Compounds of Formula I-A, I, II-A, II or pharmaceutically acceptable salts thereof, and compounds of Table 1, or pharmaceutically acceptable salts thereof, collectively or individually are sometimes referred to herein as "compounds of the invention" or "compounds provided herein".

**Table 1.**

| **Structure** | **Compound No.** |
|---|---|
| 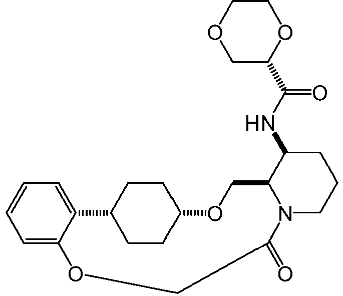 | 1 |
| 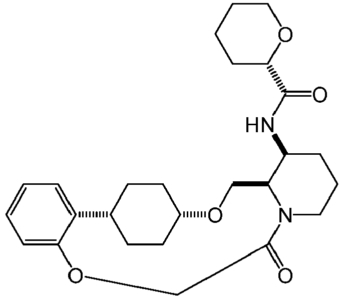 | 2 |
| 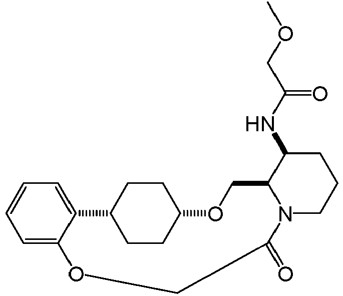 | 3 |
| 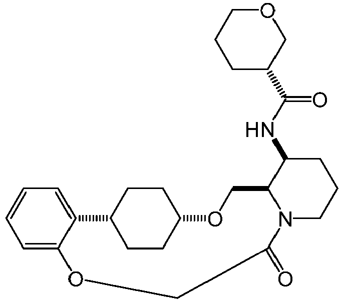 | 4 |
| 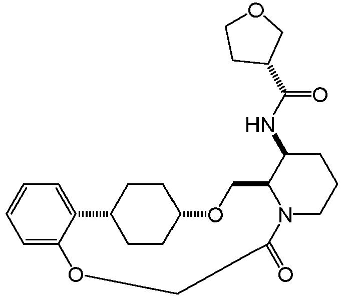 | 5 |
| 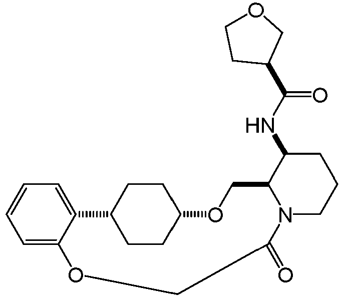 | 6 |
| 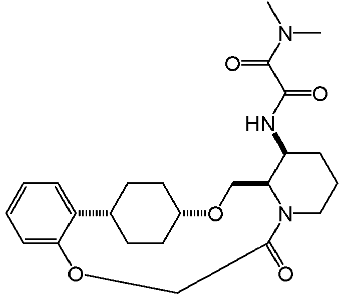 | 7 |
| 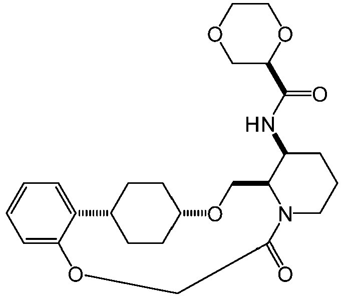 | 8 |
| 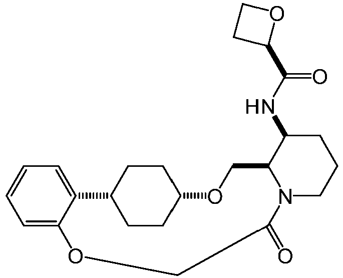 | 9 |
| 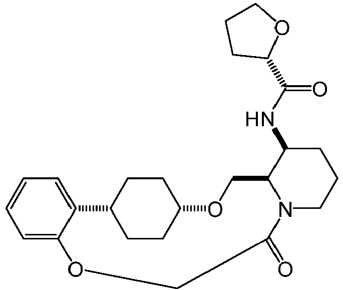 | 10 |
| 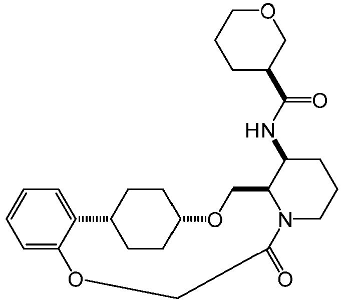 | 11 |
| 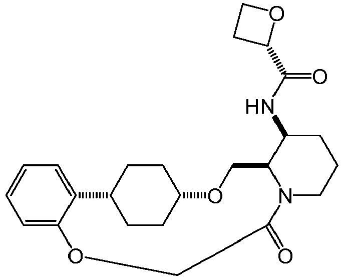 | 12 |
| 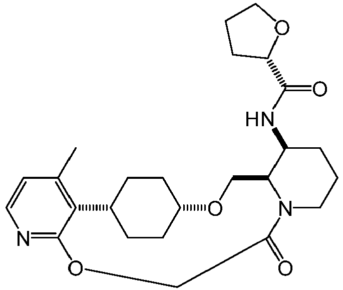 | 13 |
| 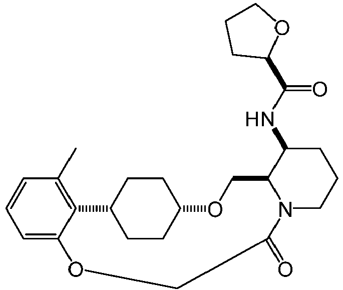 | 14 |
| 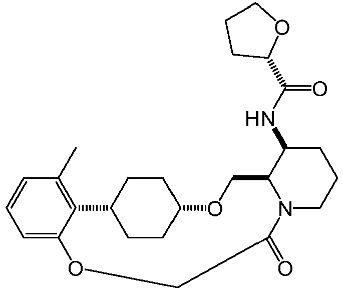 | 15 |
| 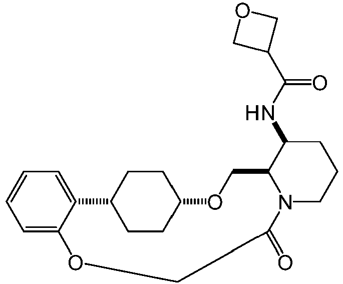 | 16 |
| 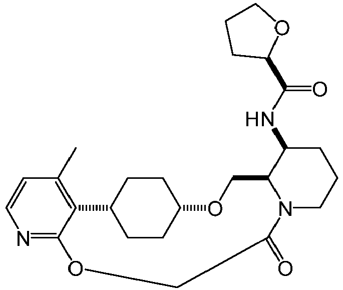 | 17 |
| 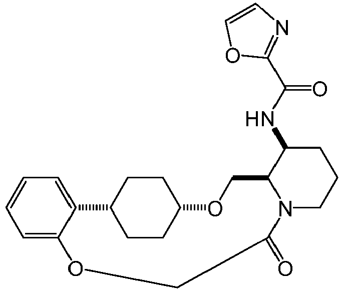 | 18 |
| 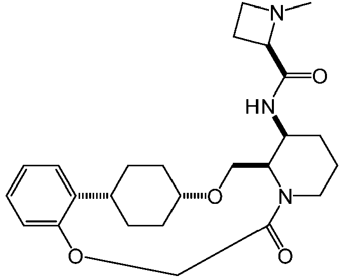 | 19 |
| 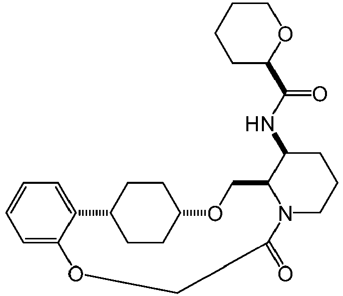 | 20 |
| 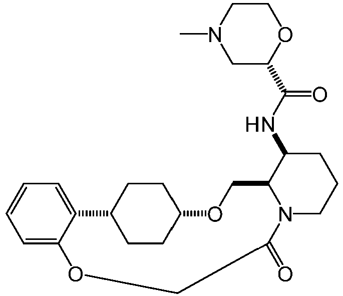 | 21 |
| 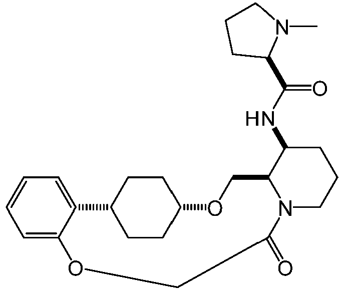 | 22 |
| 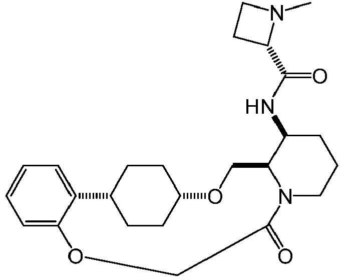 | 23 |
| 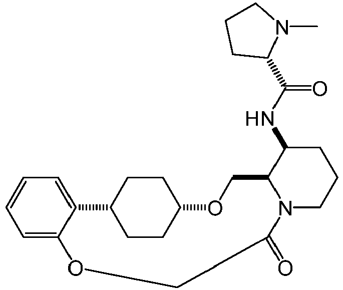 | 24 |
| 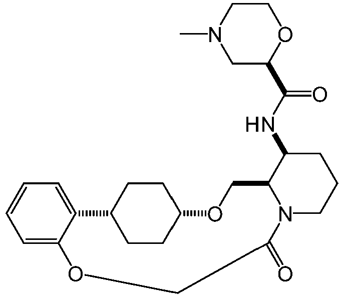 | 25 |
| 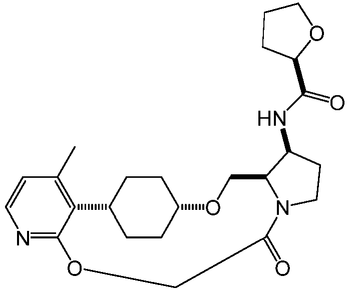 | 26 |
| 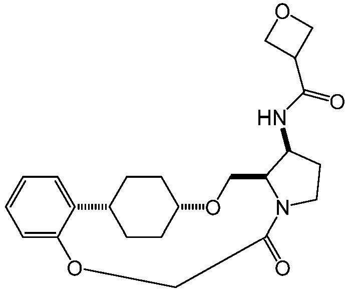 | 27 |
| 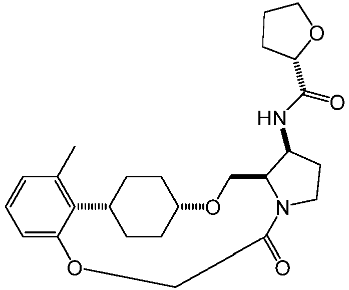 | 28 |
| 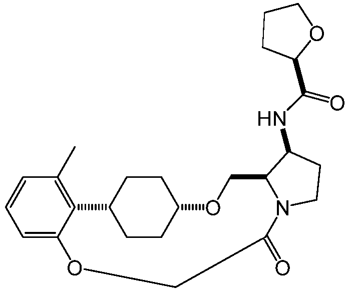 | 29 |
| 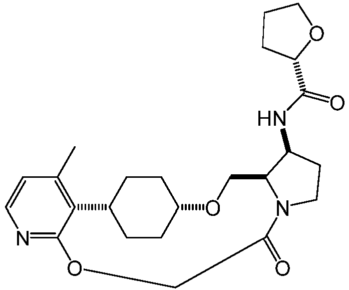 | 30 |
| 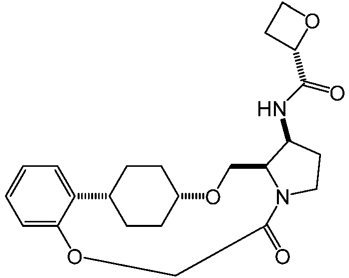 | 31 |
| 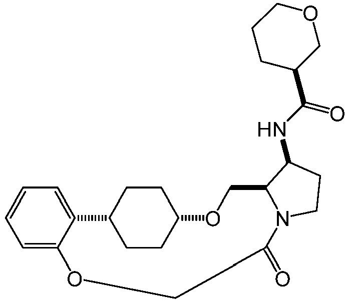 | 32 |
| 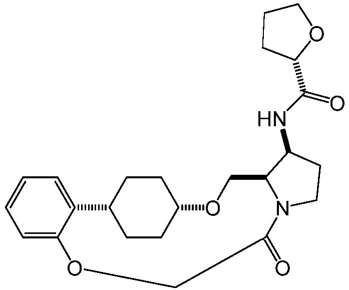 | 33 |
| 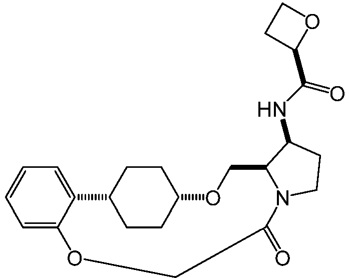 | 34 |
| 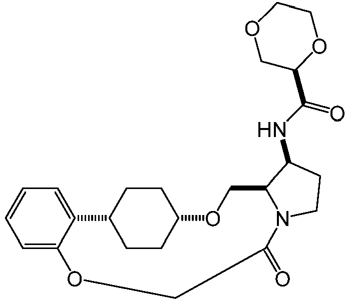 | 35 |
| 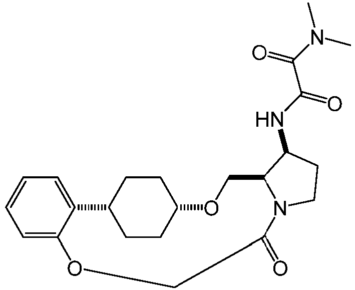 | 36 |
| 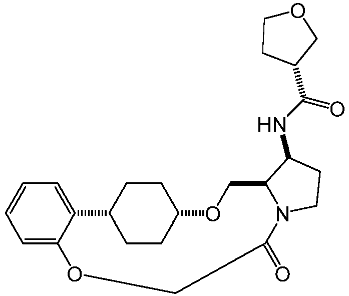 | 37 |
| 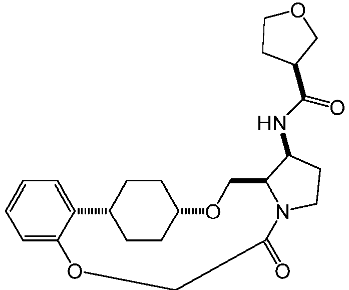 | 38 |
| 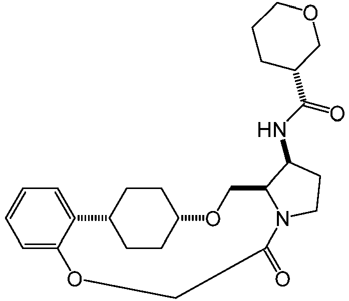 | 39 |
| 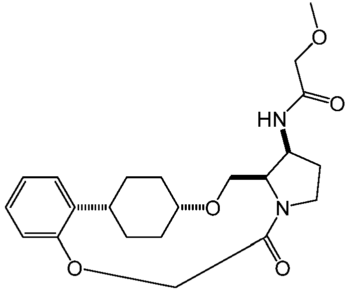 | 40 |
| 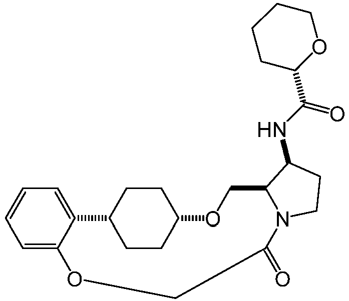 | 41 |
| 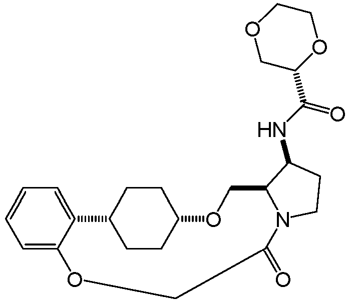 | 42 |
| 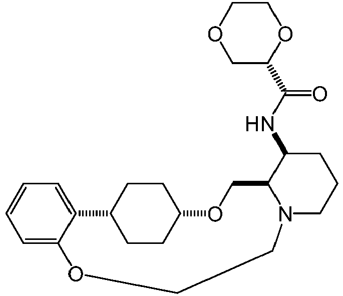 | 43 |
| 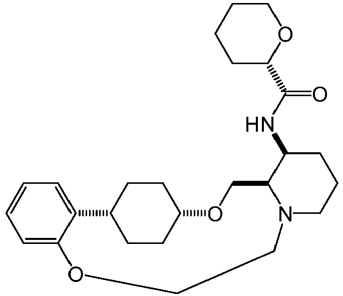 | 44 |
| 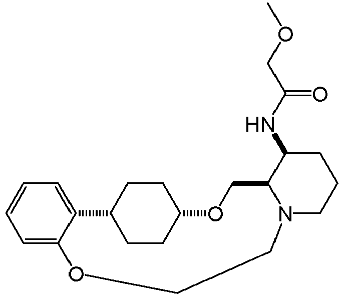 | 45 |
| 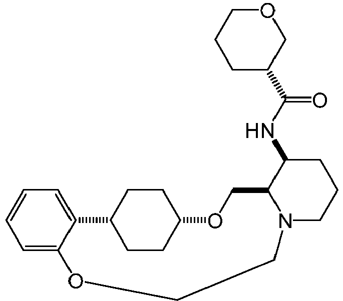 | 46 |
| 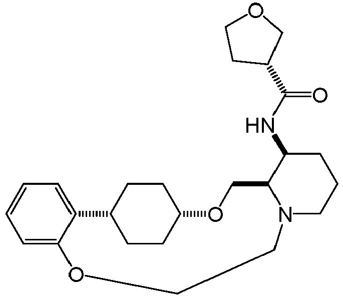 | 47 |
| 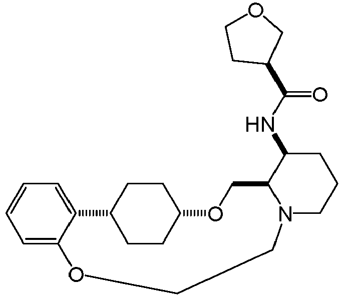 | 48 |
| 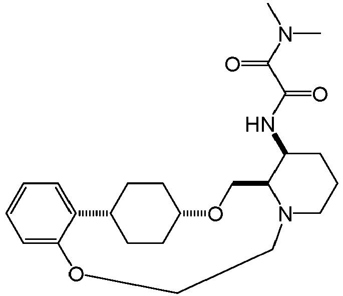 | 49 |
| 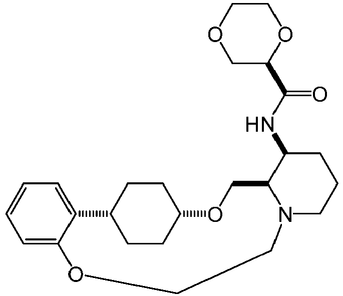 | 50 |
| 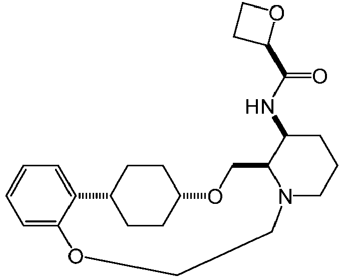 | 51 |
| 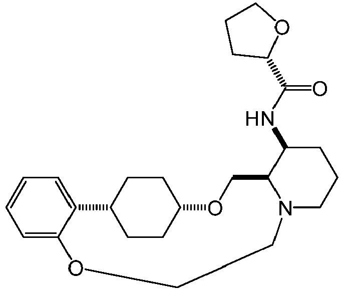 | 52 |
| 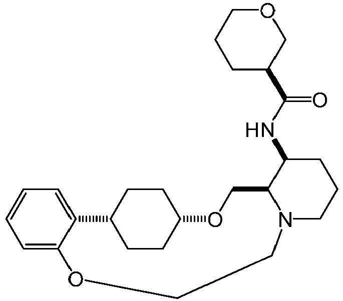 | 53 |
| 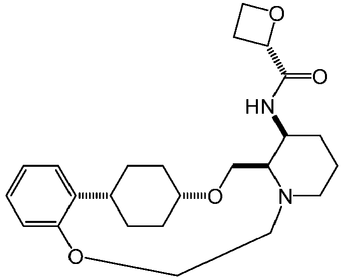 | 54 |
| 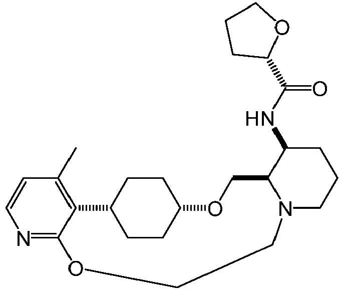 | 55 |
| 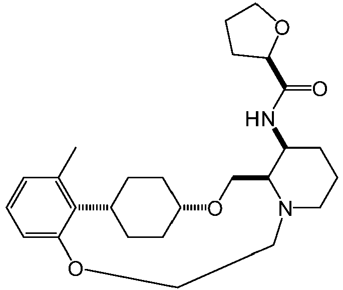 | 56 |
| 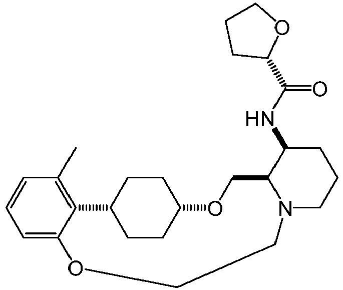 | 57 |
| 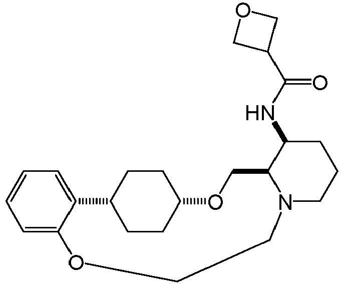 | 58 |
| 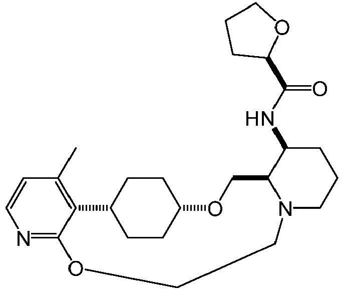 | 59 |
| 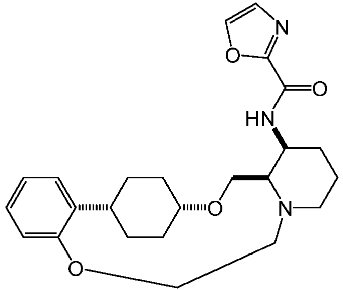 | 60 |
| 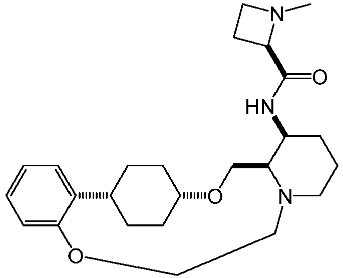 | 61 |
| 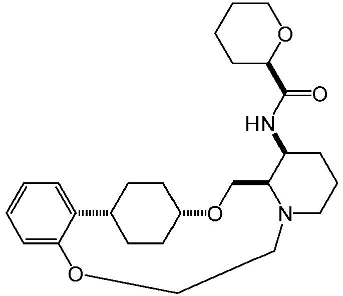 | 62 |
| 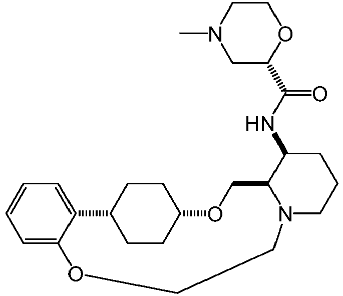 | 63 |
| 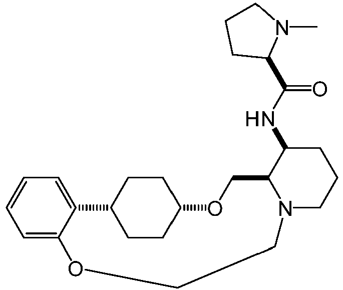 | 64 |
| 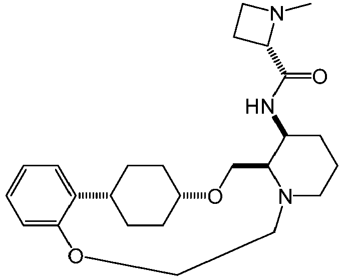 | 65 |
| 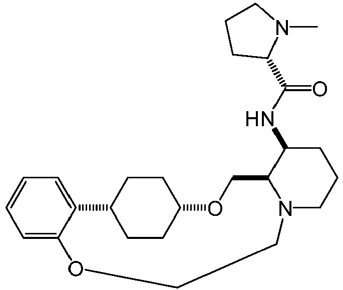 | 66 |
| 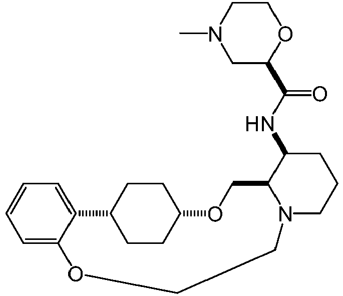 | 67 |
| 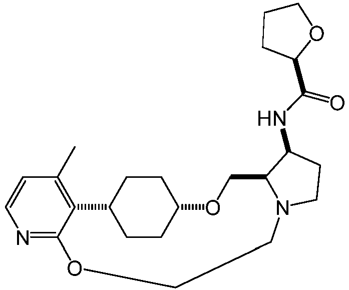 | 68 |
| 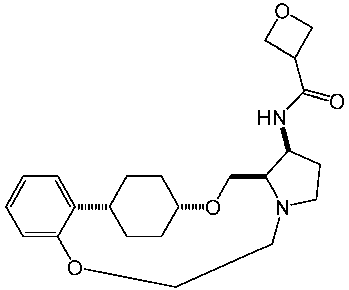 | 69 |
| 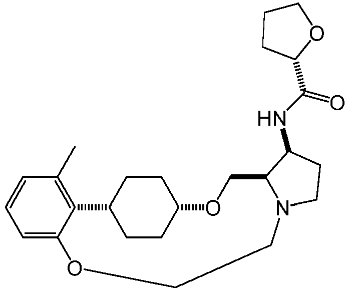 | 70 |
| 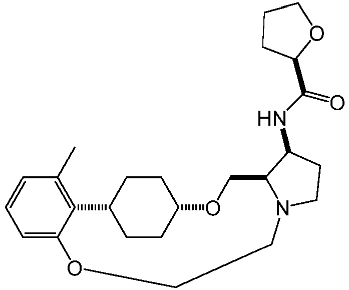 | 71 |
| 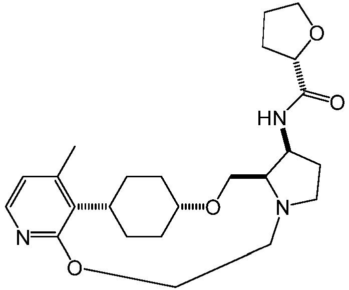 | 72 |
| 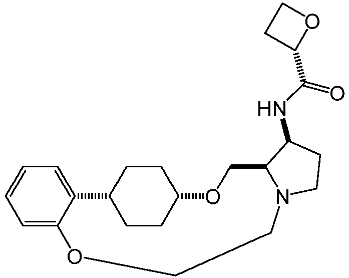 | 73 |
| 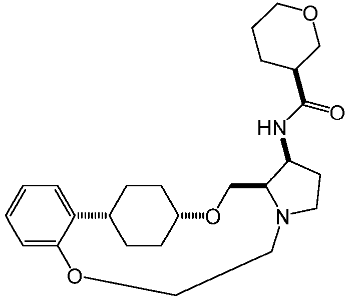 | 74 |
| 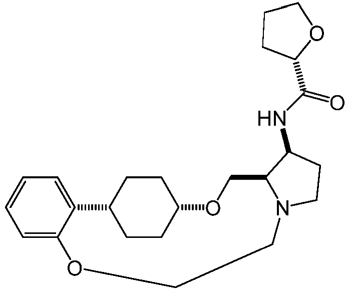 | 75 |
| 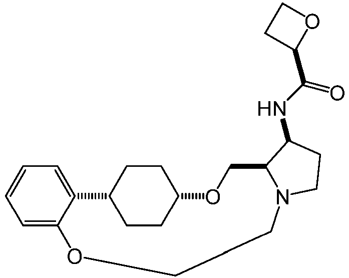 | 76 |
| 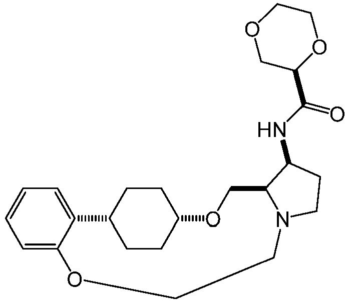 | 77 |
| 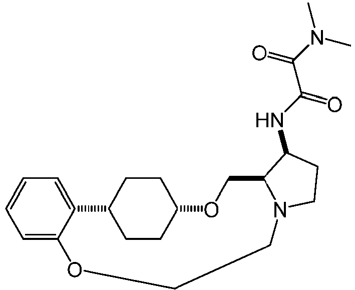 | 78 |
| 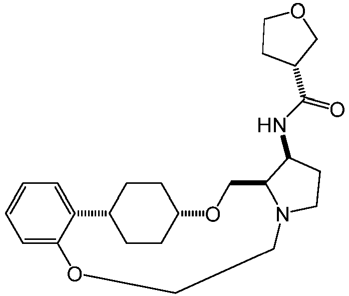 | 79 |
| 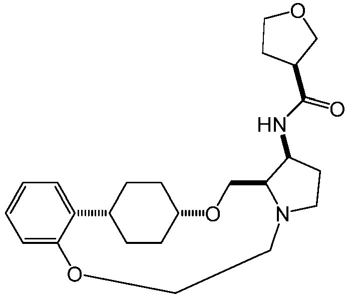 | 80 |
| 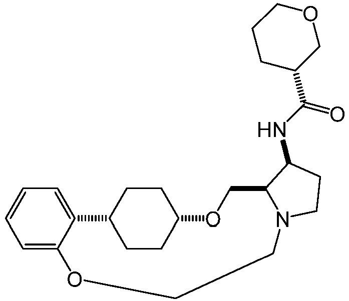 | 81 |
| 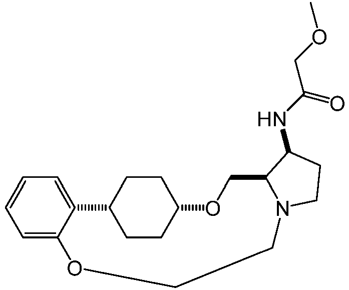 | 82 |
| 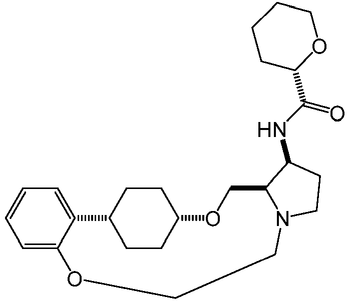 | 83 |
| 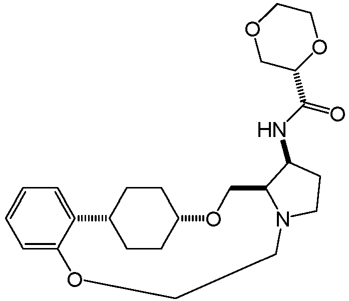 | 84 |
| 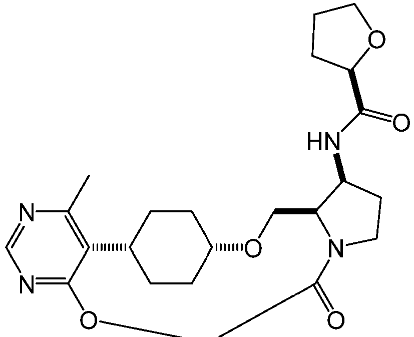 | 85 |
| 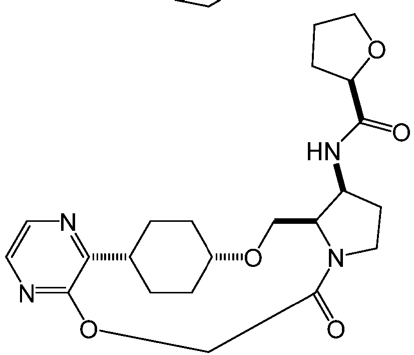 | 86 |
| 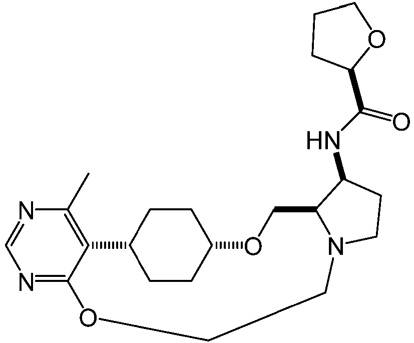 | 87 |
| 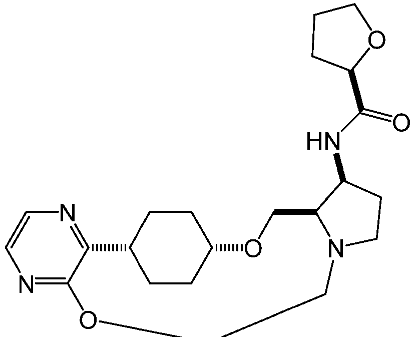 | 88 |
| 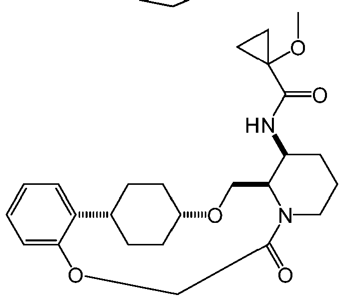 | 89 |

The disclosed compounds possess one or more stereocenters, and each stereocenter may exist independently in either the R or S configuration. In one embodiment, compounds described herein are present in optically active or racemic forms. It is to be understood that the compounds described herein encompass racemic, optically-active, regioisomeric and stereoisomeric forms, or combinations thereof that possess the therapeutically useful properties described herein.

Preparation of optically active forms is achieved in any suitable manner, including by way of non-limiting example, by resolution of the racemic form with recrystallization techniques, synthesis from optically-active starting materials, chiral synthesis, or chromatographic separation using a chiral stationary phase. In one embodiment, a mixture of two or more isomers is utilized as the disclosed compound described herein. In another embodiment, a pure isomer is utilized as the disclosed compound described herein. In another embodiment, compounds described herein contain one or more chiral centers. These compounds are prepared by any means, including stereoselective synthesis, enantioselective synthesis or separation of a mixture of enantiomers or diastereomers. Resolution of compounds and isomers thereof is achieved by any means including, by way of non-limiting example, chemical processes, enzymatic processes, fractional crystallization, distillation, and chromatography.

In one embodiment, the disclosed compounds may exist as tautomers. All tautomers are included within the scope of the compounds presented herein.

Compounds described herein also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described herein include and are not limited to ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, and ³⁵S. In one embodiment, isotopically-labeled compounds are useful in drug or substrate tissue distribution studies. In another embodiment, substitution with heavier isotopes such as deuterium affords greater metabolic stability (for example, increased in vivo half-life or reduced dosage requirements). In another embodiment, the compounds described herein include a ²H (*i.e.*, deuterium) isotope.

In yet another embodiment, substitution with positron emitting isotopes, such as ¹¹C, ¹⁸F, ¹⁵O and ¹³N, is useful in Positron Emission Topography (PET) studies for examining substrate receptor occupancy. Isotopically-labeled compounds are prepared by any suitable method or by processes using an appropriate isotopically-labeled reagent in place of the non-labeled reagent otherwise employed.

The specific compounds described herein, and other compounds encompassed by one or more of the Formulas described herein having different substituents are synthesized using techniques and materials described herein and as described, for example, in Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), Larock's Comprehensive Organic Transformations (VCH Publishers Inc., 1989), March, Advanced Organic Chemistry 4th Ed., (Wiley 1992); Carey and Sundberg, Advanced Organic Chemistry 4th Ed., Vols. A and B (Plenum 2000, 2001), and Green and Wuts, Protective Groups in Organic Synthesis 3rd Ed., (Wiley 1999).

General methods for the preparation of compounds as described herein are modified by the use of appropriate reagents and conditions, for the introduction of the various moieties found in the Formulas as provided herein.

Compounds described herein are synthesized using any suitable procedures starting from compounds that are available from commercial sources or are prepared using procedures described herein.

### Methods of Treatment

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The compounds of the invention can be used in a method of treating a disease or condition in a subject, said method comprising administering to the subject a compound of the invention, or a pharmaceutical composition comprising a compound of the invention. In one embodiment of the methods described herein, the subject is human. In one aspect, the compounds provided herein are useful in treatment of a disease or condition by acting as an agonist of the orexin-2 receptor.

The compounds of the invention can be used to treat a disease or condition selected from the group consisting of narcolepsy, cataplexy, or hypersomnia in a subject in need thereof.

In one embodiment, the compounds of the invention can be used to treat narcolepsy in a subject. In one embodiment, the compounds of the invention can be used to treat cataplexy in a subject. In one embodiment, the compounds of the invention can be used to treat hypersomnia in a subject.

Orexin-2 receptors are important in a wide range of biological functions. This suggests that orexin-2 receptors play a role in diverse disease processes in humans or other species. The compound of the present invention is useful for treating, preventing, or ameliorating the risk of one or more of the following symptoms or diseases of various neurological and psychiatric diseases associated with alterations in sleep/wake function. That is, narcolepsy, narcolepsy with cataplexy, idiopathic hypersomnia, hypersomnia, sleep apnea syndrome, narcolepsy syndrome, hypersomnolence syndrome characterized by hypersomnia (e.g., in subjects with Kleine Levin syndrome, major depression with hypersomnia, Lewy body dementia, Parkinson's disease, progressive supranuclear paralysis, Prader-Willi syndrome, Mobius syndrome, hypoventilation syndrome, Niemann-Pick disease type C, brain contusion, cerebral infarction, brain tumor, muscular dystrophy, multiple sclerosis, multiple systems atrophy, acute disseminated encephalomyelitis, Guillain-Barre syndrome, Rasmussen's encephalitis, Wernicke's encephalitis, limbic encephalitis, or Hashimoto's encephalopathy), coma, loss of consciousness, obesity (e.g., malignant mastocytosis, exogenous obesity, hyperinsulinar obesity, hyperplasmic obesity, hypop hyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, or central obesity), insulin resistance syndrome, Alzheimer's disease, disturbance of consciousness such as coma and the like, side effects and complications due to anesthesia, sleep disturbance, excessive daytime sleepiness, sleep problem, insomnia, intermittent sleep, nocturnal myoclonus, REM sleep interruption, jet lag, jet lag syndrome, sleep disorder of alternating worker, sleep disorder, night terror, depression, major depression, sleepwalking disease, enuresis, sleep disorder, Alzheimer's dusk, sundowning, diseases associated with circadian rhythm, fibromyalgia, condition arising from decline in the quality of sleep, overeating, obsessive compulsive eating disorder, obesity-related disease, hypertension, diabetes, elevated plasma insulin concentration and insulin resistance, hyperlipidemia, hyperlipemia, endometrial cancer, breast cancer, prostate cancer, colorectal cancer, cancer, osteoarthritis, obstructive sleep apnea, cholelithiasis, gallstones, cardiac disease, abnormal heartbeat, arrhythmia, myocardial infarction, congestive cardiac failure, cardiac failure, coronary heart disease, cardiovascular disorder, polycysticovarian disease, craniopharingioma, Prader-Willi syndrome, Froelich's syndrome, growth hormone deficient, normal mutant short stature, Tumer's syndrome, children suffering from acute lymphoblastic leukemia, syndrome X, reproductive hormone abnormality, declining fertility, infertility, male gonadal function decline, sexual and reproductive dysfunction such as female male hirsutism, fetal defects associated with pregnant women obesity, gastrointestinal motility disorders such as obesity-related gastroesophageal reflux, obesity hypoventilation syndrome (Pickwick syndrome), respiratory diseases such as dyspnea, inflammation such as systemic inflammation of the vascular system, arteriosclerosis, hypercholesterolemia, hyperuricemia, lower back pain, gall bladder disease, gout, kidney cancer, risk of secondary outcomes of obesity, such as lowering the risk of left ventricular hypertrophy, migraine pain, headache, neuropathic pain, Parkinson's disease, psychosis, autoimmune encephalitis, cancer related fatigue (such as excessive daytime sleepiness or fatigue associated with cancer and/or chemotherapy), cancer related nausea and vomiting, corticobasal degeneration, Huntington's disease, neuromyelitis optica, nociception, progressive supranuclear palsy, schizophrenia, systemic lupus erythematosus, traumatic brain injury, facial flushing, night sweats, diseases of the genital/urinary system, diseases related to sexual function or fertility, dysthymic disorder, bipolar disorder, bipolar I disorder, bipolar II disorder, cyclothymic disorder, acute stress disorder, agoraphobia, generalized anxiety disorder, obsessive disorder, panic attack, panic disorder, post-traumatic stress disorder (PTSD), separation anxiety disorder, social phobia, anxiety disorder, acute neurological and psychiatric disorders such as cardiac bypass surgery and post-transplant cerebral deficit, stroke, ischemic stroke, cerebral ischemia, spinal cord trauma, head trauma, perinatal hypoxia, cardiac arrest, hypoglycemic nerve injury, Huntington's chorea, amyotrophic lateral sclerosis, eye damage, retinopathy, cognitive impairment, muscle spasm, tremor, epilepsy, disorders associated with muscle spasticity, delirium, amnestic disorder, age-related cognitive decline, schizoaffective disorder, delusional disorder, drug addiction, dyskinesia, chronic fatigue syndrome, fatigue, medication-induced Parkinsonism syndrome, Jill-do La Tourette's syndrome, chorea, myoclonus, tic, restless legs syndrome, dystonia, dyskinesia, attention deficit hyperactivity disorder (ADHD), behavior disorder, urinary incontinence, withdrawal symptoms, trigeminal neuralgia, hearing loss, tinnitus, nerve damage, retinopathy, macular degeneration, vomiting, cerebral edema, pain, bone pain, arthralgia, toothache, cataplexy, and traumatic brain injury (TBI).

Particularly, the compound of the present invention is useful as a therapeutic or prophylactic drug for narcolepsy, idiopathic hypersomnia, hypersomnia, sleep apnea syndrome, narcolepsy syndrome, hypersomnolence syndrome characterized by hypersomnia (e.g., in Parkinson's disease, Guillain-Barre syndrome or Kleine Levin syndrome), Alzheimer's disease, obesity, insulin resistance syndrome, cardiac failure, diseases related to bone loss, sepsis, disturbance of consciousness such as coma and the like, side effects and complications due to anesthesia, and the like, or anesthetic antagonist.

In one embodiment, the compound of the present invention has orexin-2 receptor agonist activity and is useful as a prophylactic or therapeutic agent for narcolepsy.

In another embodiment, the compound of the present invention is useful as a prophylactic or therapeutic agent for narcolepsy type-1. In another embodiment, the compound of the present invention is useful as a prophylactic or therapeutic agent for narcolepsy type-2. In another embodiment, the compound of the present invention is useful as a prophylactic or therapeutic agent for narcolepsy and excessive daytime sleepiness. In another embodiment, the compound of the present invention is useful as a prophylactic or therapeutic agent for narcolepsy, cataplexy, and excessive daytime sleepiness. In another embodiment, the compound of the present invention is useful as a prophylactic or therapeutic agent for narcolepsy and cataplexy. In another embodiment, the compound of the present invention is useful as a prophylactic or therapeutic agent for excessive daytime sleepiness. In another embodiment, the compound of the present invention is useful as a prophylactic or therapeutic agent for idiopathic hypersomnia. In another embodiment, the compound of the present invention is useful as a prophylactic or therapeutic agent for obstructive sleep apnea.

In another embodiment, the compound of the present invention has orexin-2 receptor agonist activity and is useful as a prophylactic or therapeutic agent for hypersomnia in Parkinson's disease.

In another embodiment, the compound of the present invention has orexin-2 receptor agonist activity and is useful as a prophylactic or therapeutic agent for hypersomnia. In another embodiment, the compound of the present invention has orexin-2 receptor agonist activity and is useful as a prophylactic or therapeutic agent for excessive daytime sleepiness associated with Parkinson's disease.

In another embodiment, the compound of the present invention has orexin-2 receptor agonist activity, and is useful as a prophylactic or therapeutic agent for excessive daytime sleepiness or fatigue associated with cancer and/or chemotherapy.

In another embodiment, the present disclosure provides a method of treating narcolepsy in a subject in need thereof comprising administering to the subject a compound of Formula I-A, I, II-A, or II, or a pharmaceutically acceptable salt thereof.

In another embodiment, the present disclosure provides a method of treating narcolepsy type-1 in a subject in need thereof comprising administering to the subject a compound of Formula I-A, I, II-A, or II, or a pharmaceutically acceptable salt thereof.

In another embodiment, the present disclosure provides a method of treating narcolepsy type-2 in a subject in need thereof comprising administering to the subject a compound of Formula I-A, I, II-A, or II, or a pharmaceutically acceptable salt thereof.

In another embodiment, the present disclosure provides a method of treating narcolepsy and excessive daytime sleepiness in a subject in need thereof comprising administering to the subject a compound of Formula I-A, I, II-A, or II, or a pharmaceutically acceptable salt thereof.

In another embodiment, the present disclosure provides a method of treating narcolepsy, cataplexy, and excessive daytime sleepiness in a subject in need thereof comprising administering to the subject a compound of Formula I-A, I, II-A, or II, or a pharmaceutically acceptable salt thereof.

In another embodiment, the present disclosure provides a method of treating narcolepsy and cataplexy in a subject in need thereof comprising administering to the subject a compound of Formula I-A, I, II-A, or II, or a pharmaceutically acceptable salt thereof.

In another embodiment, the present disclosure provides a method of treating excessive daytime sleepiness in a subject in need thereof comprising administering to the subject a compound of Formula I-A, I, II-A, or II, or a pharmaceutically acceptable salt thereof.

In another embodiment, the present disclosure provides a method of treating idiopathic hypersomnia in a subject in need thereof comprising administering to the subject a compound of Formula I-A, I, II-A, or II, or a pharmaceutically acceptable salt thereof.

In another embodiment, the present disclosure provides a method of treating excessive daytime sleepiness and idiopathic hypersomnia in a subject in need thereof comprising administering to the subject a compound of Formula I-A, I, II-A, or II, or a pharmaceutically acceptable salt thereof.

In another embodiment, the present disclosure provides a method of treating obstructive sleep apnea in a subject in need thereof comprising administering to the subject a compound of Formula I-A, I, II-A, or II, or a pharmaceutically acceptable salt thereof.

In another embodiment, the present disclosure provides a method of treating excessive daytime sleepiness and obstructive sleep apnea in a subject in need thereof comprising administering to the subject a compound of Formula I-A, I, II-A, or II, or a pharmaceutically acceptable salt thereof.

In any of the methods as described herein, the subject is administered a compound of Formula I. In any of the methods as described herein, the subject is administered a compound of Formula II.

Each of the embodiments described herein with respect to the use of compounds of Formula I also applies to compounds of Formula I-A. Each of the embodiments described herein with respect to the use of compounds of Formula II also applies to compounds of Formula II-A.

In any of the compositions or methods as described herein, the compound of Formula I-A, I, II-A, II, or a pharmaceutically acceptable salt thereof, is present and/or administered in a therapeutically effective amount.

### Administration / Dosage / Formulations

In another aspect, provided herein is a pharmaceutical composition comprising at least one compound of the invention, together with a pharmaceutically acceptable carrier.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

In particular, the selected dosage level will depend upon a variety of factors including the activity of the particular compound employed, the time of administration, the rate of excretion of the compound, the duration of the treatment, other drugs, compounds or materials used in combination with the compound, the age, sex, weight, condition, general health and prior medical history of the patient being treated, and like factors well, known in the medical arts.

A medical doctor, e.g., physician or veterinarian, having ordinary skill in the art may readily determine and prescribe the effective amount of the pharmaceutical composition required. For example, the physician or veterinarian could begin administration of the pharmaceutical composition to dose the disclosed compound at levels lower than that required in order to achieve the desired therapeutic effect and gradually increase the dosage until the desired effect is achieved.

In particular embodiments, it is especially advantageous to formulate the compound in dosage unit form for ease of administration and uniformity of dosage. Dosage unit form as used herein refers to physically discrete units suited as unitary dosages for the patients to be treated; each unit containing a predetermined quantity of the disclosed compound calculated to produce the desired therapeutic effect in association with the required pharmaceutical vehicle. The dosage unit forms of the invention are dictated by and directly dependent on (a) the unique characteristics of the disclosed compound and the particular therapeutic effect to be achieved, and (b) the limitations inherent in the art of compounding/formulating such a disclosed compound for the treatment of narcolepsy or cataplexy in a patient.

In one embodiment, the compounds of the invention are formulated using one or more pharmaceutically acceptable excipients or carriers. In one embodiment, the pharmaceutical compositions of the invention comprise a therapeutically effective amount of a disclosed compound and a pharmaceutically acceptable carrier.

In some embodiments, the dose of a disclosed compound is from about 1 mg to about 1,000 mg. In some embodiments, a dose of a disclosed compound used in compositions described herein is less than about 1,000 mg, or less than about 800 mg, or less than about 600 mg, or less than about 500 mg, or less than about 300 mg, or less than about 200 mg, or less than about 100 mg, or less than about 50 mg, or less than about 20 mg, or less than about 10 mg. For example, a dose is about 10 mg, 20 mg, 25 mg, 30 mg, 40 mg, 50 mg, 60 mg, 70 mg, 80 mg, 90 mg, 100 mg, 120 mg, 140 mg, 160 mg, 180 mg, 200 mg, 220 mg, 240, 260 mg, 280 mg, 300 mg, 350 mg, 400 mg, 450 mg, 500 mg, 550 mg, or about 600 mg.

Routes of administration of any of the compositions of the invention include oral, nasal, rectal, intravaginal, parenteral, buccal, sublingual or topical. The compounds for use in the invention may be formulated for administration by any suitable route, such as for oral or parenteral, for example, transdermal, transmucosal (e.g., sublingual, lingual, (trans)buccal, (trans)urethral, vaginal (e.g., trans- and perivaginally), (intra)nasal and (trans)rectal), intravesical, intrapulmonary, intraduodenal, intragastrical, intrathecal, subcutaneous, intramuscular, intradermal, intra-arterial, intravenous, intrabronchial, inhalation, and topical administration. In one embodiment, the preferred route of administration is oral.

Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein.

For oral application, particularly suitable are tablets, dragees, liquids, drops, suppositories, or capsules, caplets and gelcaps. The compositions intended for oral use may be prepared according to any method known in the art and such compositions may contain one or more agents selected from the group consisting of inert, non-toxic pharmaceutically excipients that are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay the release of the active ingredients. Formulations for oral use may also be presented as hard gelatin capsules wherein the active ingredient is mixed with an inert diluent.

For parenteral administration, the disclosed compounds may be formulated for injection or infusion, for example, intravenous, intramuscular or subcutaneous injection or infusion, or for administration in a bolus dose or continuous infusion. Suspensions, solutions or emulsions in an oily or aqueous vehicle, optionally containing other formulatory agents such as suspending, stabilizing or dispersing agents may be used.

Those skilled in the art will recognize or be able to ascertain using no more than routine experimentation, numerous equivalents to the specific procedures, embodiments, claims, and examples described herein. Such equivalents are considered to be within the scope of this invention and covered by the claims appended hereto. For example, it should be understood, that modifications in reaction conditions, including but not limited to reaction times, reaction size/volume, and experimental reagents, such as solvents, catalysts, pressures, atmospheric conditions, *e.g*., nitrogen atmosphere, and reducing/oxidizing agents, with art-recognized alternatives and using no more than routine experimentation, are within the scope of the present application.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

The following examples further illustrate aspects of the present invention. However, they are in no way a limitation of the teachings or disclosure of the present invention as set forth herein.

### Examples

The invention is further illustrated by the following examples, which should not be construed as further limiting. The practice of the present invention will employ, unless otherwise indicated, conventional techniques of organic synthesis, cell biology, cell culture, molecular biology, transgenic biology, microbiology and immunology, which are within the skill of the art.

### General Procedures

### Example 1: Synthesis Procedures

Synthesis procedures for preparation of the compounds of the invention are readily available to the ordinary skilled artisan. Unless otherwise indicated, starting materials were generally obtained from commercial sources. Synthetic procedures for other macrocyclic compounds can be found, for example, in U.S. Application No.: 17/104,993 and in PCT

Application No.: PCT/US20/62320, both filed November 25, 2020.

The following abbreviations may be used in the synthetic examples below:
AcOH = acetic acid
DCM = dichloromethane
MsCl = methanesulfonyl chloride
MeOH = methanol
THF = tetrahydrofuran
EtOH = ethanol
PtO₂ = platinum dioxide
HATU = 1-[bis(dimethylamino)methylene]-1*H*-1,2,3-triazolo[4,5-b]pyridinium 3-oxide hexafluorophosphate
DIPEA or DIEA = *N,N*-diisopropylethylamine
^{t}Bu = *tert-*butyl
MeCN = acetonitrile
PE = petroleum ether
EtOAc = ethyl acetate
DMF = dimethyl formamide
TFA = trifluoroacetic acid
LiOH = lithium hydroxide
min = minutes
hr = hours
NaH = sodium hydride
Pd₂(dba)₃ = tris(dibenzylideneacetone)dipalladium(0)
DMSO = dimethyl sulfoxide
*i*-PrOH = isopropanol
Pd/C = palladium on carbon
XantPhos = 4,5-bis(diphenylphosphino)-9,9-dimethylxanthene
Boc = *tert*-butyloxycarbonyl
Ms = methanesulfonyl
Bn = benzyl
Et = ethyl
Cbz = carboxybenzyl
PMB =*para*-methoxybenzyl
DBU = 1,8-diazabicyclo[5.4.0]undec-7-ene
NBS = *N*-bromosuccinimide
Pd(dppf)Cl₂ = [1,1'-bis(diphenylphosphino)ferrocene]dichloropalladium(II)
DMAP = 4-(dimethylamino)pyridine
NCS = *N*-chlorosuccinimide
DMPU = 1,3-dimethyl-3,4,5,6-tetrahydro-2(1*H*)-pyrimidinone
LDA = lithium diisopropylamide
HOBt = 1-hydroxybenzotriazole
EDC = 1-ethyl-3-(3-dimethylaminopropyl)carbodiimide.

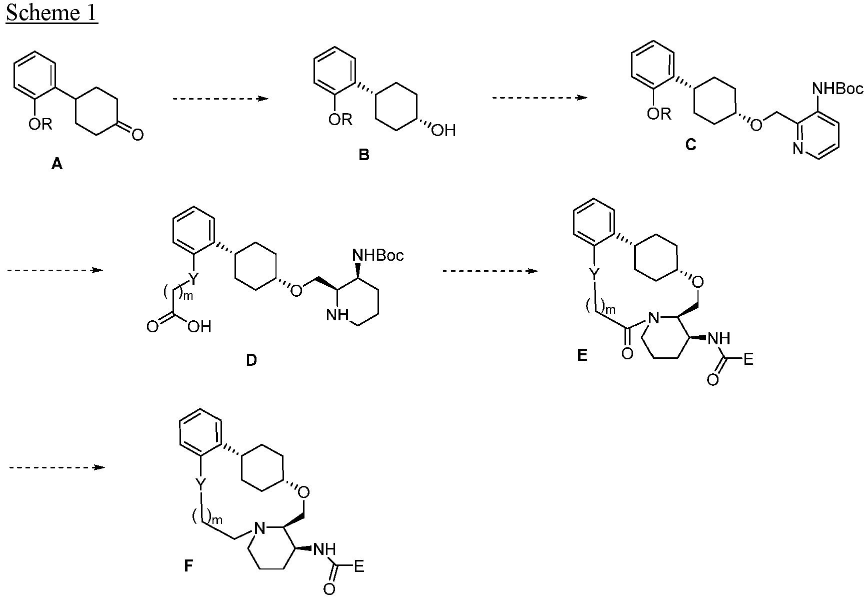

### Example 1.1

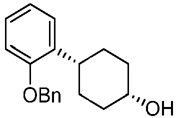

Into a 5 L 4-necked round-bottom flask purged and maintained with an inert atmosphere of nitrogen, was placed 4-[2-(benzyloxy)phenyl]cyclohexan-1-one (210 g, 0.75 mol, 1.0 equiv.) in tetrahydrofuran (2.1 L). This was followed by the addition of L-selectride (1 mol/L in THF) (1123 mL, 1.5 equiv.) dropwise with stirring at 0 degrees C. The resulting solution was stirred for 4 hr at room temperature. The reaction was then quenched by the addition of water/ice. The resulting solution was extracted with ethyl acetate, and the organic phase was washed with brine. The mixture was dried over anhydrous sodium sulfate, filtered, concentrated under vacuum. The residue was purified by silica gel column chromatography to give 137 g (64%) of (1*s*,4*s*)-4-[2-(benzyloxy)phenyl] cyclohexan-1-ol as a solid. ¹H NMR (400 MHz, CDCl₃): δ 7.45 - 7.26 (6H, m), 7.16 (1H, dd), 6.98 - 6.90 (2H, m), 5.09 (2H, s), 4.13 (1H, s), 3.12 - 3.02 (1H, m), 1.93 - 1.82 (4H, m), 1.73 - 1.41 (4H, m).

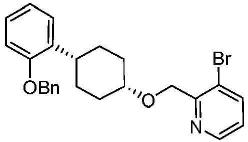

Into a 2 L 4-necked round-bottom flask purged and maintained with an inert atmosphere of nitrogen, was placed NaH (60% wt, 26.9 g, 2.0 equiv.) in tetrahydrofuran (200 mL). This was followed by the addition of a solution of (1*s*, 4*s*)-4-[2-(benzyloxy)phenyl]cyclohexan-1-ol (95.0 g, 336 mmol, 1.0 equiv.) in THF (200 mL) dropwise with stirring at 50-55 degrees C. After stirring for 2 hr, a solution of 3-bromo-2-(bromomethyl)pyridine (143.5 g, 571 mmol, 1.70 equiv.) in THF (550 mL) was added dropwise at 50-55 degrees C. The resulting solution was stirred for 14 hr at 50-55 degrees C. The reaction mixture was cooled. The reaction was then quenched by the addition of water. The resulting solution was extracted with ethyl acetate and the organic layers combined and dried over anhydrous sodium sulfate. The solids were filtered out. The resulting mixture was concentrated under vacuum. The residue was purified by silica gel column chromatography to give 94.0 g (62%) of 3-bromo-2-([[(1*s*,4*s*)-4-[2-(benzyloxy) phenyl]cyclohexyl]oxy]methyl)pyridine as a solid. ¹H NMR (400 MHz, CDCl₃): δ 8.57 (1H, d), 7.90 (1H, dd), 7.48 - 7.26 (6H, m), 7.18 - 7.14 (2H, m), 6.98 - 6.91 (2H, m), 5.12 (2H, s), 4.77 (2H, s), 3.86 (1H, s), 3.17 - 3.10 (1H, m), 2.20 - 2.15 (2H, m), 1.98 - 1.88 (2H, m), 1.69 - 1.57 (4H, m).

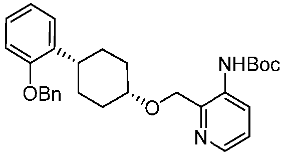

Into a 2 L 4-necked round-bottom flask purged and maintained with an inert atmosphere of nitrogen, was placed Xantphos (10.7 g, 18.0 mmol, 0.10 equiv.), Cs₂CO₃ (84 g, 258 mmol, 1.4 equiv.), 3-bromo-2-([[(1*s*,4*s*)-4-[2-(benzyloxy) phenyl]cyclohexyl]oxy]methyl)pyridine (84 g, 185 mmol, 1.0 equiv.), Pd₂(dba)₃ (8.5 g, 9.0 mmol, 0.05 equiv.) and *tert-*butyl carbamate (26 g, 222 mmol, 1.2 equiv.) in 1,4-dioxane (840 mL). The resulting solution was stirred for 5 hr at 100 degrees C. The solids were filtered out. The filtrate was concentrated under vacuum. The residue was purified by silica gel column chromatography to provide 74 g (82%) of *tert-*butyl *N*-[2-([[(1*s*,4*s*)- 4-[2-(benzyloxy)phenyl]cyclohexyl]oxy]methyl)pyridin-3-yl]carbamate as a solid.

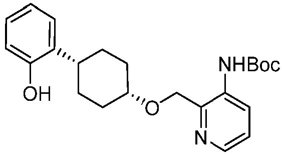

Into a 2 L 3-necked round-bottom flask, was placed *tert-*butyl *N*-[2-([[(1*s*,4*s*)- 4-[2-(benzyloxy)phenyl]cyclohexyl]oxy]methyl)pyridin-3-yl]carbamate (74 g, 151 mmol, 1.0 equiv.) and Pd/C (7.4 g, 10% wt) in ethyl alcohol (740 mL), then, hydrogen gas was bubbled through the resulting mixture. The resulting solution was stirred for 14 hr at room temperature. The solids were filtered out. The filtrate was concentrated under vacuum. The residue was purified by silica gel column chromatography to provide 51.4 g (85%) of *tert-*butyl N-[2-([[(1s,4s)-4-(2-hydroxyphenyl)cyclohexyl]oxy]methyl)pyridin-3-yl]carbamate as a solid. LCMS (ESI): *m*/*z* [M+H]⁺ = 399.1; ¹H NMR (300 MHz, CDCl₃): δ 8.65 (1H, s), 8.47 (1H, d), 8.19 (1H, q), 7.26 - 7.21 (1H, m), 7.09 - 7.03 (1H, m), 6.92 - 6.86 (1H, m), 6.75 (1H, q), 5.77 (1H, s), 4.84 (1H, s), 3.80 (1H, s), 2.94 - 2.93 (1H, m), 2.15 - 2.06 (2H, m), 1.88 - 1.47 (7H, m), 1.45 (9H, s), 1.26 (1H, d).

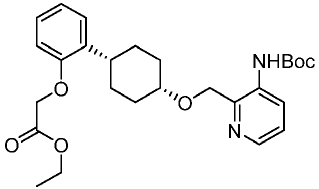

Into a 250 mL round-bottom flask purged and maintained with an inert atmosphere of nitrogen, was placed *tert-*butyl *N*-[2-([[(1*s*,4*s*)-4-(2-hydroxyphenyl)cyclohexyl]oxy]methyl)pyridin-3-yl]carbamate (8.0 g, 20.1 mmol, 1.0 equiv.), K₂CO₃ (14.0 g, 100.4 mmol, 5.0 equiv.), acetone (120 mL) and ethyl bromoacetate (5.03 g, 30.1 mmol, 1.5 equiv.). The resulting solution was stirred for 24 hr at 50 degrees C. The solids were filtered out. The filtrate was concentrated under vacuum. The residue was purified by silica gel column chromatography to provide ethyl 2-[2-[(1*s*,4*s*)-4-([3-[(*tert-*butoxycarbonyl)amino]pyridin-2- yl]methoxy)cyclohexyl]phenoxy]acetate (8.7 g, 89.4%) as an oil. LCMS (ESI): *m*/*z* [M+H]⁺ = 485.

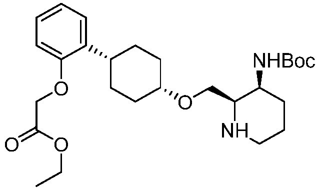

To a stirred mixture of ethyl 2-[2-[(1*s*,4*s*)-4-([3-[(*tert*-butoxycarbonyl)amino]pyridin-2-yl]methoxy)cyclohexyl]phenoxy]acetate (7.89 g, 16.3 mmol, 1.0 equiv.) in MeOH (142 mL) and AcOH (15.8 mL) were added PtO₂ (1.85 g, 8.14 mmol, 0.50 equiv.) at room temperature under hydrogen atmosphere. The resulting mixture was stirred for 2 hr at room temperature under hydrogen atmosphere. The resulting mixture was filtered, the filter cake was concentrated under reduced pressure. The reaction was quenched with sat. NaHCO₃ (aq.) at 0 degrees C. The resulting mixture was extracted with CH₂Cl₂ (3 x 500 mL). The combined organic layers were washed with brine (3 x 200 mL), dried over anhydrous Na₂SO₄. After filtration, the filtrate was concentrated under reduced pressure to afford diastereomeric *cis* and *trans* mixture (7 g, 88.7%) as a solid. The crude product was purified by Prep-TLC to afford cis-racemic mixture of ethyl 2-(2-((1*S*,4*s*)-4-((3-((*tert-*butoxycarbonyl)amino)piperidin-2-yl)methoxy)cyclohexyl)phenoxy)acetate (4.1 g) and *trans*-racemic mixture (1.7 g). LCMS (ESI): *m*/*z* [M+H]⁺ = 491.

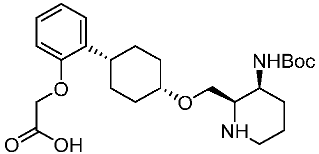

Into a 500 mL round-bottom flask purged and maintained with an atmosphere of nitrogen, was placed *cis*-racemic mixture of ethyl 2-(2-((1*S,4s*)-4-((3-((*tert-*butoxycarbonyl)amino)piperidin-2-yl)methoxy)cyclohexyl)phenoxy)acetate (4.1 g, 8.4 mmol, 1.0 equiv.), MeOH (30 mL), THF (60 mL), H₂O (30 mL ) and lithium hydroxide (83 mg, 3.5 mmol, 5.0 equiv.). The reaction was stirred for 2 hr at room temperature. The reaction was concentrated and the residue was purified by reverse phase chromatography to afford 2-(2-((1*s*,4*s*)-4-((3-((*tert*-butoxycarbonyl)amino)piperidin-2-yl)methoxy)cyclohexyl)phenoxy)acetic acid (2.35 g, 60.8%) as a solid. LCMS (ESI): *m*/*z* [M+H]⁺ = 463.

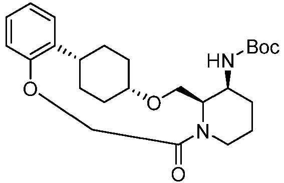

Into a 2000 mL round-bottom flask was added 2-(2-((1*s*,4*s*)-4-((3-((*tert-*butoxycarbonyl)amino)piperidin-2-yl)methoxy)cyclohexyl)phenoxy)acetic acid (100 mg, 0.216 mmol, 1.0 equiv.), MeCN (36 mL), DMF (9 mL), HATU (124 mg, 0.326 mmol, 1.5 equiv.) and DIPEA (56 mg, 0.436 mmol, 2.0 equiv.) under nitrogen atmosphere. The resulting solution was stirred for 3 hr at room temperature. The resulting mixture was concentrated. The crude product *tert-*butyl *((*2¹*S*,2⁴*S*,5²*R*,5³*S*)-6-oxo-3,8-dioxa-5(2,1)-piperidina-1(1,2)-benzena-2(1,4)-cyclohexanacyclooctaphane-5³-yl)carbamate was purified by column chromatography to provide the product. LCMS (ESI): *m*/*z* [M+H]⁺ = 445.

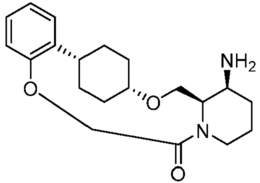

Into a 500 mL round-bottom flask purged and maintained with an inert atmosphere of nitrogen, was placed crude mixture *tert-*butyl ((2¹*S*,2⁴*S*,5²*R*,5³*S*)-6-oxo-3,8-dioxa-5(2,1)-piperidina-1(1,2)-benzena-2(1,4)-cyclohexanacyclooctaphane-5³-yl)carbamate (2 g, 4.50 mmol, 1.0 equiv.), dichloromethane (120 mL), TFA (40 mL). The resulting solution was stirred for 1 hr at 25 degrees C. The resulting mixture was concentrated under vacuum. The crude product was purified by Prep-HPLC to afford (2¹*S*,2⁴*S*,5²*R*,5³*S*)-5³-amino-3,8-dioxa-5(2,1)-piperidina-1(1,2)-benzena-2(1,4)-cyclohexanacyclooctaphan-6-one (800 mg, 51.6%) as a solid. LCMS (ESI): *m*/*z* [M+H]⁺ = 345.

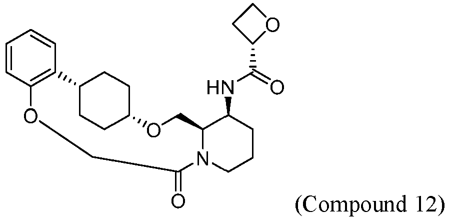

To a solution of (2¹*S*,2⁴*S*,5²*R*,5³*S*)-5³-amino-3,8-dioxa-5(2,1)-piperidina-1(1,2)-benze na-2(1,4)-cyclohexanacyclooctaphan-6-one (70 mg, 0.20 mmol, 1.0 equiv.) and (S)-oxetane-2 - carboxylic acid (62 mg, 0.61 mmol. 3.0 equiv.) in DCM (5 mL) was added 1-(3-dimethylaminopropyl)-3-ethylcarbodiimide hydrochloride (58 mg, 0.30 mmol, 1.5 equiv.) an dHOBt (47 mg, 0.30 mmol, 1.5 equiv.) and DIEA (79 mg, 0.61 mmol, 3.0 equiv.). The result ing mixture was stirred for 1 hr at 25 degrees C. The crude was purified by reverse flash chromatography to afford (S)-N-((2¹S,2⁴S,5²R,5³S)-6-oxo-3,8-dioxa-5(2,1)-piperidina-1(1,2)-benzena-2(1,4)-cyclohexanacyclooctaphane-5³-yl)oxetane-2-carboxamide (75 mg, 0.18 mmol, 86 %) as a solid. LCMS (ESI): *m*/*z* [M+H]⁺ = 429. ¹H NMR (400 MHz, Methanol-d₄) δ 7. 20 - 7.13 (m, 1H), 7.08 (dd, 1H), 6.91 - 6.83 (m, 2H), 5.34 (d, 1H), 5.29 - 5.23 (m, 1H), 5.08 (dd, 1H), 4.80 - 4.71 (m, 1H), 4.71 - 4.60 (m, 1H), 4.25 - 4.16 (m, 1H), 4.12 (d, 1H), 4.04 - 3.95 (m, 1H), 3.86 (d, 1H), 3.69 (s, 1H), 3.61 - 3.49 (m, 1H), 3.10 - 2.97 (m, 1H), 2.82 - 2.69 (m, 1H), 2.68 - 2.52 (m, 2H), 2.36 - 2.24 (m, 1H), 2.18 (d, 1H), 1.99 - 1.68 (m, 5H), 1.52 - 1.33 (m, 3H), 1.28 (d, 1H).

### Example 1.2

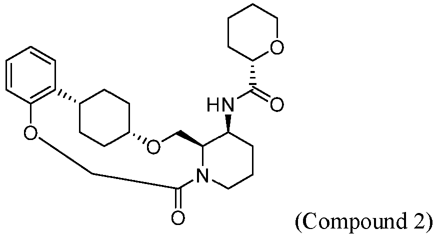

To a solution of (2¹*S*,2⁴*S,*5²*R*,5³*S*)-5³-amino-3,8-dioxa-5(2,1)-piperidina-1(1,2)-benze na-2(1,4)-cyclohexanacyclooctaphan-6-one (80 mg, 0.23 mmol, 1.0 equiv.) and (S)-tetrahydr o-2H-pyran-2-carboxylic acid (91 mg, 0.70 mmol. 3.0 equiv.) in DCM (5 mL) was added 1-( 3-dimethylaminopropyl)-3-ethylcarbodiimide hydrochloride (67 mg, 0.35 mmol, 1.5 equiv.) and HOBt (53 mg, 0.35 mmol, 1.5 equiv.) and DIEA (90 mg, 0.70 mmol, 3.0 equiv.). The resulting mixture was stirred for 1 hr at 25 degrees C The crude was purified by reverse flash chromatography to afford (S)-N-((2¹S,2⁴S,5²R,5³S)-6-oxo-3,8-dioxa-5(2,1)-piperidina-1 (1,2)-benzena-2(1,4)-cyclohexanacyclooctaphane-5³-yl) tetrahydro-2H-pyran-2-carboxamide (64.4 mg, 61 %) as a solid. LCMS (ESI): *m*/*z* [M+H]⁺ = 457. ¹H NMR (400 MHz, Methanol-d₄) δ 7.20 - 7.13 (m, 1H), 7.08 (dd, 1H), 6.92 - 6.83 (m, 2H), 5.32 (d, 1H), 5.28 - 5.20 (m, 1H ), 4.73 - 4.43 (m, 1H), 4.18 - 4.05 (m, 3H), 3.98 (dd, 1H), 3.87 - 3.76 (m, 2H), 3.70 (d, 1H), 3.58 - 3.47 (m, 2H), 2.83 - 2.68 (m, 1H), 2.64 - 2.51 (m, 1H), 2.36 - 2.11 (m, 2H), 2.07 - 1.9 9 (m, 1H), 1.98 - 1.76 (m, 5H), 1.71 - 1.53 (m, 4H), 1.52 - 1.37 (m, 4H), 1.33 - 1.23 (m, 1H).

### Example 1.3

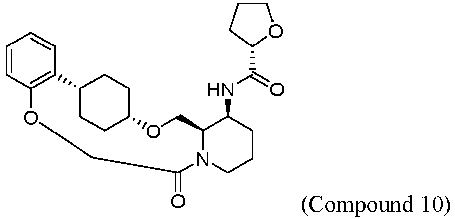

To a solution of (2¹*S*,2⁴*S*,5²*R*,5³*S*)-5³-amino-3,8-dioxa-5(2,1)-piperidina-1(1,2)-benzen a-2(1,4)-cyclohexanacyclooctaphan-6-one (200 mg, 0.58 mmol, 1.0 equiv.) and (S)-tetrahydro -2H-furan-2-carboxylic acid (101 mg, 0.87 mmol. 1.5 equiv.) in DCM (10 mL) was added HA TU (397 mg, 1.05 mmol, 1.5 equiv.) and N-diisopropylethylamine (113 mg, 0.87 mmol, 1.5 equiv.). The resulting mixture was stirred for 1 hr at 25 degrees C. The crude was purified by reverse flash chromatography to afford (S)-N-((2¹S, 2⁴S, 5²R, 5³S)-6-oxo-3,8-dioxa-5(2,1)-pi peridina-1(1,2)-benzena-2(1,4)-cyclohexanacyclooctaphane-5³-yl)tetrahydrofuran-2-carboxa mide (64.4 mg, 61 %) as a solid. LCMS (ESI): *m*/*z* [M+H]⁺ = 443. ¹H NMR (400 MHz, Chloroform-d) δ 7.18 (t, J = 7.4 Hz, 1H), 7.12 - 7.09 (m, 1H), 6.91 - 6.88 (m, 1H), 6.80 - 6.72 (m, 2H), 5.24 - 5.11 (m, 2H), 4.41 - 4.23 (m, 3H), 4.00 - 3.83 (m, 2H), 3.83 (t, J = 9.1 H z, 1H), 3.73 - 3.70 (m, 2H), 3.60 - 3.51 (m, 1H), 3.42 (dd, J = 8.6, 4.0 Hz, 1H), 2.76 - 2.54 (m, 2H), 2.41 - 2.20 (m, 2H), 2.12 - 2.09 (m, 2H), 1.99 - 1.82 (m, 5H), 1.76 - 1.64 (m, 2H), 1.53 - 1.45 (m, 1H), 1.46 - 1.32 (m, 3H), 1.28 (s, 1H).

### Example 1.4

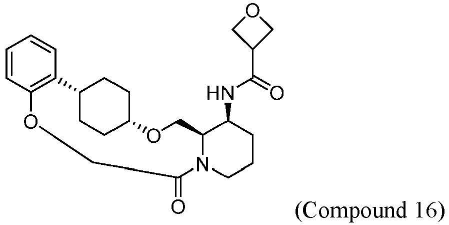

To a solution of (2¹*S*,2⁴*S*,5²*R*,5³*S*)-5³-amino-3,8-dioxa-5(2,1)-piperidina-1(1,2)-benzen a-2(1,4)-cyclohexanacyclooctaphan-6-one (80 mg, 0.23 mmol, 1.0 equiv.) and oxetane-3-carboxylic acid (71 mg, 0.70 mmol. 3.0 equiv.) in DCM (5 mL) was added 1-(3-dimethylamin opropyl)-3-ethylcarbodiimide hydrochloride (67 mg, 0.35 mmol, 1.5 equiv.) and HOBt (53 m g, 0.35 mmol, 1.5 equiv.) and DIEA (90 mg, 0.70 mmol, 3.0 equiv.). The resulting mixture was stirred for 1 hr at 25 degrees C. The crude was purified by reverse flash chromatography to afford (N-((2¹S, 2⁴S, 5²R, 5³S)-6-oxo-3,8-dioxa-5(2,1)-piperidina-1(1,2)-benzena-2(1,4)-cyclohexanacyclooctaphane-5³-yl)oxetane-3-carboxamide (58 mg, 58 %) as a solid. LCMS (ESI): *m*/*z* [M+H]⁺ = 429. ¹H NMR (400 MHz, Methanol-d₄) δ 7.16 - 7.13 (m, 1H), 7.08 (dd, 1H), 6.92 - 6.82 (m, 2H), 5.37 - 5.24 (m, 2H), 4.89 - 4.59 (m, 5H), 4.13 (dd, 1H), 4.05 - 3.91 (m, 1H), 3.86 (ddd, 2H), 3.69 (s, 1H), 3.60 - 3.48 (m, 1H), 2.82 - 2.66 (m, 1H), 2.59 -2.55 (m, 1H), 2.29 - 2.26 (m, 1H), 2.16 (d, 1H), 1.91 (d, 1H), 1.81 (s, 3H), 1.82 - 1.65 (m, 2H), 1.52 - 1.33 (m, 2H), 1.33 - 1.24 (m, 1H).

### Example 1.5

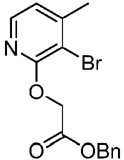

To a stirred mixture of benzyl 2-hydroxyacetate (21.9 g, 5.0 equiv., 131.6 mmol) in D MF(100 mL) was added NaH (60%, 5.26 g, 5.0 equiv., 131.6 mmol) in portions at 0 degrees C. The resulting mixture was stirred for 30 min at 0 degrees C under nitrogen atmosphere. To the above mixture was added 3-bromo-2-fluoro-4-methylpyridine (5.00 g, 1.0 equiv., 26.3 14 mmol) at room temperature and the resulting mixture was stirred for additional 2 hr at 80 degrees C. The reaction was quenched by addition of sat. NH₄Cl (aq.) (500 mL) at 0 degrees C. The resulting mixture was extracted with EtOAc (3 x 200 mL). The combined organic layers were washed with water (3x300 mL), dried over anhydrous Na₂SO₄. After filtration, the filtrate was concentrated under reduced pressure. The residue was purified by reverse flash chromatography to provide benzyl 2-((3-bromo-4-methylpyridin-2-yl)oxy)acetate (7.3 g, 83.0%) as an oil. LCMS (ESI): m/z [M+H]+ = 337.

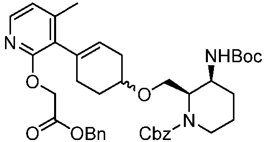

To a solution of benzyl (2R,3S)-3-((*tert*-butoxycarbonyl)amino)-2-(((4-(4,4,5,5-tetramethyl-1,3,2-dioxaborolan-2-yl)cyclohex-3-en-1-yl)oxy)methyl)piperidine-1-carboxylate (5.00 g, 1.0 equiv., 3.51 mmol) and benzyl 2-((3-bromo-4-methylpyridin-2-yl)oxy)acetate (1.41 g, 1.2 equiv., 4.21 mmol) in 1,4-dioxane (20 mL) and water (5.0 mL) were added Na₂C O₃ (1.12 g, 3.0 equiv., 10.5 mmol) and Pd(dppf)Cl₂ (385 mg, 0.15 equiv., 0.53 mmol). The reaction mixture was stirred for 2 hr at 80 degrees C under nitrogen atmosphere. The crude was purified by reverse flash chromatography to afford benzyl (2R,3S)-2-(((4-(2-( 2-(benzyloxy)-2-oxoethoxy)-4-methylpyridin-3-yl)cyclohex-3-en- 1-yl)oxy)methyl)-3-((*tert*-butoxycarbonyl)amino)piperidine-1-carboxylate (2.0 g, 80.0 %) as an oil.

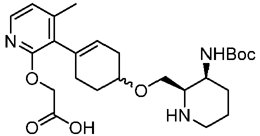

To a solution of benzyl (2R,3S)-2-(((4-(2-(2-(benzyloxy)-2-oxoethoxy)-4-methylpyrid in-3-yl)cyclohex-3-en-1-yl)oxy)methyl)-3-((*tert*-butoxycarbonyl)amino)piperidine-1-carboxylate (2.00 g, 1.0 equiv., 2.86 mmol) in *i*-PrOH (30 mL) was added Pd/C (152 mg, 0.5 equiv., 1.43 mmol) under nitrogen atmosphere. The resulting mixture was hydrogenated at room temperature for 15 hr under hydrogen atmosphere using a hydrogen balloon. The mixture was filtered through a CELITE^{®} (Imerys Minerals California Inc., San Jose, CA) pad and concentrated under reduced pressure to afford the crude product. The crude was purified by reverse flash chromatography to afford 2-((3-(4-(((2R,3S)-3-((*tert*-butoxycarbonyl)amino)piperidin-2-yl)methoxy)cyclohex-1-en-1-yl)-4-methylpyridin-2-yl)oxy)acetic acid (950 mg, 69.9 %) as a solid. LCMS (ESI): m/z [M+H]+ = 477.

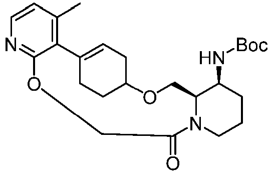

To a stirred mixture of 2-((3-(4-(((2R,3S)-3-((*tert*-butoxycarbonyl)amino)piperidin-2-yl)methoxy)cyclohex-1-en-1-yl)-4-methylpyridin-2-yl)oxy)acetic acid (900 mg, 1.0 equiv., 1. 89 mmol) and DIEA (734 mg, 3.0 equiv., 5.68 mmol) in MeCN (400 mL) was added HATU (1.08 g, 1.5 equiv., 2.84 mmol) at 25 degrees C under nitrogen atmosphere. The resulting mixture was stirred for 2 hr at 25 degrees C .The mixture was concentrated under reduced pressure. The crude was purified by reverse flash chromatography to afford *tert-*butyl ((5²R,5 ³S,E)-1⁴-methyl-6-oxo-3,8-dioxa-1(3,2)-pyridina-5(2,1)-piperidina-2(1,4)-cyclohexanacyclooctaphan-2¹-en-5³-yl)carbamate (650 mg, 75.1 %) as a solid. LCMS (ESI): m/z [M+H]+ = 469.

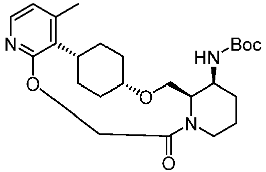

To a solution of *tert-*butyl ((5²R,5³S,E)-1⁴-methyl-6-oxo-3,8-dioxa-1(3,2)-pyridina-5(2,1)-piperidina-2(1,4)-cyclohexanacyclooctaphan-2¹-en-5³-yl)carbamate (1.2 g, 1.0 equiv., 2.6 mmol) in MeOH (200 mL) and acetic acid (20.0 mL) were added Pd/C (0.56 g, 10% Wt, 0.2 equiv., 0.52 mmol). The reaction mixture was stirred for 5 days under hydrogen atmosphere. The crude was purified by reverse flash chromatography to afford *tert-*butyl ((2¹S,2⁴S,5²R,5³S)-1⁴-methyl-6-oxo-3,8-dioxa-1(3,2)-pyridina-5(2,1)-piperidina-2(1,4)-cyclohexanacyclooctaphane-5³-yl)carbamate (500 mg, 41.0 %) as a solid. LCMS (ESI): m/z [M+H]+ = 461.

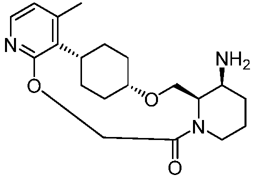

To a solution of *tert-*butyl ((2¹S,2⁴S,5²R,5³S)-1⁴-methyl-6-oxo-3,8-dioxa-1(3,2)-pyridi na-5(2,1)-piperidina-2(1,4)-cyclohexanacyclooctaphane-5³-yl)carbamate (400 mg, 1.0 equiv., 0.87 mmol) in DCM (15 mL) was added TFA (3 mL). The reaction mixture was stirred for 25 degrees C at 1 hr under nitrogen atmosphere. After the filtrate was concentrated under reduce d pressure, aqueous Na₂CO₃ solution was added. The resulting mixture was extracted with dichloromethane (3x30 mL). The combined organic layers were washed with brine, dried over anhydrous Na₂SO₄, after filtration, the filtrate was concentrated under reduced pressure. The crude was purified by reverse flash chromatography to afford (2¹S,2⁴S,5²R,5³S)-5³-amino-1⁴-methyl-3,8-dioxa-1(3,2)-pyridina-5(2,1)-piperidina-2(1,4)-cyclohexanacyclooctaphan-6-one (230 mg, 73.5 %) as a solid. LCMS (ESI): m/z [M+H]+ =360.

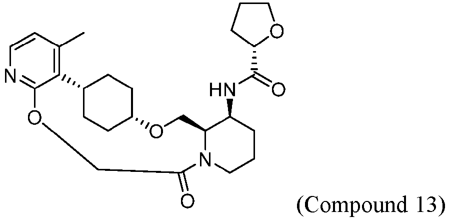

To a solution of (2¹S, 2⁴S, 5²R, 5³S)-5³-amino-1⁴-methyl-3,8-dioxa-1(3,2)-pyridina-5(2,1)-piperidina-2(1,4)-cyclohexanacyclooctaphan-6-one (50 mg, 1.0 equiv., 0.14 mmol) and (S)-tetrahydrofuran-2-carboxylic acid (19 mg, 1.2 equiv., 0.17 mmol) in DCM (1.5 mL) was added DIEA (36 mg, 2.0 equiv., 0.28 mmol), HOBt (32 mg, 1.5 equiv., 0.21 m mol) and EDC (40 mg, 1.5 equiv., 0.21 mmol). The resulting mixture was stirred for 1.5 hr at 25 degrees C. The resulting mixture was extracted with DCM (3 x 20 mL). The combined organic layers were washed with H₂O and brine, dried over anhydrous Na₂SO₄. After filtration, the filtrate was concentrated under reduced pressure. The residue was purified by Prep-TLC to afford (S)-N-((2¹S, 2⁴S, 5²R, 5³S)-1⁴-methyl-6-oxo-3,8-dioxa-1(3,2)-pyridina-5(2,1)-piperidina-2(1,4)-cyclohexanacyclooctaphane-5³-yl)tetrahydrofuran-2-carboxamide (6 9 mg, 63 %) as a solid.
LCMS (ESI): m/z [M+H]+ = 458; ¹H NMR (400 MHz, Methanol-d₄) δ 7.81 (dd, J = 5.2, 4.1 Hz, 1H), 6.81 - 6.79 (m, 1H), 5.39 - 5.23 (m, 2H), 4.79 - 4.75 (m, 1H), 4.40 - 4.23 (m, 2H), 4.19 -4.08 (m, 1H), 4.06 - 3.97 (m, 2H), 3.94 - 3.75 (m, 2H), 3.71 (s, 1H), 3.60 - 3.45 (m, 1H), 3.30 - 3.27 (m, 1H), 3.18 - 2.92 (m, 1H), 2.66 - 2.62 (m, 1H), 2.32 (s, 2H), 2.24 - 2.20 (m, 1H), 2.14 - 1.97 (m, 2H), 1.97 - 1.81 (m, 5H), 1.74 - 1.58 (m, 1H), 1.58 - 1.25 (m, 4H), 1.24 - 1.14 (m, 1H).

### Example 1.6

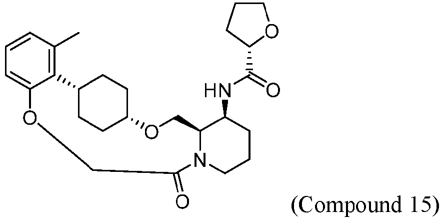

To a solution of (2¹S, 2⁴S, 5²R, 5³S)-5³-amino-1⁶-methyl-3,8-dioxa-5(2,1)-piperidina-1(1,2)-benzena-2(1,4)-cyclohexanacyclooctaphan-6-one (55 mg, 1.0 equiv., 0.15 mmol) and DIEA (30 mg, 1.5 equiv., 0.23 mmol) in DMF (2 mL) were added (S)-tetrahydrofuran-2-carboxylic acid (21 mg, 1.2 equiv., 0.18 mmol) and HATU (88 mg, 1.5 equiv., 0.23 mmol). After stirring for 2 hr at 25 degrees C under a nitrogen atmosphere, the resulting mixture was purified by reverse flash chromatography to afford (S)-N-((2¹S, 2⁴S, 5²R, 5³S)-1⁶-methyl-6-oxo-3,8-dioxa-5(2,1)-piperidina-1(1,2)-benzena-2(1,4)-cyclohexanacyclooctaphane-5³-yl)tetrahydrofuran-2-carboxamide (50 mg, 0.11 mmol, 71 %) as a solid. LCMS (ESI): m/z [M+H]+ = 457; ¹H NMR (400 MHz, Chloroform-d) δ 7.06 (t, J = 7.8 Hz, 1H), 6.82 (d, J = 7.6 Hz, 1H), 6.72 (d, J = 8.8 Hz, 1H), 6.64 (d, J = 8.0 Hz, 1H), 5.28-5.16 (m, 1H), 5.1 - 5.071 (m, 1H), 4.41-4.22 (m, 3H), 4.01-3.90 (m, 2H), 3.89-3.80 (m, 1H), 3.72 - 3.69 (m, 2H), 3.59-3.47 (m, 1H), 3.39 - 3.35 (m, 1H), 3.00-2.89 (m, 1H), 2.77-2.60 (m, 1H), 2.37-2.20 (m, 5H), 2.18-2.05 (m, 2H), 2.02-1.82 (m, 6H), 1.73-1.66 (m, 1H), 1.49-1.36 (m, 3H), 1.29 - 1.26 (m, 1H).

### Example 2: Human OX₂R IP1 assay

T-Rex CHO cells stably overexpressing the human orexin-2 receptor (OX₂R) were induced overnight with 1 µg/mL of doxycycline in a T225 flask. 24 hours post induction, cells were lifted with accutase and plated into a 384-well proxy plate at 30,000 cells/well. Cells were then treated with different test compounds in 1X stimulation buffer containing 10 mM Hepes, 1 mM CaCl₂, 0.5 mM MgCl₂, 4.2 mM KCl, 146 mM NaCl, 5.5 mM glucose, and 50 mM LiCl, pH 7.4, for 1 hr at 37 degrees C. Following incubation, the reaction was terminated by the addition of detection mix, which is composed of IP1-d2 and anti-IP1-cryptate diluted in lysis buffer as well as 1X stimulation buffer. The plates were allowed to incubate for 1 hour at room temperature and were then read in the ENVISION^{®} multimode plate reader, measuring inositol phosphate levels.

Cisbio IP1 is a cell-based functional assay quantifying the accumulation of inositol monophosphate (IP), a metabolite released as a result of orexin 2 receptor activation through the phospholipase C-Gq signaling pathway. This is a competitive immunoassay in which the IP1 produced by the cells upon receptor activation competes with the IP1 analog coupled to the d2 fluorophore (acceptor) for binding to an anti-IP1 monoclonal antibody labeled with Eucryptate (donor). The measured HTRF-FRET based signal is inversely proportional to the IP1 concentration produced.

The EC₅₀ values reported in Table 2 were obtained according to the human OX₂R IP1 assay described above. Data are the mean EC₅₀ values ± S.E.M.

**Table 2**

| **Compound** | **Compound No.** | **EC₅₀ (nM)** |
|---|---|---|
| 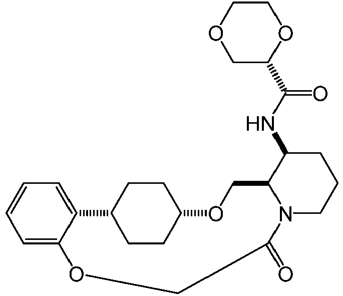 | 1 | *** |
| 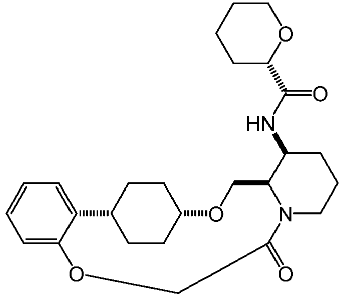 | 2 | *** |
| 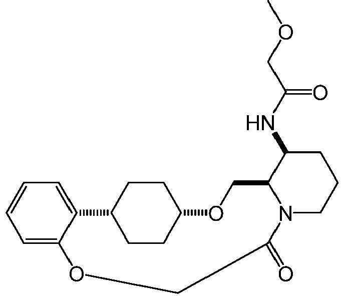 | 3 | *** |
| 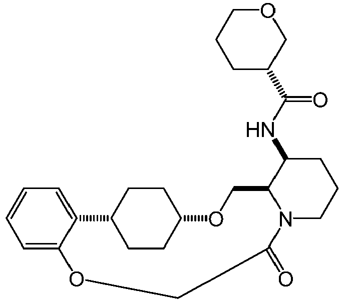 | 4 | ** |
| 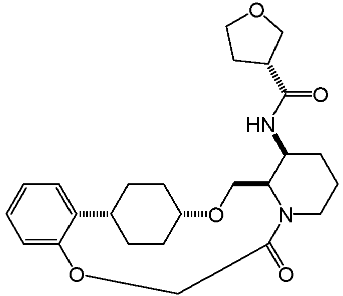 | 5 | ** |
| 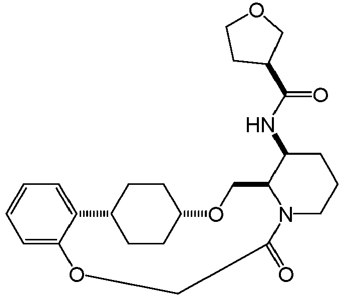 | 6 | ** |
| 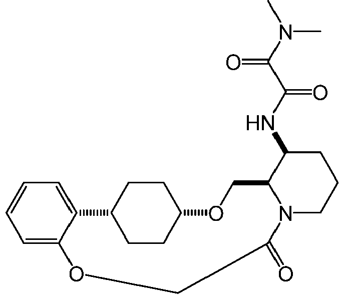 | 7 | *** |
| 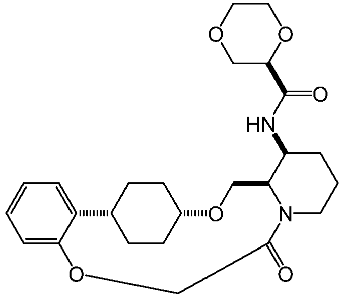 | 8 | ** |
| 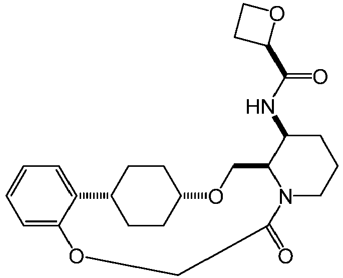 | 9 | ** |
| 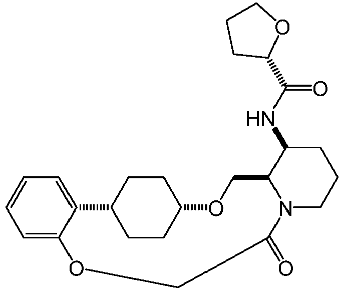 | 10 | *** |
| 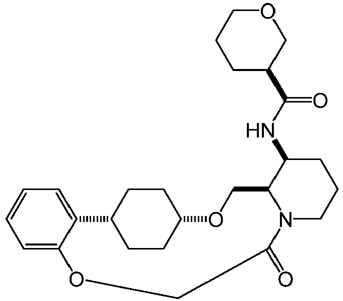 | 11 | ** |
| 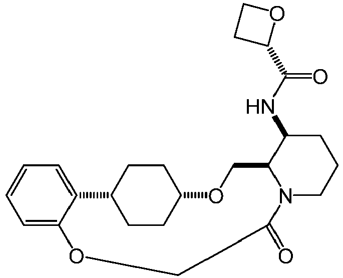 | 12 | *** |
| 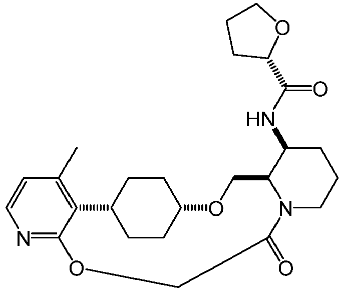 | 13 | *** |
| 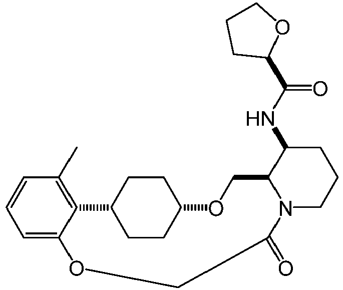 | 14 | *** |
| 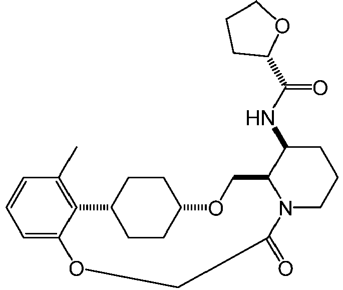 | 15 | *** |
| 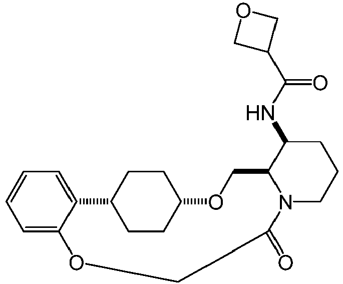 | 16 | *** |
| 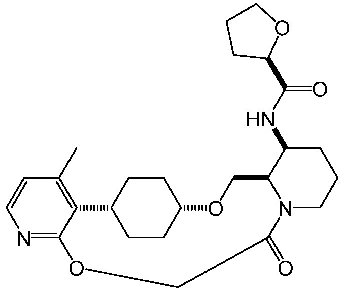 | 17 | *** |
| 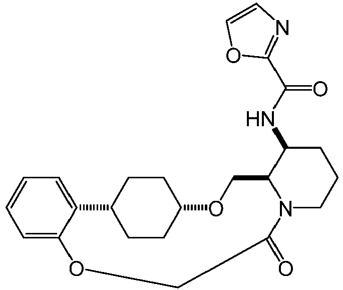 | 18 | * |
| 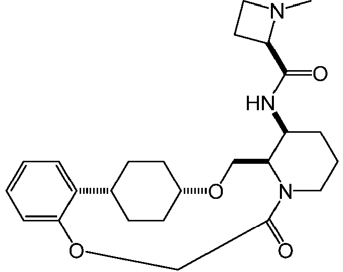 | 19 | * |
| 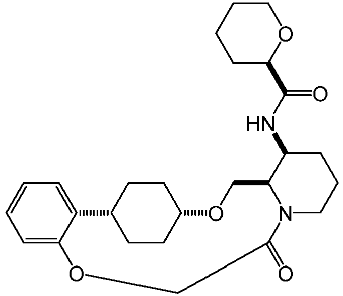 | 20 | * |
| 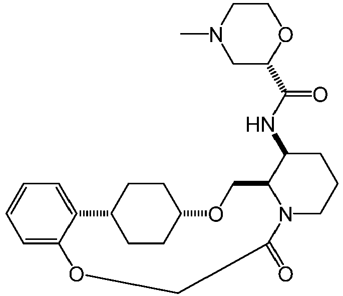 | 21 | * |
| 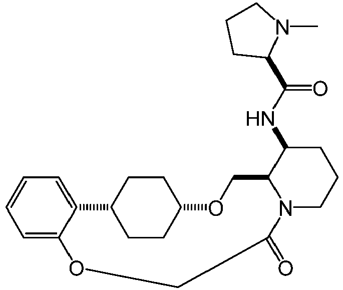 | 22 | * |
| 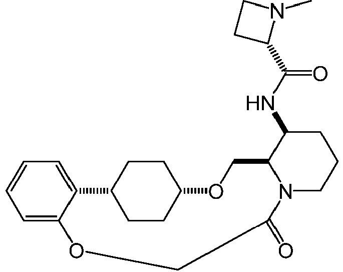 | 23 | * |
| 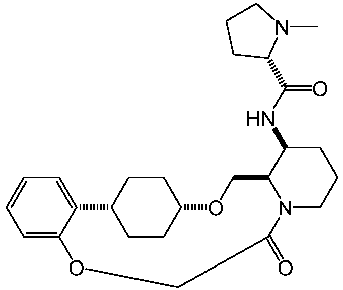 | 24 | * |
| 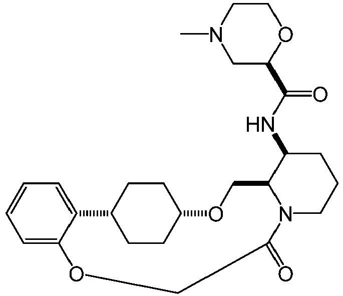 | 25 | * |
| 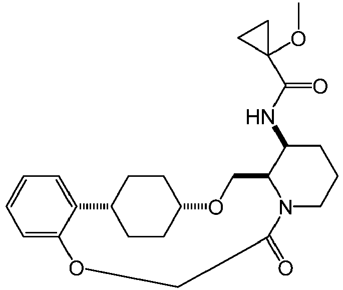 | 89 | * |

| | | |
|---|---|---|
| ***EC₅₀ < 100 nM **EC₅₀ 100-1,000 nM *EC₅₀ > 1,000 nM | | |

### Example 3: Assessment of Wake Promotion in Sprague-Dawley Rats

Wake promotion is assessed using electroencephalography (EEG) and electromyography (EMG) in adult male Sprague-Dawley rats. All rats (Charles River Laboratories, Raleigh, NC, USA) are intraperitoneally implanted with telemetry devices (F50-EEE, Data Sciences International Inc., MN, USA) under isoflurane anesthesia. For EEG, stainless steel screws are implanted over frontal cortex and parietal cortex, and reference screws are placed over cerebellum. Additionally, an electrode is placed in neck muscle for EMG. Rats are given carprofen post-surgery and undergo a 7 to 10-day recovery period. Rats are habituated to the experimental room for 7 days and are maintained on a 12-hour light-dark cycle.

EEG and EMG data are recorded using the DSI telemetry system and Ponemah software (Data Sciences International Inc., MN, USA). Sleep-wake stages are scored both manually and with Somnivore, a supervised machine learning software platform, in 10 second epochs. Records are visually inspected as needed post-processing.

All test compounds are dissolved in 5% DMSO and suspended in 95% saline with 0.5% methylcellulose and 0.5% tween. In a cross-over design, rats are dosed during the inactive light phase at zeitgeber time 5 (ZT5) at a dose volume of 3.33 ml/kg body weight. Unless otherwise indicated, all compounds are dosed orally. Recordings for each rat are initiated immediately after dosing and last for 6 hours post-dose.

Two key endpoints include wakefulness time and cortical activation time. Wakefulness time is derived from the sleep-wake stage analysis. Cortical activation time is based on the duration in which frontal gamma oscillatory activity (30-100Hz), a key feature of wakefulness, was elevated relative to a pre-treatment baseline. Mean cortical activation time is computed relative to vehicle treatment for the 6-hour post-dose period.

While this invention has been particularly shown and described with references to preferred embodiments thereof, it will be understood by those skilled in the art that various changes in form and details may be made therein without departing from the scope of the invention defined by the appended claims.

## Claims

1. A compound of Formula I or a pharmaceutically acceptable salt thereof: 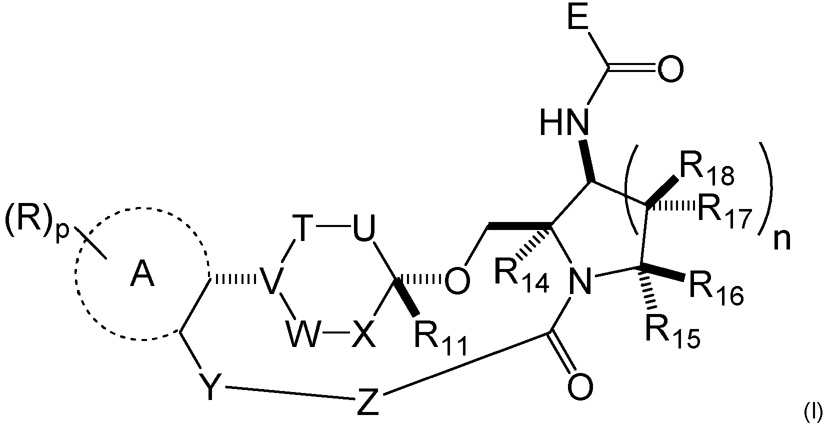 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4-to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 1, 2, 3, or 4;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

2. The compound of claim 1, wherein m is 1 or 2, preferably wherein m is 1.

3. A compound of Formula II or a pharmaceutically acceptable salt thereof: 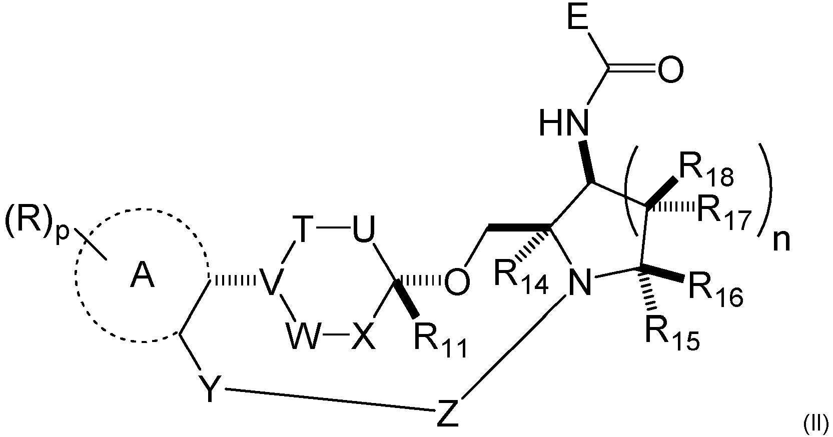 wherein:
ring A is selected from the group consisting of phenyl, pyridinyl, pyridazinyl, pyrimidinyl, pyrazinyl, and triazinyl;
n is 1, 2, or 3;
E is selected from the group consisting of NRₐR_{b}, C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4-to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl;
T is CR₁R₂ or O;
W is CR₄R₅ or O;
U is CR₆R₇;
X is CR₈R₉;
V is CR₃ or N;
Y is NR₁₀, O or absent;
Z is (CR₁₂R₁₃)ₘ;
each R is, independently, selected from the group consisting of halogen, deuterium, hydroxyl, cyano, unsubstituted C₁-C₃ alkyl, and C₁-C₃ alkyl substituted with one or more halogen or deuterium;
p is 0, 1, 2, 3, or 4;
Rₐ and R_{b} are each, independently, H or unsubstituted C₁-C₃ alkyl;
m is 2, 3, 4, or 5 when Y is absent; or
m is 1, 2, 3, or 4 when Y is NR₁₀ or O;
and further wherein:
R₁, R₂, R₄, and R₅ are each, independently, selected from the group consisting of H, hydroxyl, halogen, and deuterium;
or, alternatively, R₂ and R₅ together with the carbon atoms to which they are attached, form a single bond;
R₃ is selected from the group consisting of H, deuterium, halogen, hydroxyl, and cyano;
or, alternatively, R₃ and R₁, together with the carbon atoms to which they are attached, form a C₃-C₅ cycloalkyl;
or, alternatively, R₃ and R₄, together with the carbon atoms to which they are attached, form a C₃-C₅ cycloalkyl;
R₆, R₇, R₈, R₉, and R₁₁ are each, independently, selected from the group consisting of H, hydroxyl, halogen, and deuterium;
R₁₀ is selected from the group consisting of H, unsubstituted C₁-C₃ alkyl, and C₁-C₃ alkyl substituted with one or more halogen;
each R₁₂ and R₁₃ is, independently, selected from the group consisting of H, halogen, deuterium, unsubstituted C₁-C₃ alkyl, and C₁-C₃ alkyl substituted with hydroxyl or one or more halogen; and
R₁₄, R₁₅, and R₁₆ are each, independently, selected from the group consisting of H, unsubstituted C₁-C₃ alkyl or C₁-C₃ alkyl substituted with one or more halogen; and
each R₁₇ and R₁₈ is, independently, selected from the group consisting of H, unsubstituted C₁-C₃ alkyl or C₁-C₃ alkyl substituted with one or more halogen.

4. The compound of claim 3, wherein m is 2 or 3, preferably wherein m is 2.

5. The compound of any one of claims 1-4, wherein n is 1, preferably wherein n is 2.

6. The compound of any one of claims 1-5, wherein ring A is phenyl, or pyridinyl.

7. The compound of any one of claims 1-6, wherein Y is O, or absent.

8. The compound of any one of claims 1-7, wherein:
(i) T is CR₁R₂, or O;
(ii) W is CR₄R₅, or O; and/or
(iii) V is CR₃.

9. The compound of any one of claims 1-8, wherein E is selected from the group consisting of:
(i) C(=O)NRₐR_{b}, C₁-C₃ alkylene-NRₐR_{b}, C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃ alkylene-NRₐR_{b}, C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl,
optionally wherein E is selected from the group consisting of C₁-C₃ alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the C₁-C₃alkyl, C₂-C₄ alkenyl, C₂-C₄ alkynyl, C₃-C₈ cycloalkyl, C₁-C₃ alkylene-(C₃-C₈ cycloalkyl), 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl,
preferably wherein E is selected from the group consisting of C₁-C₃ alkyl, C₂-C₄ alkenyl, C₂-C₄ alkynyl, C₃-C₈ cycloalkyl, and C₁-C₃ alkylene-(C₃-C₈ cycloalkyl), wherein the C₁-C₃alkyl, C₂-C₄ alkenyl, C₂-C₄ alkynyl, C₃-C₈ cycloalkyl, or C₁-C₃ alkylene-(C₃-C₈ cycloalkyl) is unsubstituted or substituted with one or more halogen, hydroxyl, C₁-C₃ alkyl, or C₁-C₃ alkoxyl; or
(ii) 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, and C₁-C₃ alkylene-(5- to 10-membered heteroaryl), wherein the 4- to 10-membered heterocyclyl, C₁-C₃ alkylene-(4- to 10-membered heterocyclyl), C₆-C₁₀ aryl, C₁-C₃ alkylene-(C₆-C₁₀ aryl), 5- to 10-membered heteroaryl, or C₁-C₃ alkylene-(5- to 10-membered heteroaryl) is unsubstituted or substituted with one or more halogen, hydroxyl, C₁-C₃ alkyl, or C₁-C₃ alkoxyl,
preferably wherein E is selected from the group consisting of 4- to 7-membered heterocyclyl and C₁-C₃ alkylene-(4- to 7-membered heterocyclyl), wherein the 4-to 7-membered heterocyclyl or C₁-C₃ alkylene-(4- to 7-membered heterocyclyl) is unsubstituted or substituted with one or more halogen, hydroxyl, C₁-C₃ alkyl, or C₁-C₃ alkoxyl.

10. The compound of any one of claims 1-7, wherein T is CR₁R₂, W is CR₄R₅, and V is CR₃.

11. A compound or a pharmaceutically acceptable salt thereof according to claims 1 and 3 selected from the group consisting of: 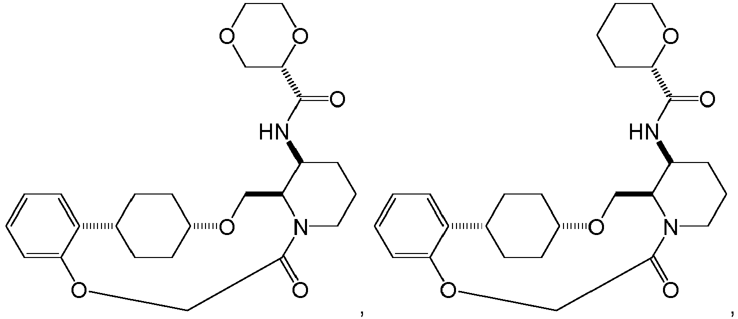 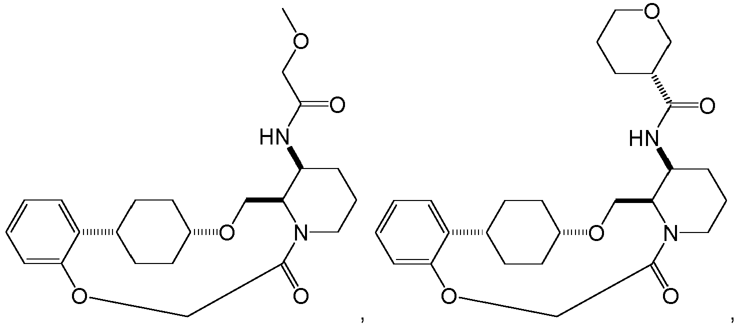 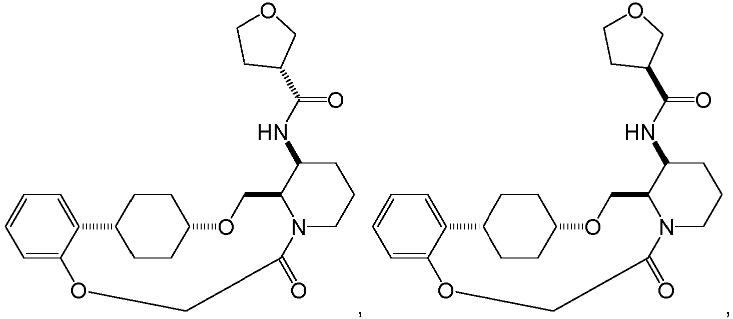 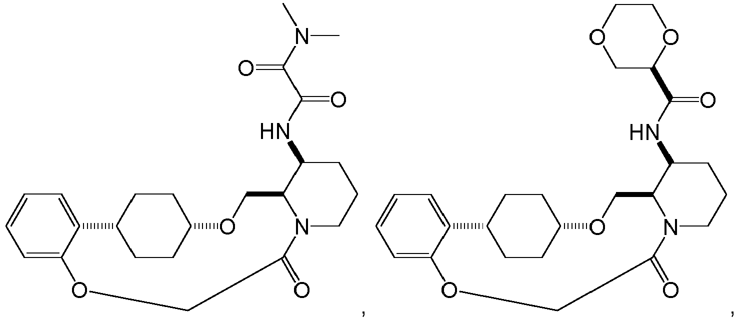 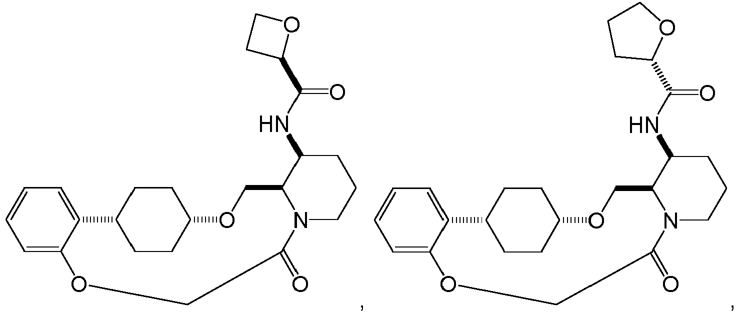 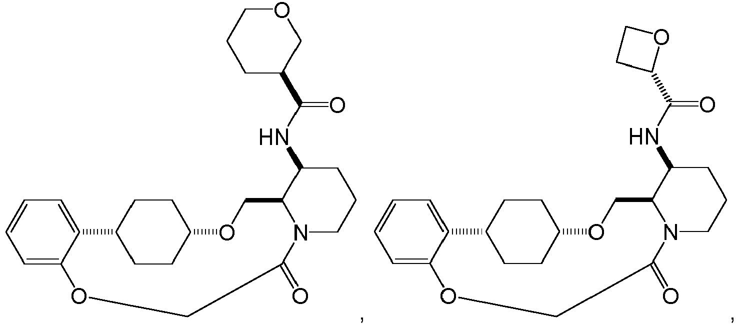 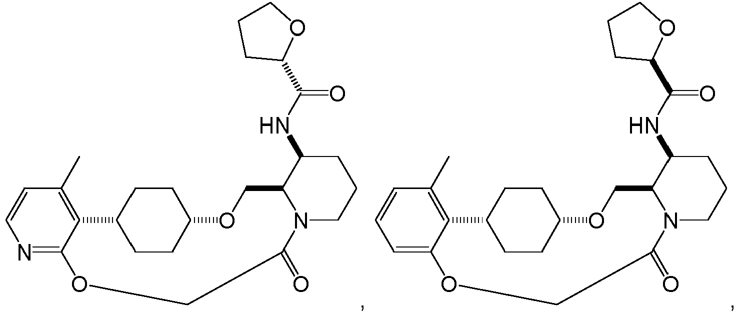 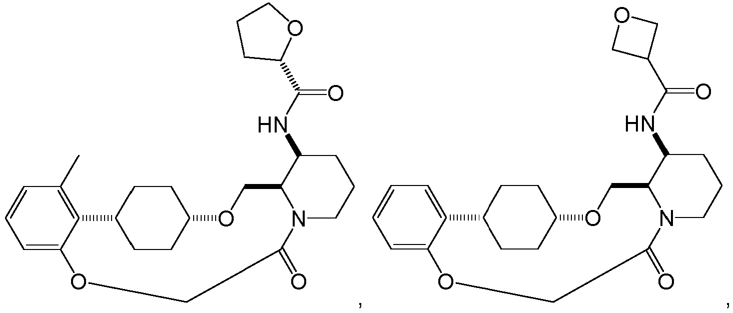 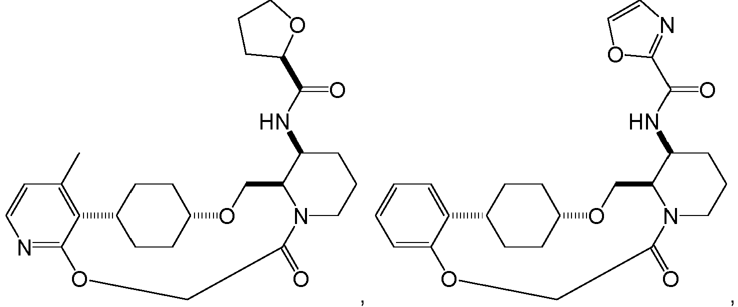 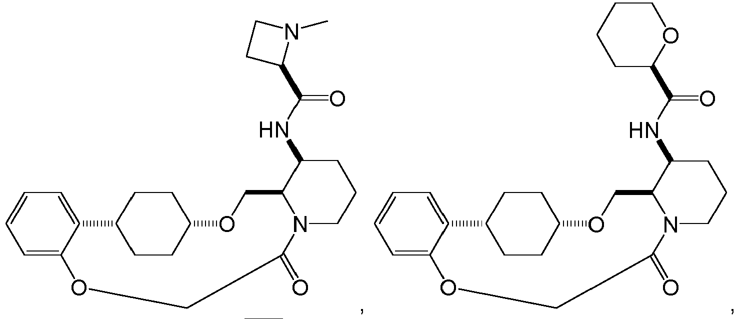 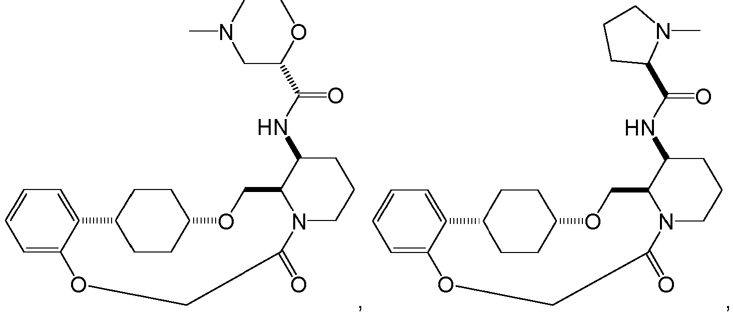 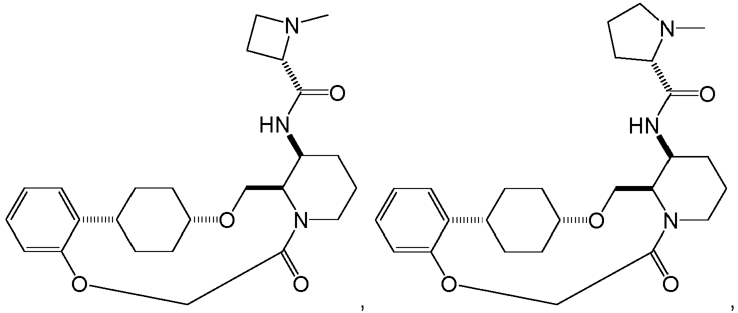 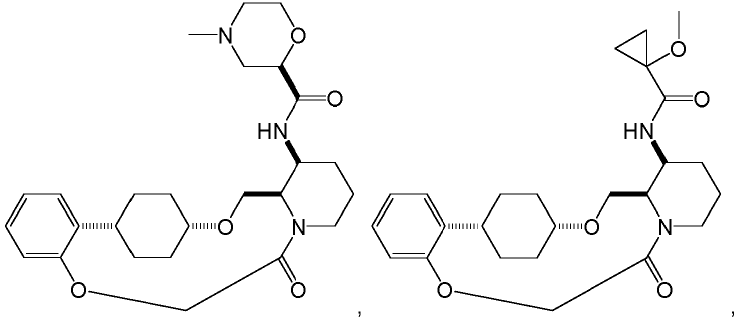 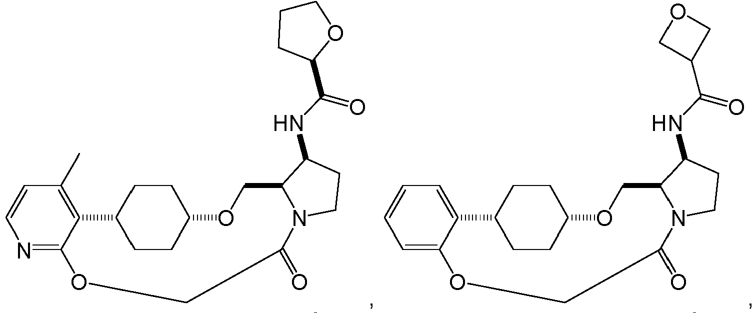 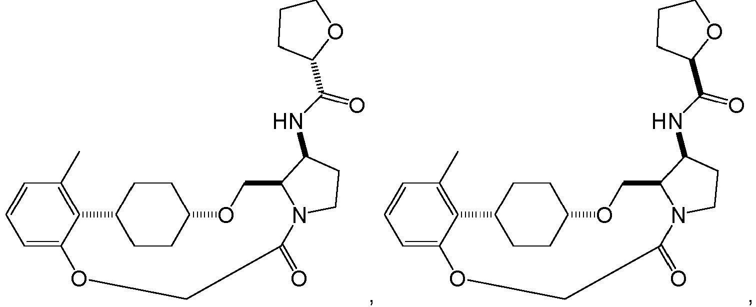 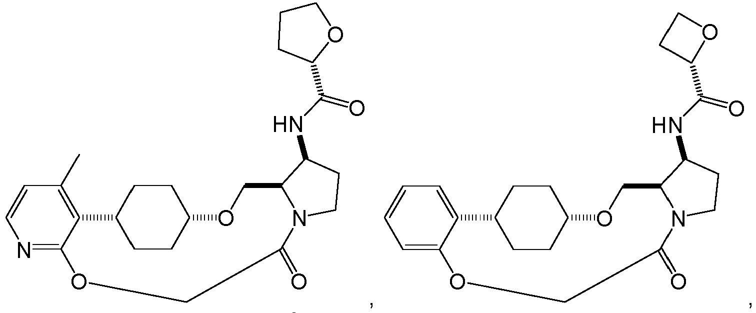 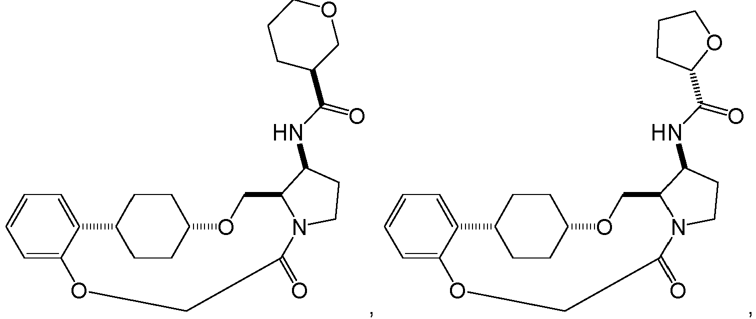 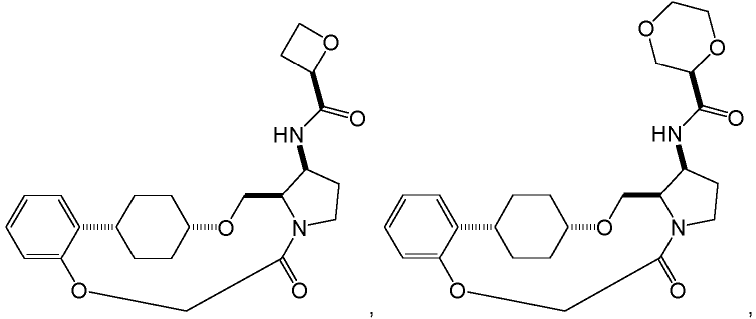 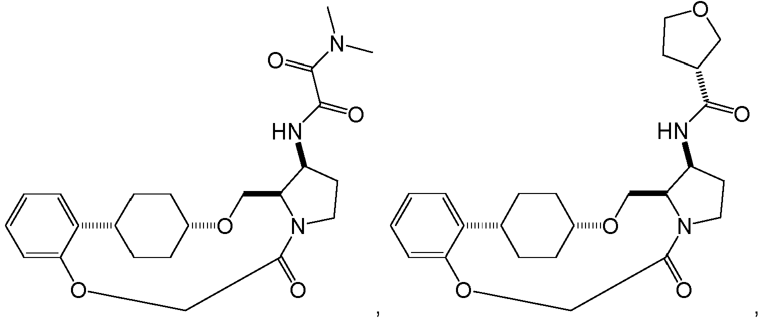 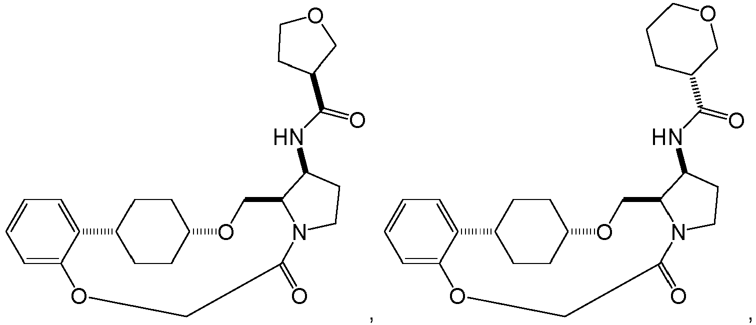 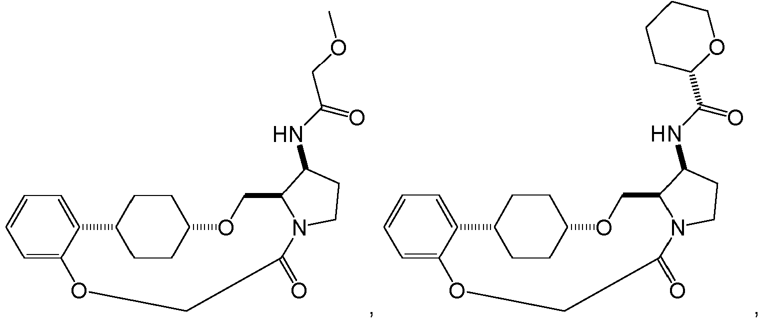 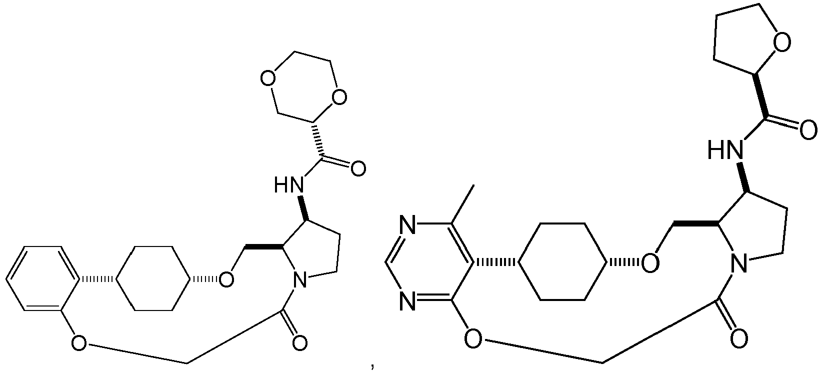 and

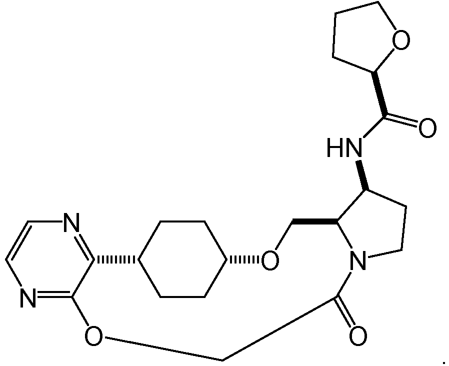

12. A compound or a pharmaceutically acceptable salt thereof according to claims 1 and 3 selected from the group consisting of:

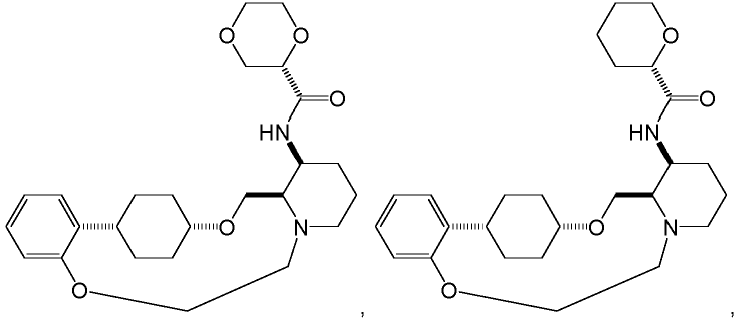

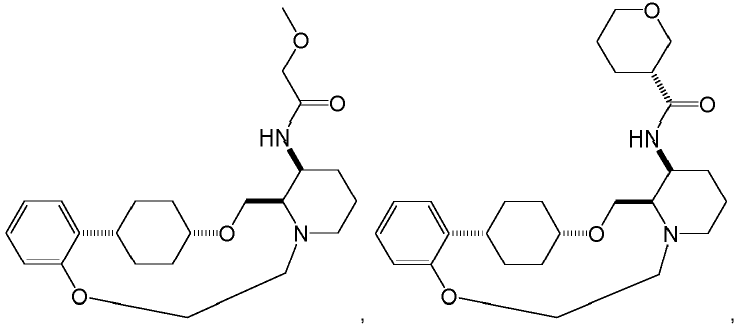

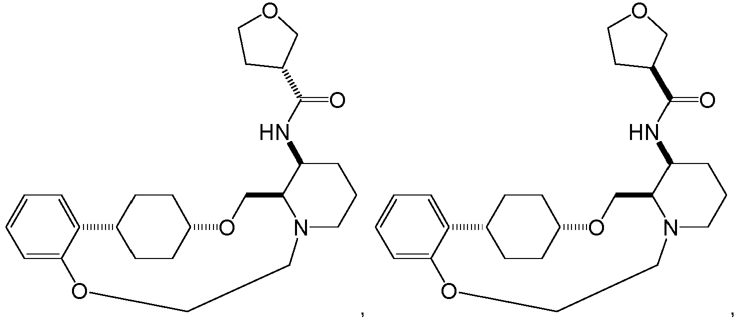

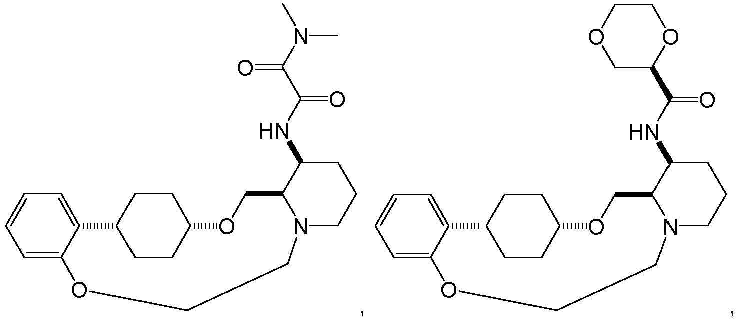

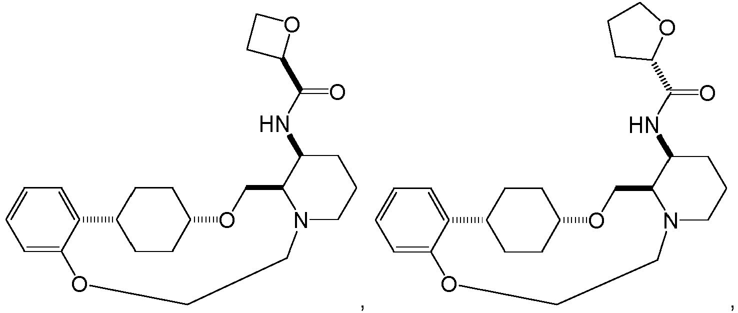

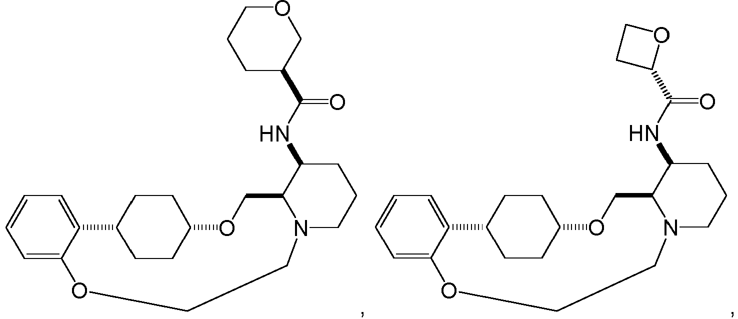

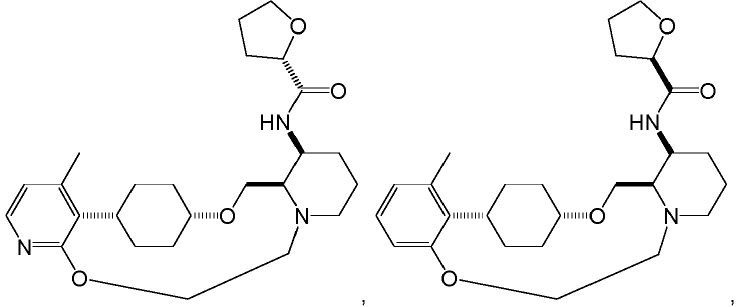

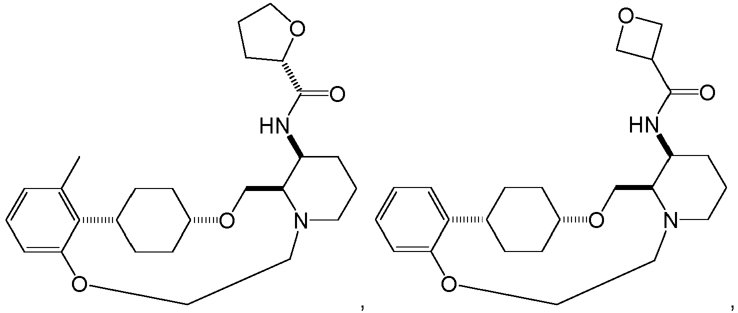

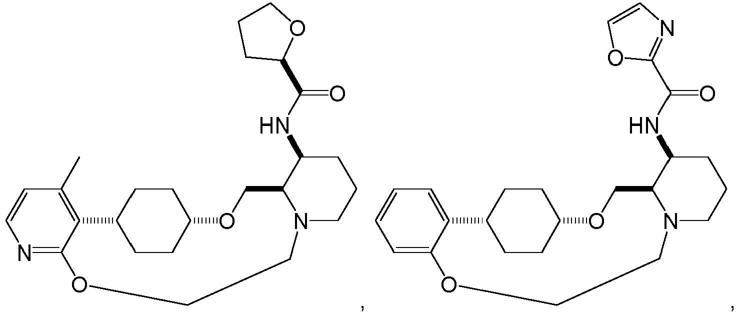

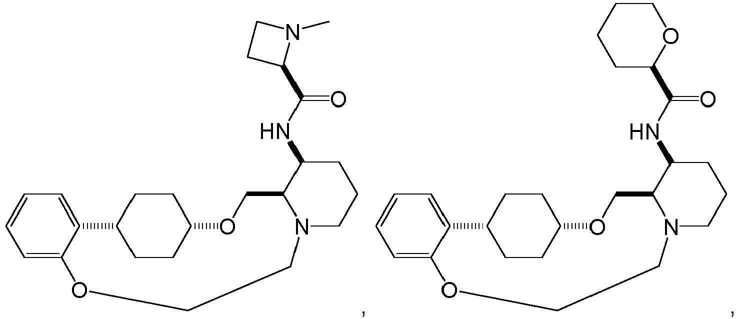

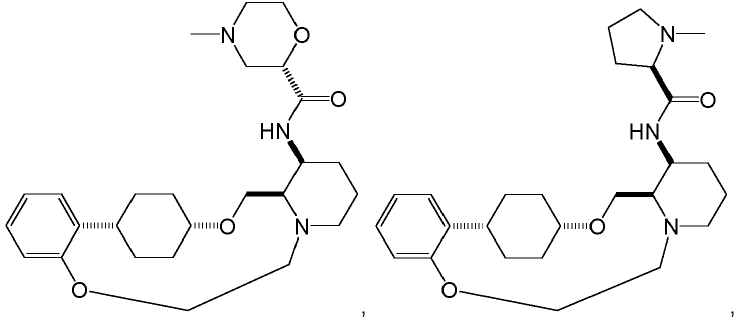

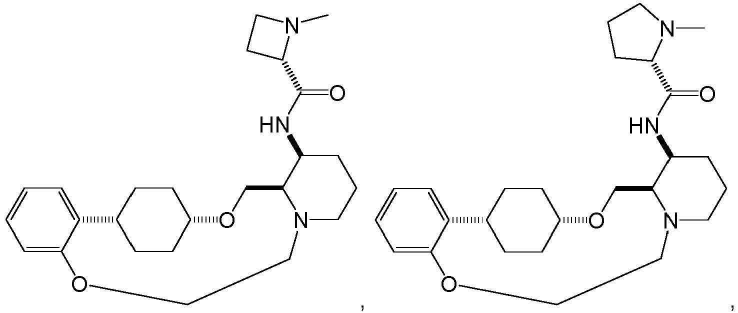

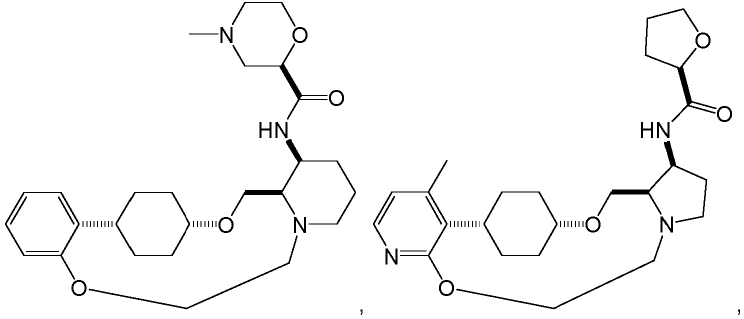

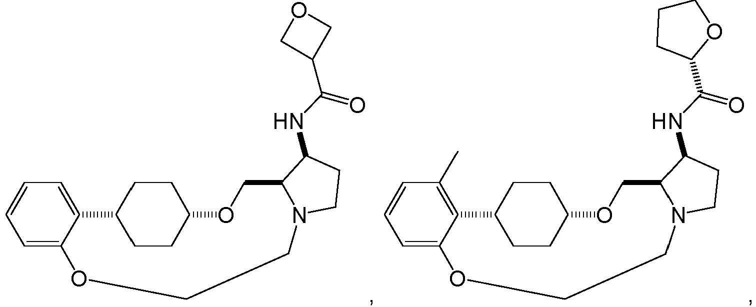

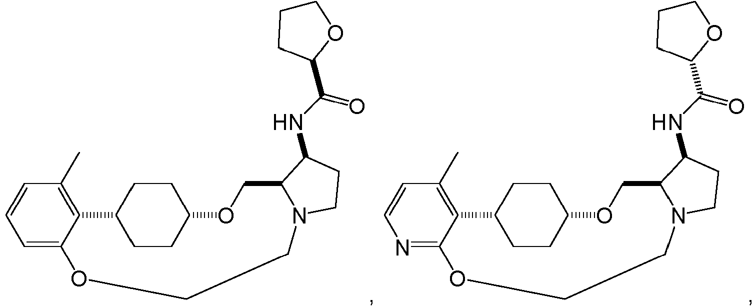

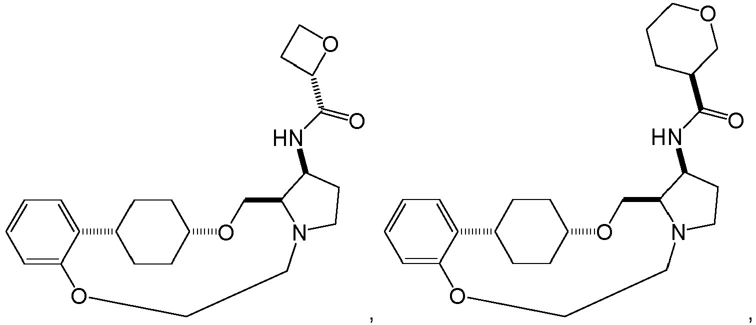

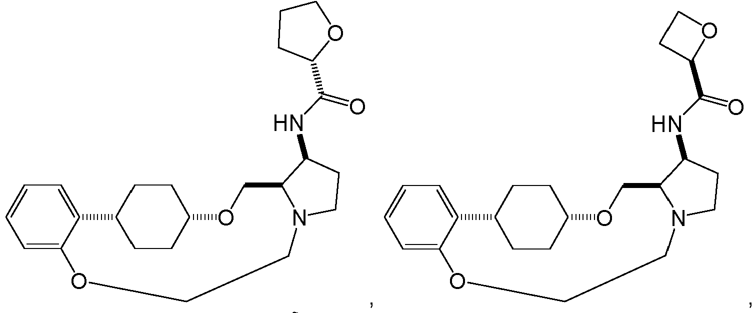

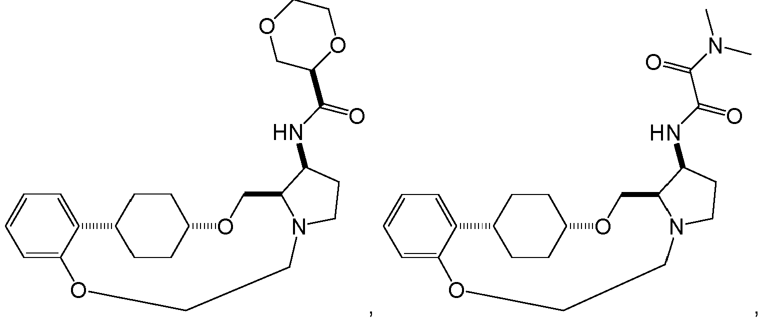

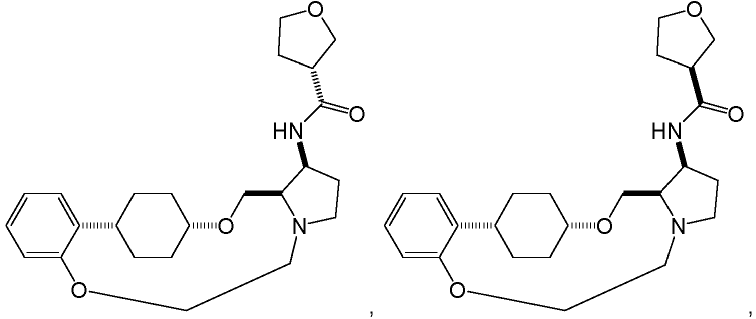

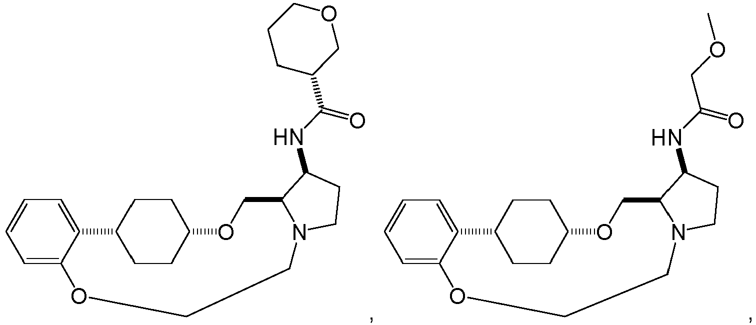

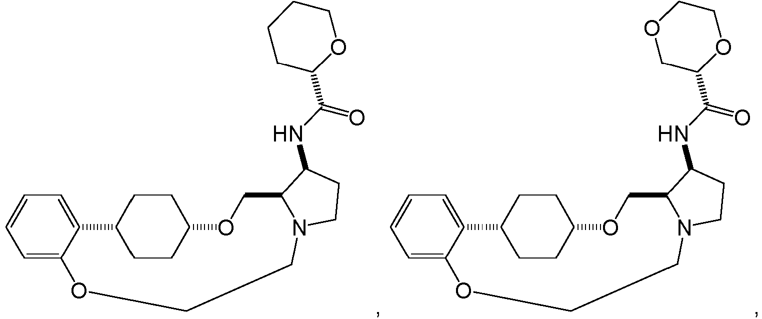

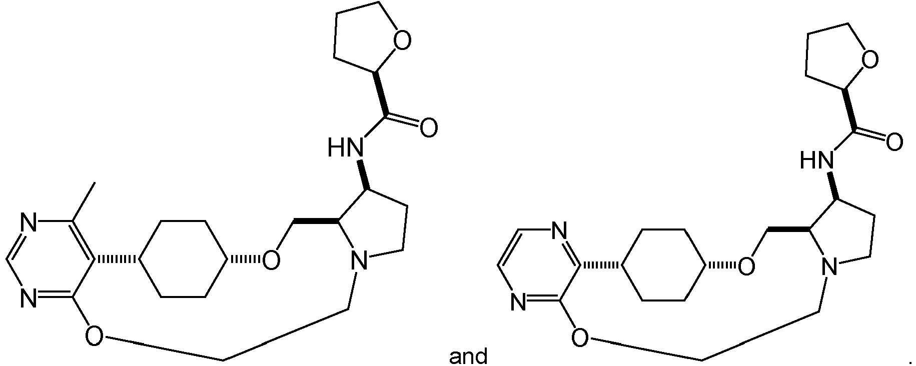

13. A pharmaceutical composition comprising a compound of any one of claims 1-12 or a pharmaceutically acceptable salt thereof, and a pharmaceutically acceptable carrier.

14. A compound of any one of claims 1-12 or a pharmaceutically acceptable salt thereof, or a composition according to claim 13 for use in treating narcolepsy.

15. A compound of any one of claims 1-12 or a pharmaceutically acceptable salt thereof, or a composition according to claim 13 for use in treating cataplexy.

## Patentansprüche

1. Verbindung von Formel I oder ein pharmazeutisch verträgliches Salz davon: 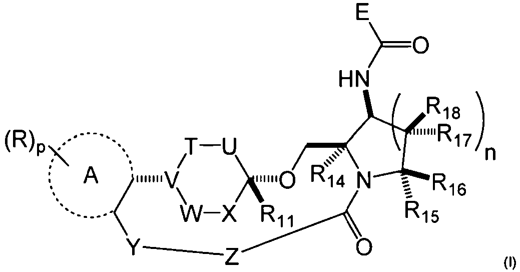 wobei:
Ring A ausgewählt ist aus der Gruppe bestehend aus Phenyl, Pyridinyl, Pyridazinyl, Pyrimidinyl, Pyrazinyl und Triazinyl;
n 1, 2 oder 3 ist;
E ausgewählt ist aus der Gruppe bestehend aus NRₐR_{b}, C(=O)NRₐR_{b}, C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrigem Heterocyclyl, C₁-C₃-Alkylen-(4- bis 10-gliedriges Heterocyclyl), C₆-C₁₀-Aryl, C₁-C₃-Alkylen-(C₆-C₁₀-Aryl), 5- bis 10-gliedrigem Heteroaryl und C₁-C₃-Alkylen-(5- bis 10-gliedriges Heteroaryl), wobei das C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrige Heterocyclyl, C₁-C₃-Alkylen-(4- bis 10-gliedriges Heterocyclyl), C₆-C₁₀-Aryl, C₁-C₃-Alkylen-(C₆-C₁₀-aryl), 5- bis 10-gliedrige Heteroaryl oder C₁-C₃-Alkylen-(5- bis 10-gliedriges Heteroaryl) unsubstituiert oder mit einem oder mehreren Halogenen, Hydroxyl, C₁-C₃-Alkyl oder C₁-C₃-Alkoxyl substituiert ist;
T CR₁R₂ oder O ist;
W CR₄R₅ oder O ist;
U CR₆R₇ ist;
X CR₈R₉ ist;
V CR₃ oder N ist;
Y NR₁₀, O ist oder fehlt;
Z (CR₁₂R₁₃)ₘ ist;
jedes R unabhängig ausgewählt ist aus der Gruppe bestehend aus Halogen, Deuterium, Hydroxyl, Cyano, unsubstituiertem C₁-C₃-Alkyl und C₁-C₃-Alkyl, das mit einem oder mehreren Halogenen oder Deuterium substituiert ist;
p 0, 1, 2, 3 oder 4 ist;
Rₐ und R_{b} jeweils unabhängig H oder unsubstituiertes C₁-C₃-Alkyl sind;
m 1, 2, 3 oder 4 ist;
und wobei weiter:
R₁, R₂, R₄ und R₅ jeweils unabhängig voneinander ausgewählt sind aus der Gruppe bestehend aus H, Hydroxyl, Halogen und Deuterium;
oder alternativ R₂ und R₅ zusammen mit den Kohlenstoffatomen, an die sie gebunden sind, eine Einfachbindung bilden;
R₃ ausgewählt ist aus der Gruppe bestehend aus H, Deuterium, Halogen, Hydroxyl und Cyano;
oder alternativ R₃ und R₁ zusammen mit den Kohlenstoffatomen, an die sie gebunden sind, ein C₃-C₅-Cycloalkyl bilden;
oder alternativ R₃ und R₄ zusammen mit den Kohlenstoffatomen, an die sie gebunden sind, ein C₃-C₅-Cycloalkyl bilden;
R₆, R₇, R₈, R₉ und R₁₁ jeweils unabhängig voneinander ausgewählt sind aus der Gruppe bestehend aus H, Hydroxyl, Halogen und Deuterium;
R₁₀ ausgewählt ist aus der Gruppe bestehend aus H, unsubstituiertem C₁-C₃-Alkyl und C₁-C₃-Alkyl, das mit einem oder mehreren Halogenen substituiert ist;
jedes R₁₂ und R₁₃ unabhängig ausgewählt ist aus der Gruppe bestehend aus H, Halogen, Deuterium, unsubstituiertem C₁-C₃-Alkyl und C₁-C₃-Alkyl, das mit Hydroxyl oder einem oder mehreren Halogenen substituiert ist; und
R₁₄, R₁₅ und R₁₆ jeweils unabhängig ausgewählt sind aus der Gruppe bestehend aus H, unsubstituiertem C₁-C₃-Alkyl oder C₁-C₃-Alkyl, das mit einem oder mehreren Halogenen substituiert ist; und
jedes R₁₇ und R₁₈ unabhängig ausgewählt ist aus der Gruppe bestehend aus H, unsubstituiertem C₁-C₃-Alkyl oder C₁-C₃-Alkyl, das mit einem oder mehreren Halogenen substituiert ist.

2. Verbindung nach Anspruch 1, wobei m 1 oder 2 ist, vorzugsweise wobei m 1 ist.

3. Verbindung von Formel II oder ein pharmazeutisch verträgliches Salz davon: 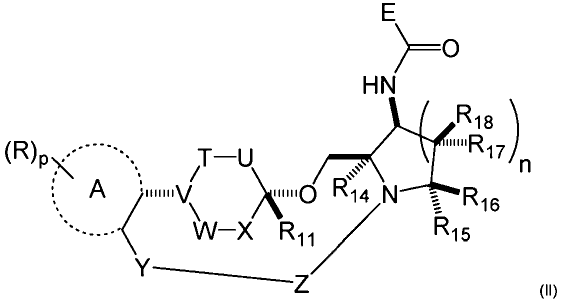 wobei:
Ring A ausgewählt ist aus der Gruppe bestehend aus Phenyl, Pyridinyl, Pyridazinyl, Pyrimidinyl, Pyrazinyl und Triazinyl;
n 1, 2 oder 3 ist;
E ausgewählt ist aus der Gruppe bestehend aus NRₐR_{b}, C(=O)NRₐR_{b}, C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrigem Heterocyclyl, C₁-C₃ Alkylen-(4- bis 10-gliedriges Heterocyclyl), C₆-C₁₀-Aryl, C₁-C₃-Alkylen-(C₆-C₁₀-Aryl), 5- bis 10-gliedrigem Heteroaryl und C₁-C₃-Alkylen-(5- bis 10-gliedriges Heteroaryl), wobei das C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrige Heterocyclyl, C₁-C₃-Alkylen-(4- bis 10-gliedrige Heterocyclyl), C₆-C₁₀-Aryl, C₁-C₃-Alkylen-(C₆-C₁₀-Aryl), 5- bis 10-gliedrige Heteroaryl oder C₁-C₃-Alkylen-(5- bis 10-gliedriges Heteroaryl) unsubstituiert oder mit einem oder mehreren Halogenen, Hydroxyl, C₁-C₃-Alkyl oder C₁-C₃-Alkoxyl substituiert ist;
T CR₁R₂ oder O ist;
W CR₄R₅ oder O ist;
U CR₆R₇ ist;
X CR₈R₉ ist;
V CR₃ oder N ist;
Y NR₁₀, O ist oder fehlt;
Z (CR₁₂R₁₃)ₘ ist;
jedes R unabhängig ausgewählt ist aus der Gruppe bestehend aus Halogen, Deuterium, Hydroxyl, Cyano, unsubstituiertem C₁-C₃-Alkyl und C₁-C₃-Alkyl, das mit einem oder mehreren Halogenen oder Deuterium substituiert ist;
p 0, 1, 2, 3 oder 4 ist;
Rₐ und R_{b} jeweils unabhängig H oder unsubstituiertes C₁-C₃-Alkyl sind;
m 2, 3, 4 oder 5 ist, wenn Y fehlt; oder
m 1, 2, 3 oder 4 ist, wenn Y NR₁₀ oder O ist;
und wobei weiter:
R₁, R₂, R₄ und R₅ jeweils unabhängig voneinander ausgewählt sind aus der Gruppe bestehend aus H, Hydroxyl, Halogen und Deuterium;
oder alternativ R₂ und R₅ zusammen mit den Kohlenstoffatomen, an die sie gebunden sind, eine Einfachbindung bilden;
R₃ ausgewählt ist aus der Gruppe bestehend aus H, Deuterium, Halogen, Hydroxyl und Cyano;
oder alternativ R₃ und R₁ zusammen mit den Kohlenstoffatomen, an die sie gebunden sind, ein C₃-C₅-Cycloalkyl bilden;
oder alternativ R₃ und R₄ zusammen mit den Kohlenstoffatomen, an die sie gebunden sind, ein C₃-C₅-Cycloalkyl bilden;
R₆, R₇, R₈, R₉ und R₁₁ jeweils unabhängig voneinander ausgewählt sind aus der Gruppe bestehend aus H, Hydroxyl, Halogen und Deuterium;
R₁₀ ausgewählt ist aus der Gruppe bestehend aus H, unsubstituiertem C₁-C₃-Alkyl und C₁-C₃-Alkyl, das mit einem oder mehreren Halogenen substituiert ist;
jedes R₁₂ und R₁₃ unabhängig ausgewählt ist aus der Gruppe bestehend aus H, Halogen, Deuterium, unsubstituiertem C₁-C₃-Alkyl und C₁-C₃-Alkyl, das mit Hydroxyl oder einem oder mehreren Halogenen substituiert ist; und
R₁₄, R₁₅ und R₁₆ jeweils unabhängig ausgewählt sind aus der Gruppe bestehend aus H, unsubstituiertem C₁-C₃-Alkyl oder C₁-C₃-Alkyl, das mit einem oder mehreren Halogenen substituiert ist; und
jedes R₁₇ und R₁₈ unabhängig ausgewählt ist aus der Gruppe bestehend aus H, unsubstituiertem C₁-C₃-Alkyl oder C₁-C₃-Alkyl, das mit einem oder mehreren Halogenen substituiert ist.

4. Verbindung nach Anspruch 3, wobei m 2 oder 3 ist, vorzugsweise wobei m 2 ist.

5. Verbindung nach einem der Ansprüche 1-4, wobei n 1 ist, vorzugsweise wobei n 2 ist.

6. Verbindung nach einem der Ansprüche 1-5, wobei Ring A Phenyl oder Pyridinyl ist.

7. Verbindung nach einem der Ansprüche 1-6, wobei Y O ist oder fehlt.

8. Verbindung nach einem der Ansprüche 1-7, wobei:
(i) T CR₁R₂ oder O ist;
(ii) W CR₄R₅ oder O ist; und/oder
(iii) V CR₃ ist.

9. Verbindung nach einem der Ansprüche 1-8, wobei E aus der Gruppe ausgewählt ist, bestehend aus:
(i) C(=O)NRₐR_{b}, C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrigem Heterocyclyl, C₁-C₃-Alkylen-(4-bis 10-gliedriges Heterocyclyl), C₆-C₁₀-Aryl, C₁-C₃-Alkylen-(C₆-C₁₀-Aryl), 5- bis 10-gliedriges Heteroaryl und C₁-C₃-Alkylen-(5- bis 10-gliedriges Heteroaryl), wobei das C₁-C₃-Alkylen-NRₐR_{b}, C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrige Heterocyclyl, C₁-C₃-Alkylen-(4- bis 10-gliedrige Heterocyclyl), C₆-C₁₀-Aryl, C₁-C₃-Alkylen-(C₆-C₁₀-Aryl), 5- bis 10-gliedrige Heteroaryl oder C₁-C₃-Alkylen-(5- bis 10-gliedrige Heteroaryl) unsubstituiert oder mit einem oder mehreren Halogenen, Hydroxyl, C₁-C₃-Alkyl oder C₁-C₃-Alkoxyl substituiert ist,
wobei optional E ausgewählt ist aus der Gruppe bestehend aus C₁-C₃-Alkyl, C₂-C₄-Alkenyl, C₂-C₄-Alkinyl, C₃-C₈-Cycloalkyl, C₁-C₃-Alkylen-(C₃-C₈ Cycloalkyl), 4- bis 10-gliedrigem Heterocyclyl, C₁-C₃-Alkylen-(4- bis 10-gliedriges Heterocyclyl), C₆-C₁₀-Aaryl, C₁-C₃-Alkylen-(C₆-C₁₀-Aryl), 5- bis 10-gliedrigem Heteroaryl und C₁-C₃-Alkylen-(5- bis 10-gliedriges Heteroaryl), wobei das C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), 4- bis 10-gliedrige Heterocyclyl, C₁-C₃-Alkylen-(4- bis 10-gliedriges Heterocyclyl), C₆-C₁₀-Aryl, C₁-C₃-Alkylen-(C₆-C₁₀-Aryl), 5- bis 10-gliedrige Heteroaryl oder C₁-C₃-Alkylen-(5- bis 10-gliedriges Heteroaryl) unsubstituiert oder mit einem oder mehreren Halogenen, Hydroxyl, C₁-C₃-Alkyl oder C₁-C₃-Alkoxyl substituiert ist,
wobei vorzugsweise E ausgewählt ist aus der Gruppe bestehend aus C₁-C₃-Alkyl, C₂-C₄-Alkenyl, C₂-C₄-Alkinyl, C₃-C₈-Cycloalkyl, C₁-C₃-Alkylen-(C₃-C₈-Cycloalkyl), wobei das C₁-C₃-Alkyl, C₂-C₄-Alkenyl, C₂-C₄-Alkinyl, C₃-C₅-Cycloalkyl oder C₁-C₃-Alkylen-(C₃-C₈-Cycloalkyl) unsubstituiert oder mit einem oder mehreren Halogenen, Hydroxyl, C₁-C₃-Alkyl oder C₁-C₃-Alkoxyl substituiert ist; oder
(ii) 4- bis 10-gliedriges Heterocyclyl, C₁-C₃-Alkylen-(4- bis 10-gliedriges Heterocyclyl), C₆-C₁₀-Aryl, C₁-C₃-Alkylen-(C₆-C₁₀-Aryl), 5- bis 10-gliedriges Heteroaryl und C₁-C₃-Alkylen-(5- bis 10-gliedriges Heteroaryl), wobei das 4- bis 10-gliedrige Heterocyclyl, C₁-C₃-Alkylen-(4- bis 10-gliedriges Heterocyclyl), C₆-C₁₀-Aryl, C₁-C₃-Alkylen-(C₆-C₁₀-Aryl), 5- bis 10-gliedrige Heteroaryl oder C₁-C₃ -Alkylen-(5- bis 10-gliedriges Heteroaryl) unsubstituiert oder mit einem oder mehreren Halogenen, Hydroxy, C₁-C₃-Alkyl oder C₁-C₃-Alkoxy substituiert ist,
vorzugsweise wobei E aus der Gruppe ausgewählt ist, bestehend aus 4- bis 7-gliedrigem Heterocyclyl und C₁-C₃-Alkylen-(4- bis 7-gliedriges Heterocyclyl), wobei das 4- bis 7-gliedrige Heterocyclyl oder C₁-C₃ -Alkylen-(4- bis 7-gliedriges Heterocyclyl) unsubstituiert oder mit einem oder mehreren Halogenen, Hydroxy, C₁-C₃-Alkyl oder C₁-C₃-Alkoxy substituiert ist.

10. Verbindung nach einem der Ansprüche 1-7, wobei T CR₁R₂ ist, W CR₄R₅ ist und V CR₃ ist.

11. Verbindung oder pharmazeutisch verträgliches Salz davon nach Anspruch 1 und 3, ausgewählt aus der Gruppe bestehend aus: 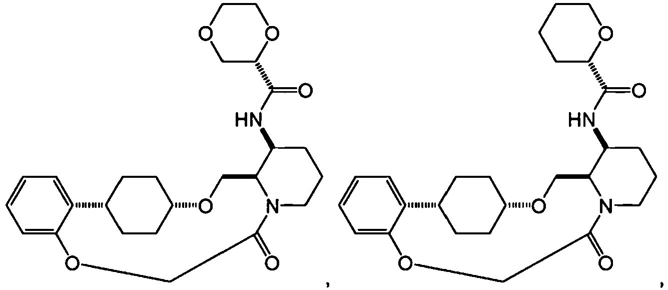 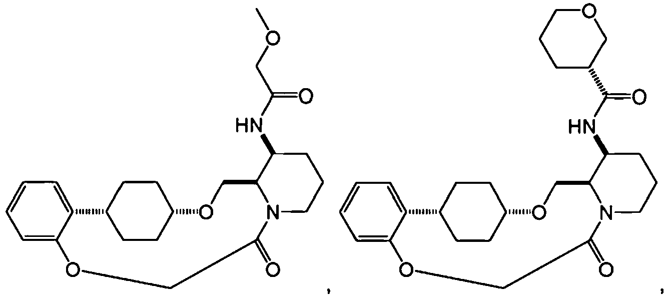 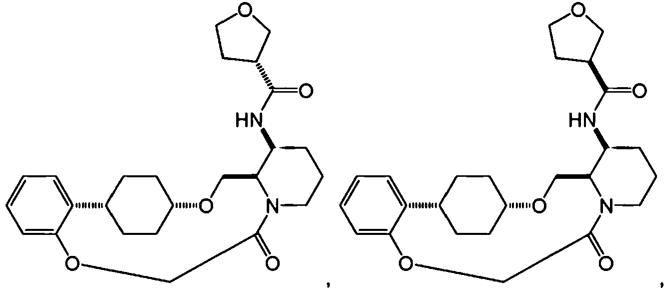 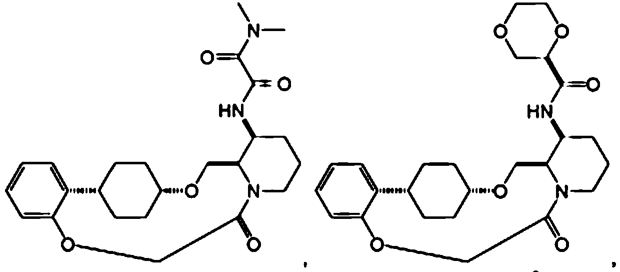 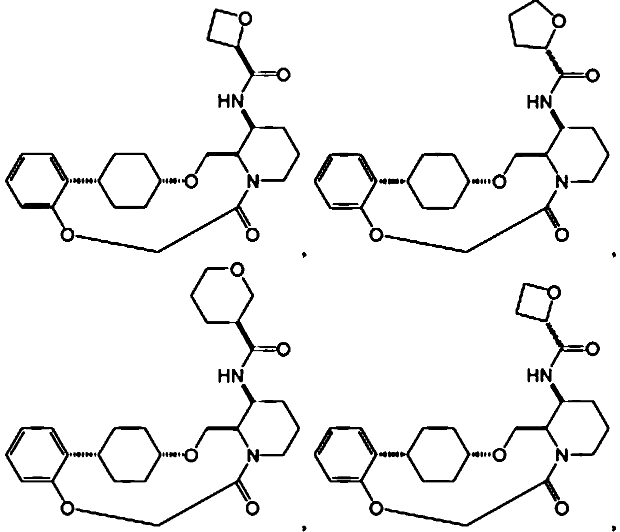 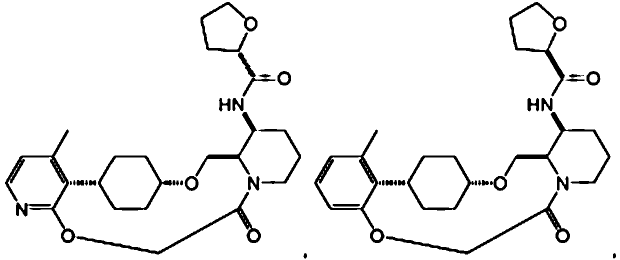 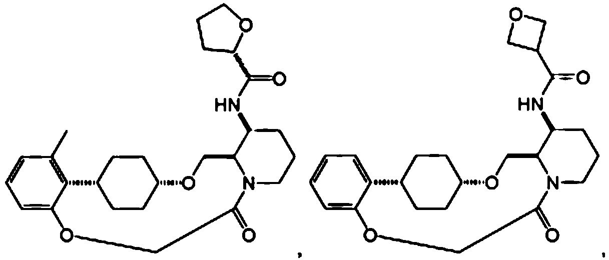 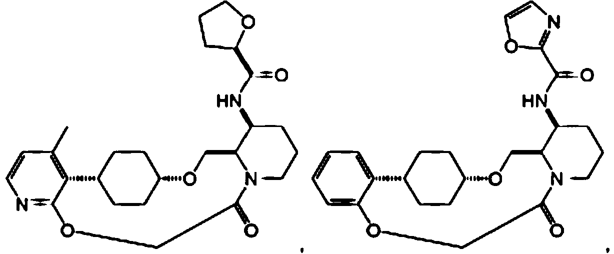 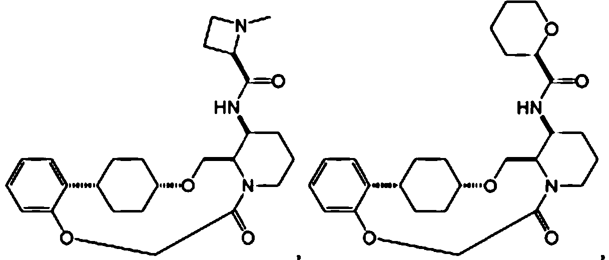 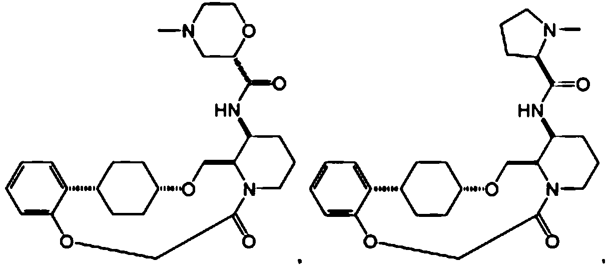 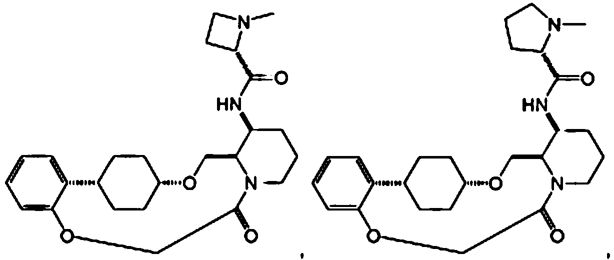 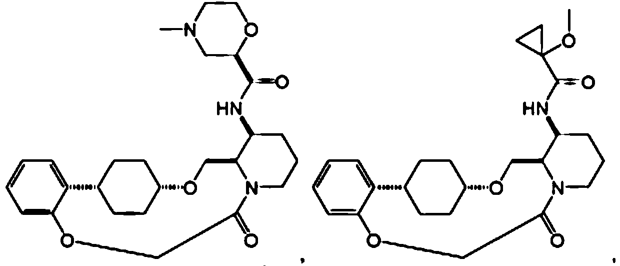 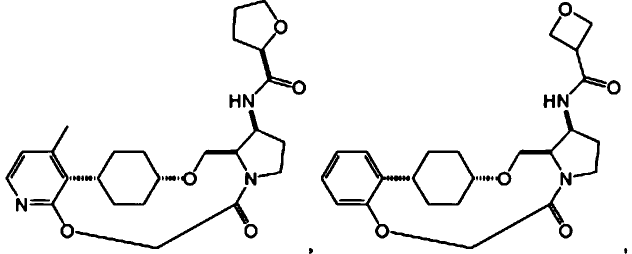 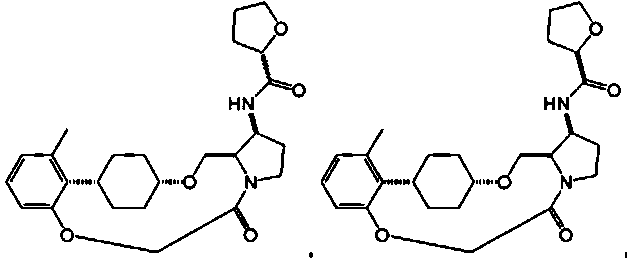 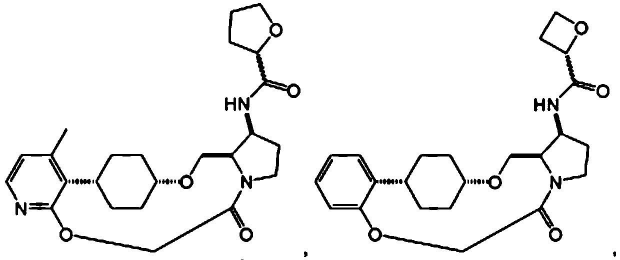 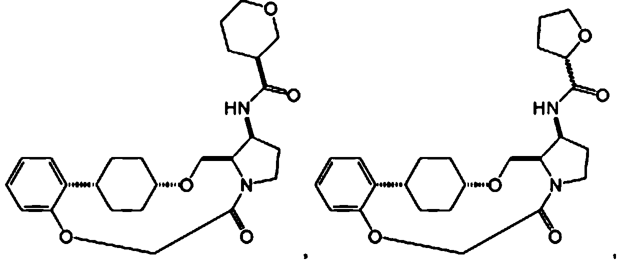 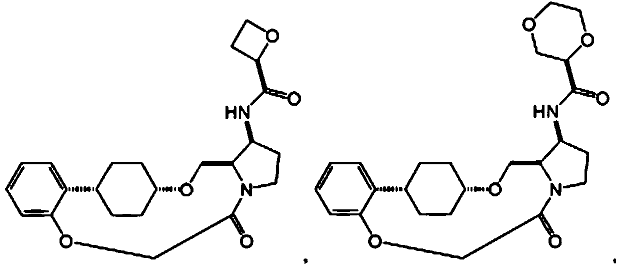 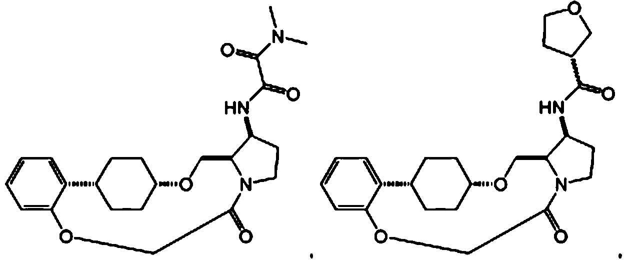 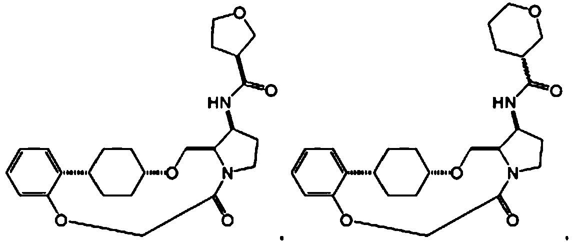 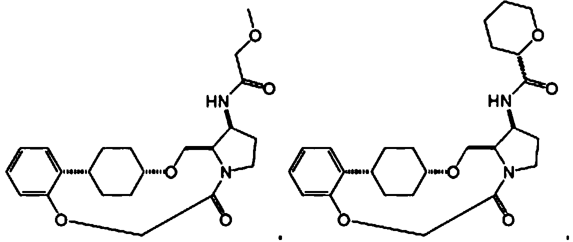 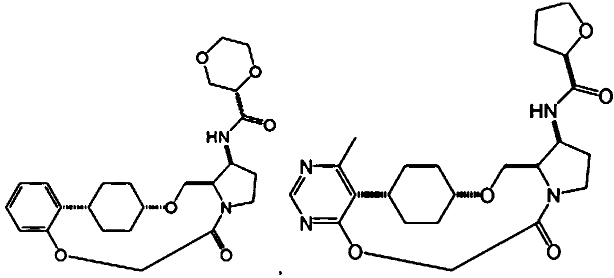 und

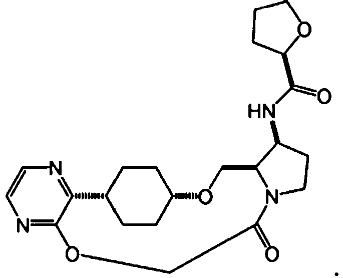

12. Verbindung oder pharmazeutisch verträgliches Salz davon nach Anspruch 1 und 3, ausgewählt aus der Gruppe bestehend aus:

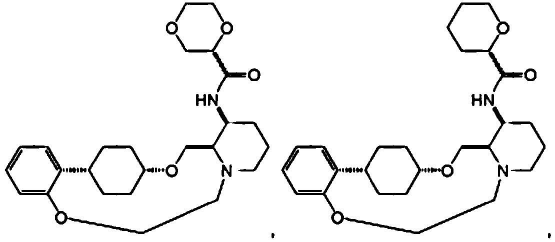

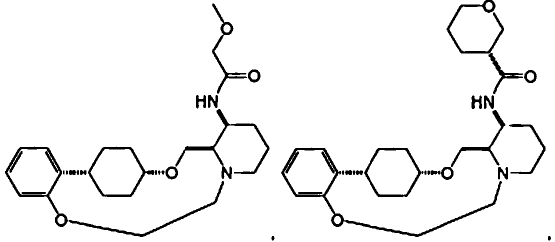

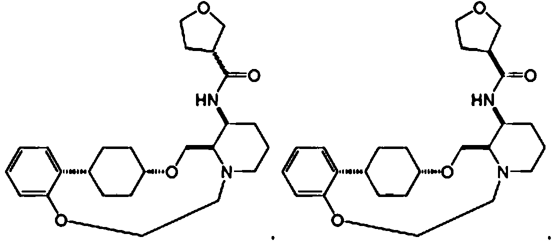

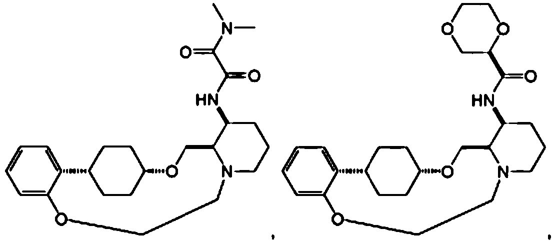

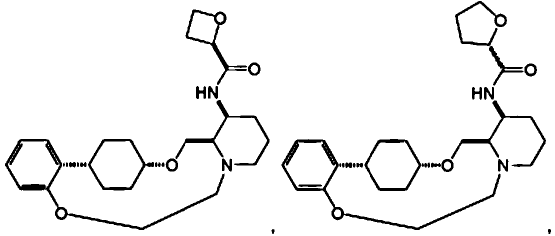

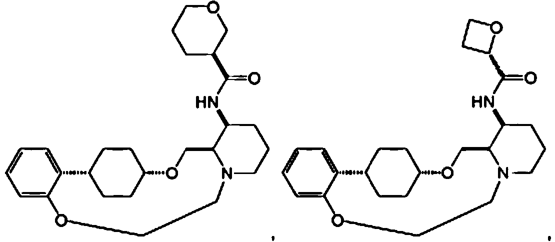

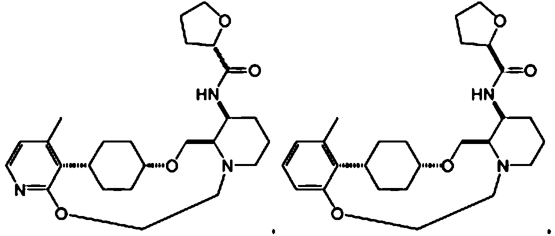

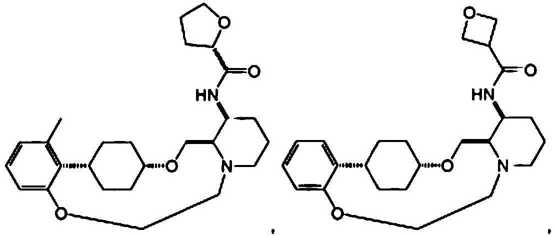

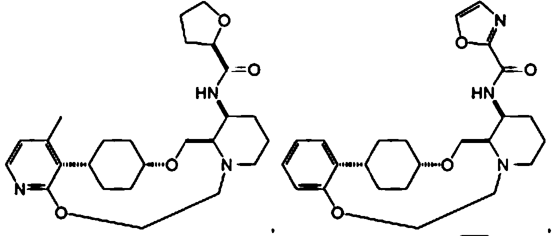

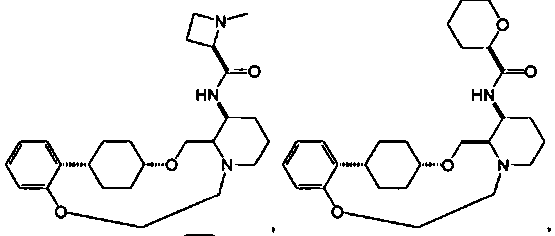

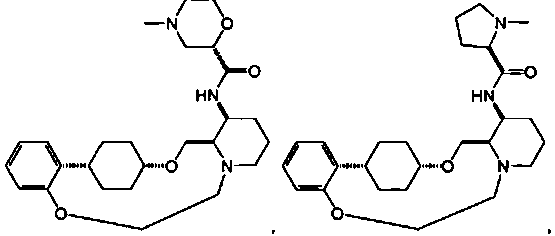

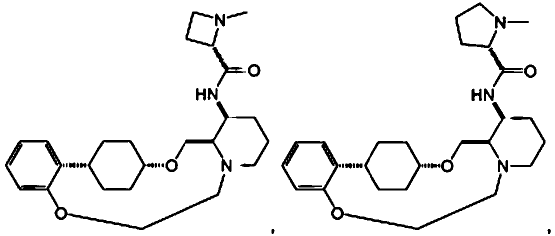

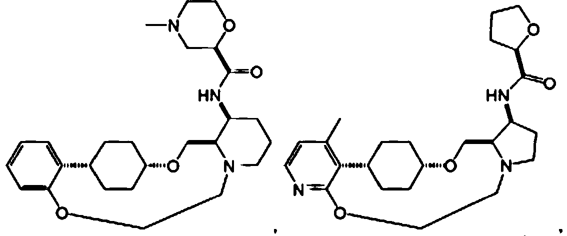

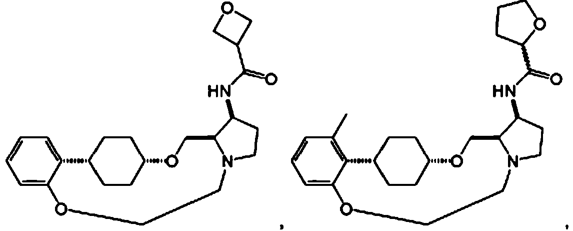

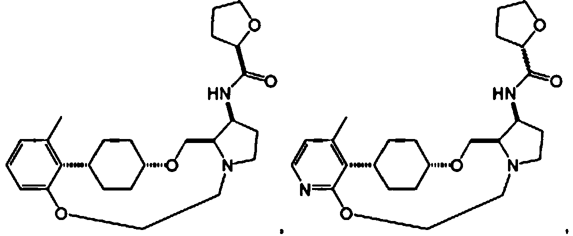

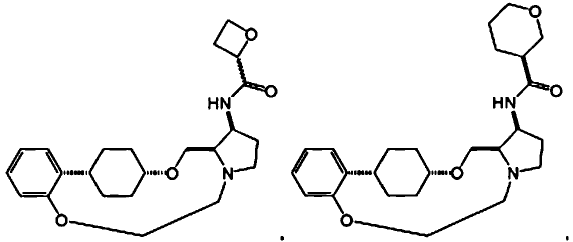

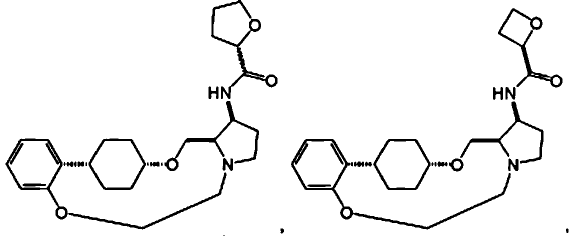

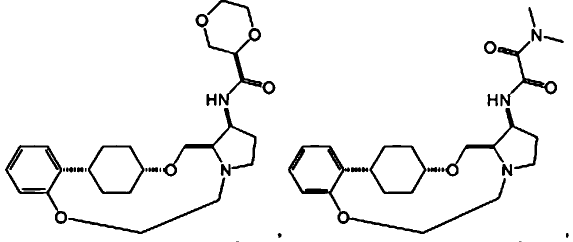

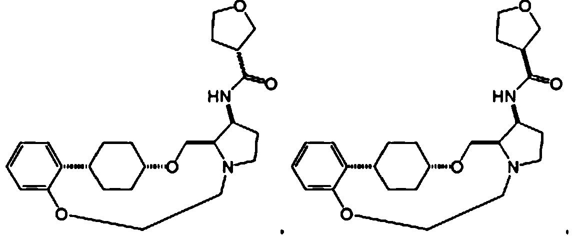

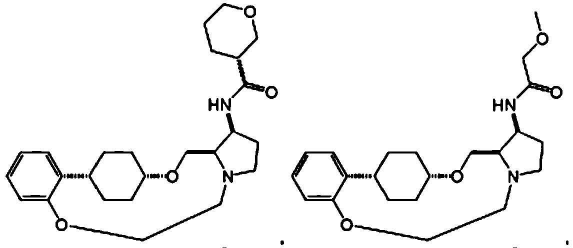

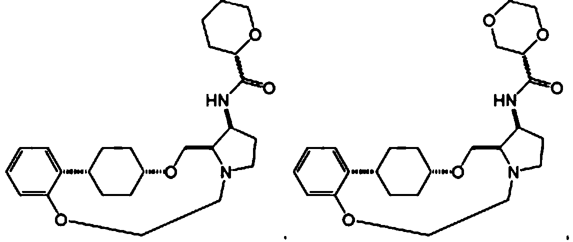

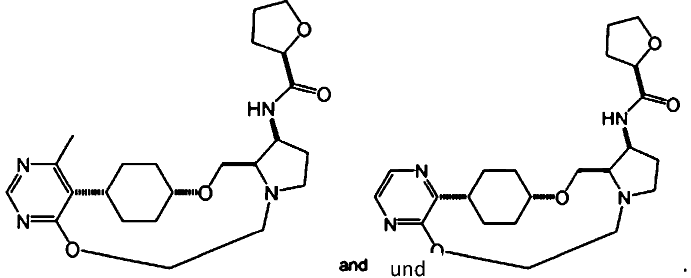

13. Pharmazeutische Zusammensetzung, die eine Verbindung nach einem der Ansprüche 1-12 oder ein pharmazeutisch verträgliches Salz davon und einen pharmazeutisch verträglichen Träger umfasst.

14. Verbindung nach einem der Ansprüche 1-12 oder ein pharmazeutisch verträgliches Salz davon oder eine Zusammensetzung nach Anspruch 13 zur Verwendung in einer Behandlung von Narkolepsie.

15. Verbindung nach einem der Ansprüche 1-12 oder ein pharmazeutisch verträgliches Salz davon oder eine Zusammensetzung nach Anspruch 13 zur Verwendung in einer Behandlung von Kataplexie.

## Revendications

1. Composé de Formule I ou sel pharmaceutiquement acceptable de celui-ci : 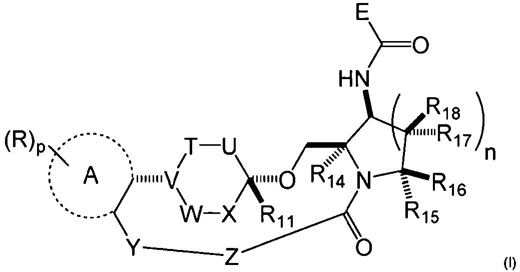 dans lequel :
le cycle A est sélectionné dans le groupe consistant en phényle, pyridinyle, pyridazinyle, pyrimidinyle, pyrazinyle et triazinyle ;
n est 1, 2 ou 3 ;
E est sélectionné dans le groupe consistant en NRₐR_{b}, C(=O)NRₐR_{b}, alkylène en C₁-C₃-NRₐR_{b}, alkyle en C₁-C₃, alcényle en C₂-C₄, alcynyle en C₂-C₄, cycloalkyle en C₃-C₈, alkylène en C₁-C₃-(cycloalkyle en C₃-C₈), hétérocyclyle à 4 à 10 chaînons, alkylène en C₁-C₃-(hétérocyclyle à 4 à 10 chaînons), aryle en C₆-C₁₀, alkylène en C₁-C₃-(aryle en C₆-C₁₀), hétéroaryle à 5 à 10 chaînons et alkylène en C₁-C₃-(hétéroaryle à 5 à 10 chaînons), dans lequel l'alkylène en C₁-C₃-NRₐR_{b}, l'alkyle en C₁-C₃, l'alcényle en C₂-C₄, l'alcynyle en C₂-C₄, le cycloalkyle en C₃-C₈, l'alkylène en C₁-C₃-(cycloalkyle en C₃-C₈), l'hétérocyclyle à 4 à 10 chaînons, l'alkylène en C₁-C₃-(hétérocyclyle à 4 à 10 chaînons), l'aryle en C₆-C₁₀, l'alkylène en C₁-C₃-(aryle en C₆-C₁₀), l'hétéroaryle à 5 à 10 chaînons ou l'alkylène en C₁-C₃-(hétéroaryle à 5 à 10 chaînons) est non substitué ou substitué par un ou plusieurs halogènes, hydroxyles, alkyles en C₁-C₃ ou alcoxyles en C₁-C₃ ;
T est CR₁R₂ ou O ;
W est CR₄R₅ ou O ;
U est CR₆R₇ ;
X est CR₈R₉ ;
V est CR₃ ou N ;
Y est NR₁₀, O ou absent ;
Z est (CR₁₂R₁₃)ₘ ;
chaque R est, indépendamment, sélectionné dans le groupe consistant en halogène, deutérium, hydroxyle, cyano, alkyle en C₁-C₃ non substitué et alkyle en C₁-C₃ substitué par un ou plusieurs halogènes ou deutérium ;
p est 0, 1, 2, 3 ou 4 ;
Rₐ et R_{b} sont chacun, indépendamment, H ou alkyle en C₁-C₃ non substitué ;
m est 1, 2, 3 ou 4 ;
et en outre dans lequel :
R₁, R₂, R₄ et R₅ sont chacun, indépendamment, sélectionnés dans le groupe consistant en H, hydroxyle, halogène et deutérium ;
ou, alternativement, R₂ et R₈, conjointement avec les atomes de carbone auxquels ils sont liés, forment une liaison simple ;
R₃ est sélectionné dans le groupe consistant en H, deutérium, halogène, hydroxyle et cyano ;
ou, en variante, R₃ et R₁, conjointement avec les atomes de carbone auxquels ils sont liés, forment un cycloalkyle en C₃-C₅ ;
ou, en variante, R₃ et R₄, conjointement avec les atomes de carbone auxquels ils sont liés, forment un cycloalkyle en C₃-C₅ ;
R₆, R₇, R₈, R₉ et R₁₁ sont chacun, indépendamment, sélectionnés dans le groupe consistant en H, hydroxyle, halogène et deutérium ;
R₁₀ est sélectionné dans le groupe consistant en H, alkyle en C₁-C₃ non substitué et alkyle en C₁-C₃ substitué par un ou plusieurs halogènes ;
chaque R₁₂ et R₁₃ est, indépendamment, sélectionné dans le groupe consistant en H, halogène, deutérium, alkyle en C₁-C₃ non substitué et alkyle en C₁-C₃ substitué par hydroxyle ou un ou plusieurs halogènes ; et
R₁₄, R₁₅ et R₁₆ sont chacun, indépendamment, sélectionnés dans le groupe consistant en H, alkyle en C₁-C₃ non substitué ou alkyle en C₁-C₃ substitué par un ou plusieurs halogènes ; et
chaque R₁₇ et R₁₈ est, indépendamment, sélectionné dans le groupe consistant en H, alkyle en C₁-C₃ non substitué ou alkyle en C₁-C₃ substitué par un ou plusieurs halogènes.

2. Composé selon la revendication 1, dans lequel m est 1 ou 2, de préférence dans lequel m est 1.

3. Composé de Formule II ou sel pharmaceutiquement acceptable de celui-ci : 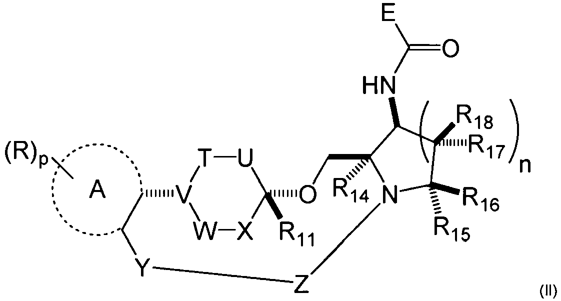 dans lequel :
le cycle A est sélectionné dans le groupe consistant en phényle, pyridinyle, pyridazinyle, pyrimidinyle, pyrazinyle et triazinyle ;
n est 1, 2 ou 3 ;
E est sélectionné dans le groupe consistant en NRₐR_{b}, C(=O)NRₐR_{b}, alkylène en C₁-C₃-NRₐR_{b}, alkyle en C₁-C₃, alcényle en C₂-C₄, alcynyle en C₂-C₄, cycloalkyle en C₃-C₈, alkylène en C₁-C₃-(cycloalkyle en C₃-C₈), hétérocyclyle à 4 à 10 chaînons, alkylène en C₁-C₃-(hétérocyclyle à 4 à 10 chaînons), aryle en C₆-C₁₀, alkylène en C₁-C₃-(aryle en C₆-C₁₀), hétéroaryle à 5 à 10 chaînons et alkylène en C₁-C₃-(hétéroaryle à 5 à 10 chaînons), dans lequel l'alkylène en C₁-C₃-NRₐR_{b}, l'alkyle en C₁-C₃, l'alcényle en C₂-C₄, l'alcynyle en C₂-C₄, le cycloalkyle en C₃-C₈, l'alkylène en C₁-C₃-(cycloalkyle en C₃-C₈), l'hétérocyclyle à 4 à 10 chaînons, l'alkylène en C₁-C₃-(hétérocyclyle à 4 à 10 chaînons), l'aryle en C₆-C₁₀, l'alkylène en C₁-C₃-(aryle en C₆-C₁₀), l'hétéroaryle à 5 à 10 chaînons ou l'alkylène en C₁-C₃-(hétéroaryle à 5 à 10 chaînons) est non substitué ou substitué par un ou plusieurs halogènes, hydroxyles, alkyles en C₁-C₃ ou alcoxyles en C₁-C₃ ;
T est CR₁R₂ ou O ;
W est CR₄R₅ ou O ;
U est CR₆R₇ ;
X est CR₈R₉ ;
V est CR₃ ou N ;
Y est NR₁₀, O ou absent ;
Z est (CR₁₂R₁₃)ₘ ;
chaque R est, indépendamment, sélectionné dans le groupe consistant en halogène, deutérium, hydroxyle, cyano, alkyle en C₁-C₃ non substitué et alkyle en C₁-C₃ substitué par un ou plusieurs halogènes ou deutérium ;
p est 0, 1, 2, 3 ou 4 ;
Rₐ et R_{b} sont chacun, indépendamment, H ou alkyle en C₁-C₃ non substitué ;
m est 2, 3, 4 ou 5 lorsque Y est absent ; ou
m est 1, 2, 3 ou 4 lorsque Y est NR₁₀ ou O ;
et en outre dans lequel :
R₁, R₂, R₄ et R₅ sont chacun, indépendamment, sélectionnés dans le groupe consistant en H, hydroxyle, halogène et deutérium ;
ou, alternativement, R₂ et R₈, conjointement avec les atomes de carbone auxquels ils sont liés, forment une liaison simple ;
R₃ est sélectionné dans le groupe consistant en H, deutérium, halogène, hydroxyle et cyano ;
ou, en variante, R₃ et R₁, conjointement avec les atomes de carbone auxquels ils sont liés, forment un cycloalkyle en C₃-C₅ ;
ou, en variante, R₃ et R₄, conjointement avec les atomes de carbone auxquels ils sont liés, forment un cycloalkyle en C₃-C₅ ;
R₆, R₇, R₈, R₉ et R₁₁ sont chacun, indépendamment, sélectionnés dans le groupe consistant en H, hydroxyle, halogène et deutérium ;
R₁₀ est sélectionné dans le groupe consistant en H, alkyle en C₁-C₃ non substitué et alkyle en C₁-C₃ substitué par un ou plusieurs halogènes ;
chaque R₁₂ et R₁₃ est, indépendamment, sélectionné dans le groupe consistant en H, halogène, deutérium, alkyle en C₁-C₃ non substitué et alkyle en C₁-C₃ substitué par un hydroxyle ou un ou plusieurs halogènes ; et
R₁₄, R₁₅ et R₁₆ sont chacun, indépendamment, sélectionnés dans le groupe consistant en H, alkyle en C₁-C₃ non substitué ou alkyle en C₁-C₃ substitué par un ou plusieurs halogènes ; et
chaque R₁₇ et R₁₈ est, indépendamment, sélectionné dans le groupe consistant en H, alkyle en C₁-C₃ non substitué ou alkyle en C₁-C₃ substitué par un ou plusieurs halogènes.

4. Composé selon la revendication 3, dans lequel m est 2 ou 3, de préférence dans lequel m est 2.

5. Composé selon l'une quelconque des revendications 1-4, dans lequel n est 1, de préférence dans lequel n est 2.

6. Composé selon l'une quelconque des revendications 1-5, dans lequel le cycle A est un phényle ou un pyridinyle.

7. Composé selon l'une quelconque des revendications 1-6, dans lequel Y est O, ou absent.

8. Composé selon l'une quelconque des revendications 1-7, dans lequel :
(i) T est CR₁R₂ ou O ;
(ii) W est CR₄R₅ ou O ; et/ou
(iii) V est CR₃.

9. Composé selon l'une quelconque des revendications 1-8, dans lequel E est sélectionné dans le groupe consistant en :
(i) C(=O)NRₐR_{b}, alkylène en C₁-C₃-NRₐR_{b}, alkyle en C₁-C₃, alcényle en C₂-C₄, alcynyle en C₂-C₄, cycloalkyle en C₃-C₈, alkylène en C₁-C₃-(cycloalkyle en C₃-C₈), hétérocyclyle à 4 à 10 chaînons, alkylène en C₁-C₃-(hétérocyclyle à 4 à 10 chaînons), aryle en C₆-C₁₀, alkylène en C₁-C₃-(aryle en C₆-C₁₀), hétéroaryle à 5 à 10 chaînons et alkylène en C₁-C₃-(hétéroaryle à 5 à 10 chaînons), dans lequel l'alkylène en C₁-C₃-NRₐR_{b}, l'alkyle en C₁-C₃, l'alcényle en C₂-C₄, l'alcynyle en C₂-C₄, le cycloalkyle en C₃-C₈, l'alkylène en C₁-C₃-(cycloalkyle en C₃-C₈), l'hétérocyclyle à 4 à 10 chaînons, l'alkylène en C₁-C₃-(hétérocyclyle à 4 à 10 chaînons), l'aryle en C₆-C₁₀, l'alkylène en C₁-C₃-(aryle en C₆-C₁₀), l'hétéroaryle à 5 à 10 chaînons ou l'alkylène en C₁-C₃-(hétéroaryle à 5 à 10 chaînons) est non substitué ou substitué par un ou plusieurs halogènes, hydroxyles, alkyles en C₁-C₃ ou alcoxyles en C₁-C₃,
facultativement dans lequel E est sélectionné dans le groupe consistant en alkyle en C₁-C₃, alcényle en C₂-C₄, alcynyle en C₂-C₄, cycloalkyle en C₃-C₈, alkylène en C₁-C₃-(cycloalkyle en C₃-C₈), hétérocyclyle à 4 à 10 chaînons, alkylène en C₁-C₃-(hétérocyclyle à 4 à 10 chaînons), aryle en C₆-C₁₀, alkylène en C₁-C₃-(aryle en C₆-C₁₀), hétéroaryle à 5 à 10 chaînons et alkylène en C₁-C₃-(hétéroaryle à 5 à 10 chaînons), dans lequel l'alkyle en C₁-C₃, l'alcényle en C₂-C₄, l'alcynyle en C₂-C₄, le cycloalkyle en C₃-C₈, l'alkylène en C₁-C₃-(cycloalkyle en C₃-C₈), l'hétérocyclyle à 4 à 10 chaînons, l'alkylène en C₁-C₃-(hétérocyclyle à 4 à 10 chaînons), l'aryle en C₆-C₁₀, l'alkylène en C₁-C₃-(aryle en C₆-C₁₀), l'hétéroaryle à 5 à 10 chaînons ou l'alkylène en C₁-C₃-(hétéroaryle à 5 à 10 chaînons) est non substitué ou substitué par un ou plusieurs halogènes, hydroxyles, alkyles en C₁-C₃ ou alcoxyles en C₁-C₃,
de préférence dans lequel E est sélectionné dans le groupe consistant en alkyle en C₁-C₃, alcényle en C₂-C₄, alcynyle en C₂-C₄, cycloalkyle en C₃-C₈, et alkylène en C₁-C₃-(cycloalkyle en C₃-C₈), dans lequel l'alkyle en C₁-C₃, l'alcényle en C₂-C₄, l'alcynyle en C₂-C₄, le cycloalkyle en C₃-C₈, l'alkylène en C₁-C₃-(cycloalkyle en C₃-C₈) est non substitué ou substitué par un ou plusieurs halogènes, hydroxyles, alkyles en C₁-C₃ ou alcoxyles en C₁-C₃ ; ou
(ii) hétérocyclyle à 4 à 10 chaînons, alkylène en C₁-C₃-(hétérocyclyle à 4 à 10 chaînons), aryle en C₆-C₁₀, alkylène en C₁-C₃-(aryle en C₆-C₁₀), hétéroaryle à 5 à 10 chaînons et alkylène en C₁-C₃-(hétéroaryle à 5 à 10 chaînons), dans lequel l'hétérocyclyle à 4 à 10 chaînons, l'alkylène en C₁-C₃-(hétérocyclyle à 4 à 10 chaînons), l'aryle en C₆-C₁₀, l'alkylène en C₁-C₃-(aryle en C₆-C₁₀), l'hétéroaryle à 5 à 10 chaînons ou l'alkylène en C₁-C₃-(hétéroaryle à 5 à 10 chaînons) est non substitué ou substitué par un ou plusieurs halogènes, hydroxyles, alkyles en C₁-C₃ ou alcoxyles en C₁-C₃,
de préférence dans lequel E est sélectionné dans le groupe consistant en hétérocyclyle à 4 à 7 chaînons et alkylène en C₁-C₃-(hétérocyclyle à 4 à 7 chaînons), dans lequel l'hétérocyclyle à 4 à 7 chaînons ou l'alkylène en C₁-C₃-(hétérocyclyle à 4 à 7 chaînons) est non substitué ou substitué par un ou plusieurs halogènes, hydroxyles, alkyles en C₁-C₃ ou alcoxyles en C₁-C₃.

10. Composé selon l'une quelconque des revendications 1-7, dans lequel T est CR₁R₂, W est CR₄R₅ et V est CR₃.

11. Composé ou sel pharmaceutiquement acceptable de celui-ci selon les revendications 1 et 3, sélectionné dans le groupe consistant en :

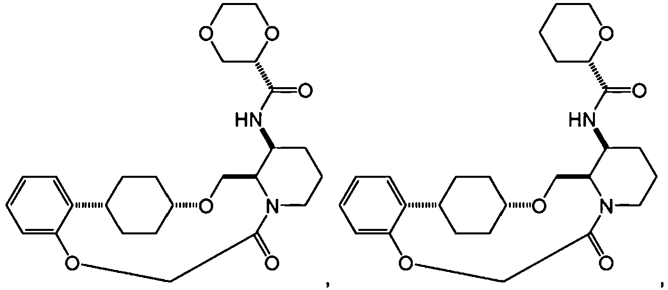

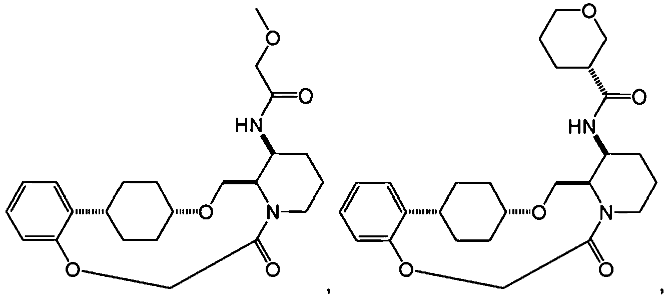

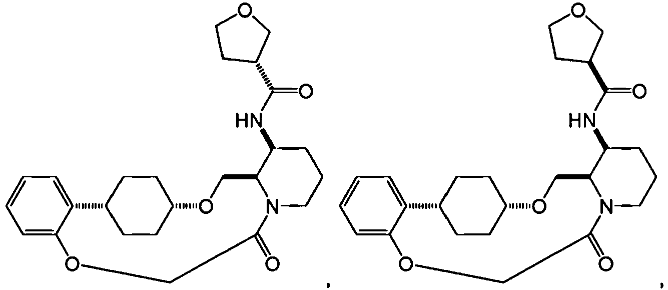

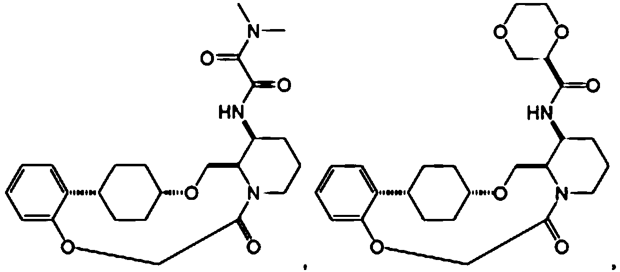

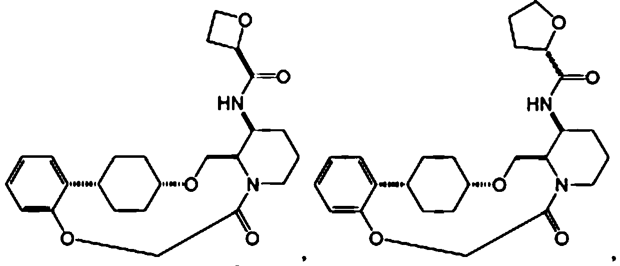

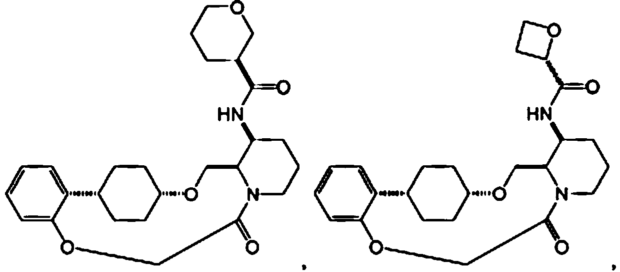

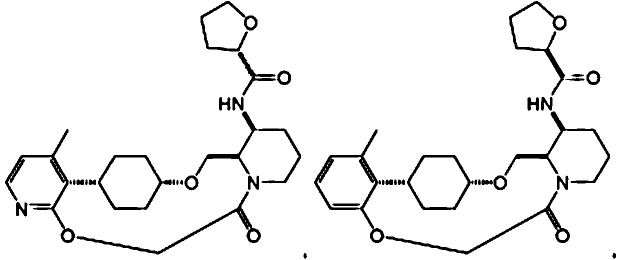

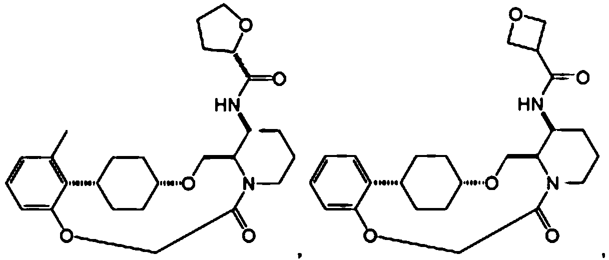

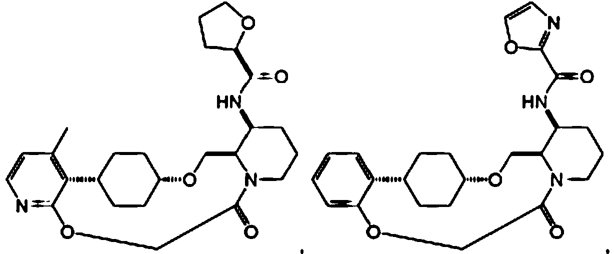

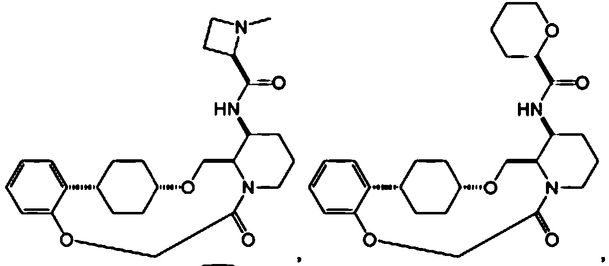

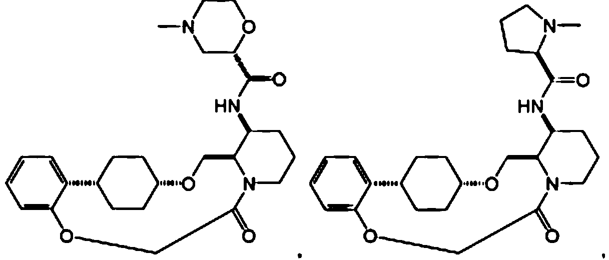

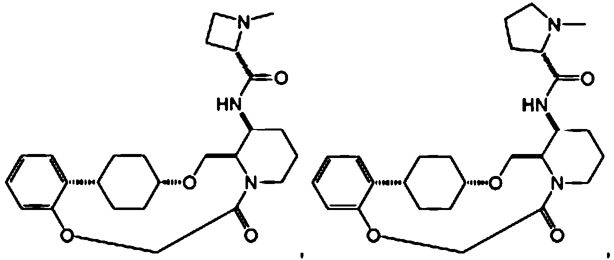

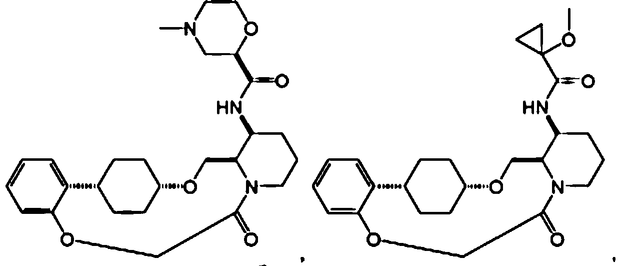

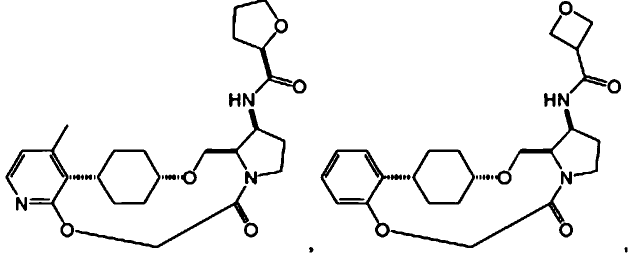

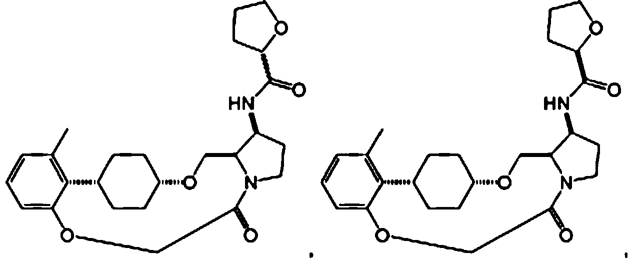

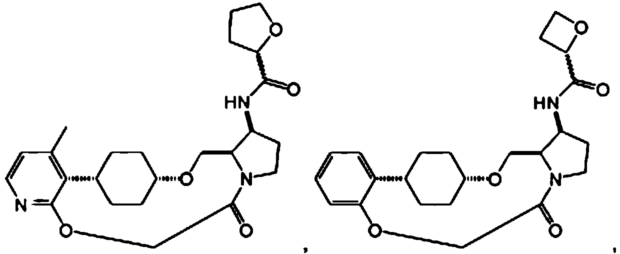

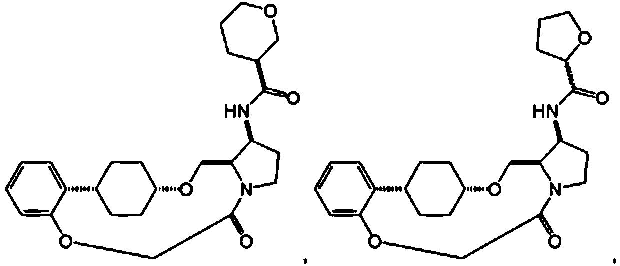

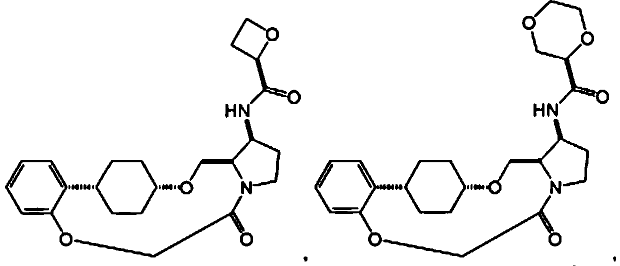

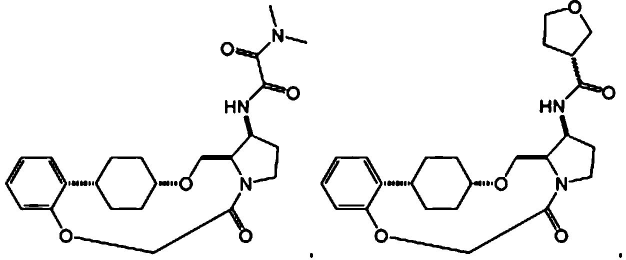

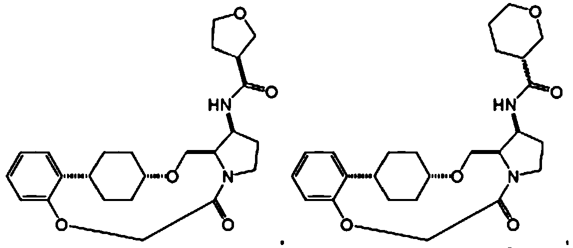

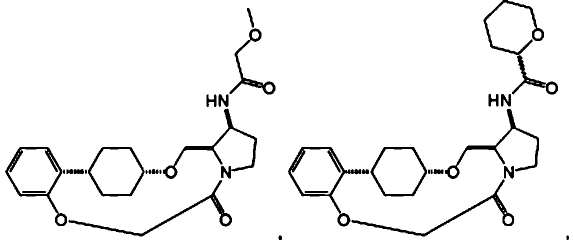

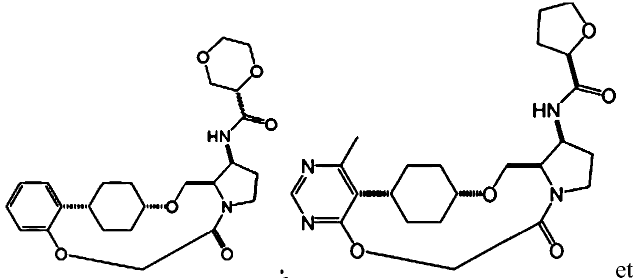

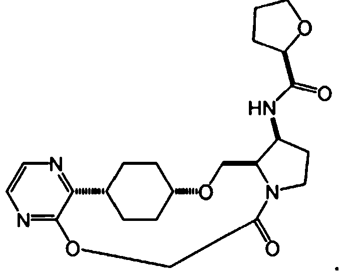

12. Composé ou sel pharmaceutiquement acceptable de celui-ci selon les revendications 1 et 3, sélectionné dans le groupe consistant en :

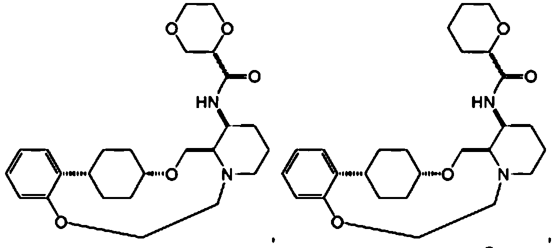

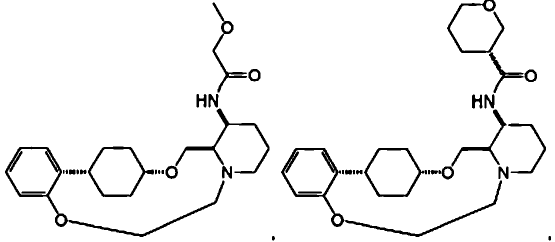

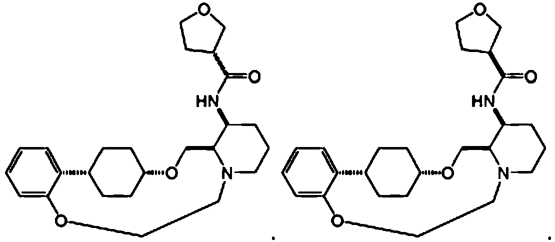

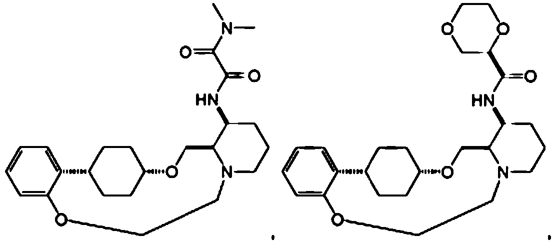

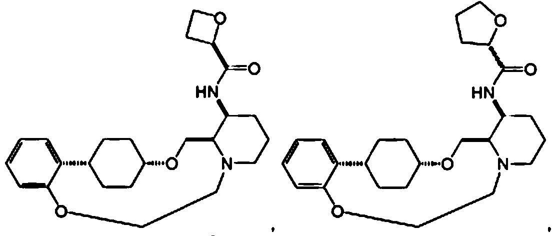

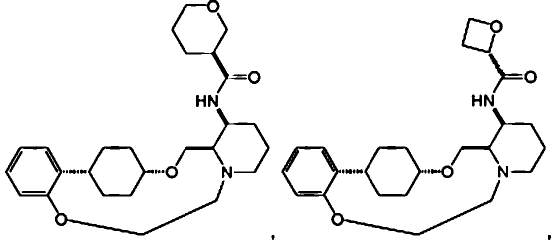

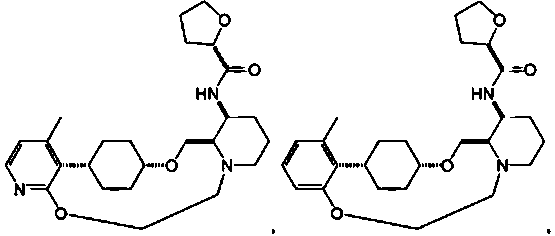

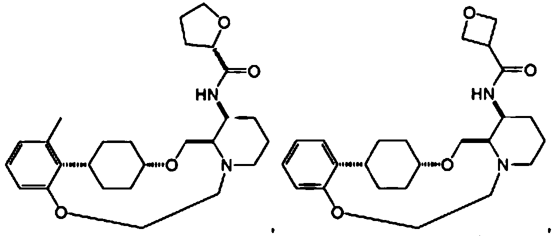

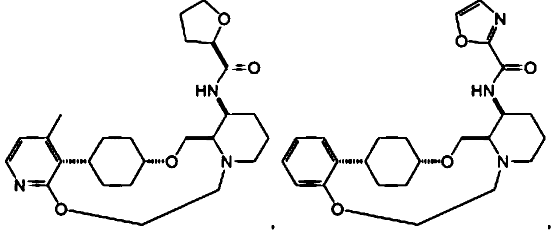

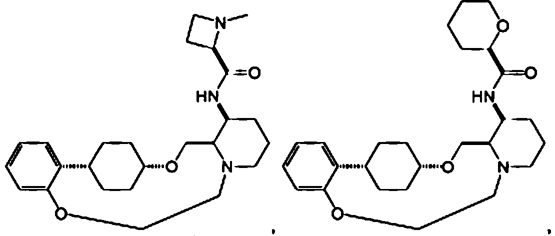

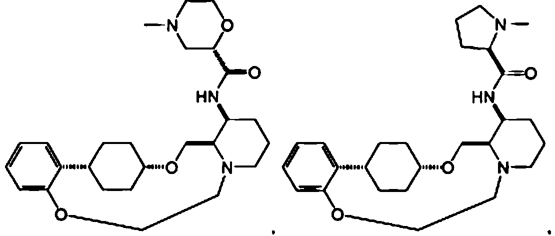

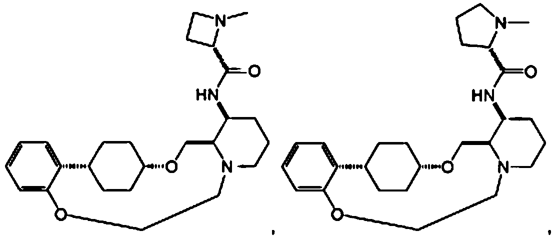

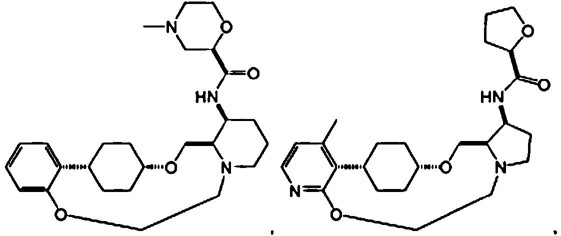

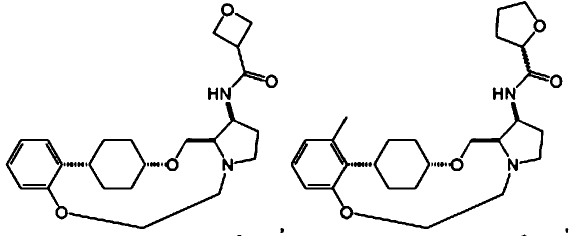

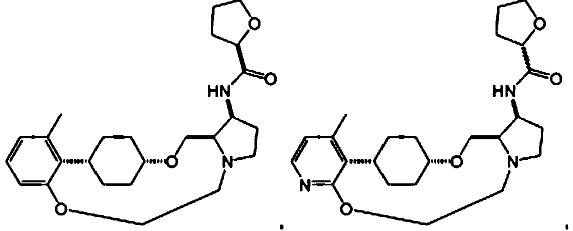

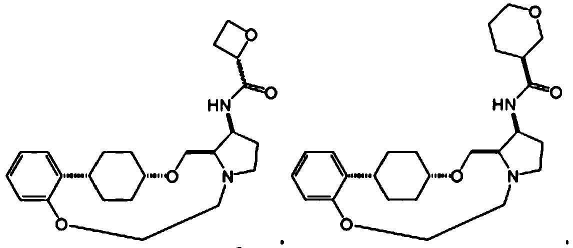

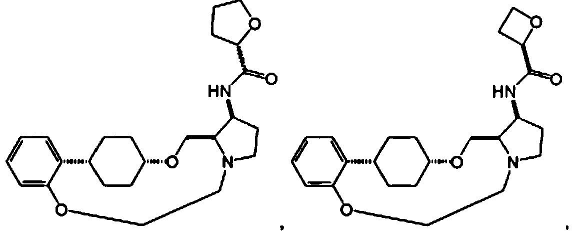

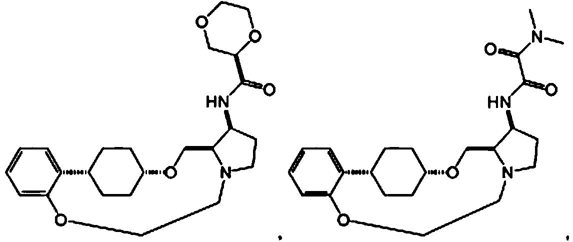

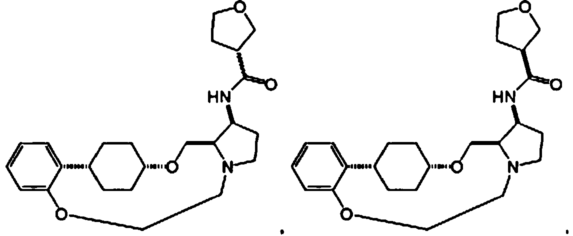

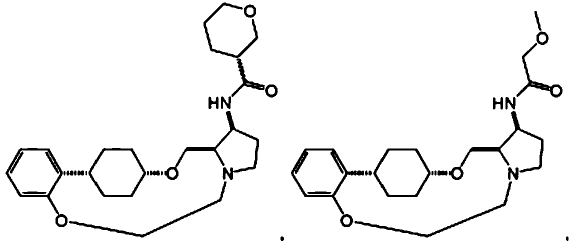

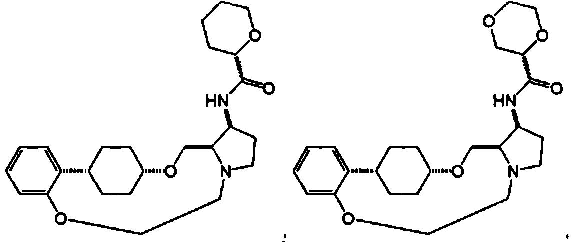

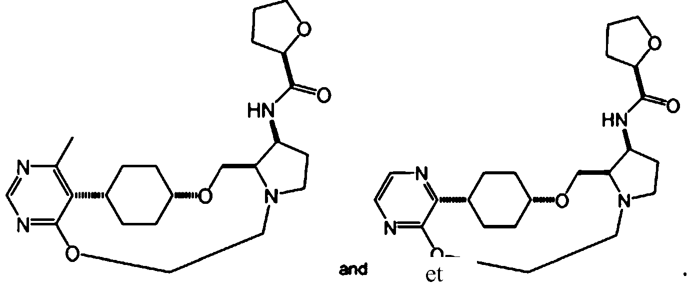

13. Composition pharmaceutique comprenant un composé selon l'une quelconque des revendications 1-12 ou un sel pharmaceutiquement acceptable de celui-ci, et un excipient pharmaceutiquement acceptable.

14. Composé selon l'une quelconque des revendications 1-12 ou sel pharmaceutiquement acceptable de celui-ci, ou composition selon la revendication 13 pour utilisation dans le traitement de la narcolepsie.

15. Composé selon l'une quelconque des revendications 1-12 ou sel pharmaceutiquement acceptable de celui-ci, ou composition selon la revendication 13 pour utilisation dans le traitement de la cataplexie.
